# EUROPEAN PATENT APPLICATION

(11) **EP 4 534 073 A2**
(43) Date of publication of application: **09.04.2025**
(21) Application number: 24221637.2
(22) Date of filing: 19.10.2021
(51) Int. Cl.: A61K 9/00

(54) **COMPOSITIONS SUITABLE FOR USE IN NEONATES**

(30) Priority: 19.10.2020 US 202063093696 P
(62) Divisional of application: 21807381.5
(71) Applicant: OAK HILL BIO LIMITED, Altrincham Cheshire WA14 2DT (GB)
(72) Inventor: SALAMAT-MILLER, Nazila, Lexington, MA 02421 (US); TAYLOR, Katherine, Lexington, MA 02421 (US); HE, Bing, Lexington, MA 02421 (US); BHATACHARYA, Indranil, Lexington, MA 02421 (US); HAN, Linda, Lexington, MA 02421 (US); AMSDEN, Benita, Lexington, MA 02421 (US); KRANTZ, James, Lexington, MA 02421 (US)
(74) Representative: Hambleton, Bernadette Angelina

(57) **Abstract**

The present invention provides stable, pure and potent compositions optimized for administration to neonates and/or preterm infants, and use of the same in methods for treating intraventricular hemorrhage (IVH), bronchopulmonary dysplasia (BPD), and/or chronic lung disease of prematurity (CLD), comprising administering to a subject in need of treatment a pharmaceutical composition comprising insulin-like growth factor-1 (IGF-1) and insulin-like growth factor binding protein-3 (IGFBP-3), and a polysorbate 20 surfactant at high effective doses in low volumes in an infant resulting high serum IGF-1 exposure and treatment of IVH, BPD and/or CLD. In some aspects, provided herein are methods for manufacturing the compositions comprising a single-use bag, wherein the compositions have improved stability, reduced oxidation and increased potency. In some aspects, the methods provided result in reduced incidence of symptoms and features of intraventricular hemorrhage, bronchopulmonary dysplasia, right ventricular hypertrophy (RVH), pulmonary hypertension (PH), necrotizing enterocolitis, or chronic lung disease of prematurity.

## Description

This application claims priority to and benefit of U.S. Provisional Application No. 63/093,696, filed on October 19, 2020, the content of which is hereby incorporated by reference in its entirety.

### BACKGROUND

There is a great need for compositions optimized for therapeutic use in neonates and/or preterm infants, for example, to treat or reduce the incidence of conditions such as intraventricular hemorrhage, bronchopulmonary dysplasia and chronic lung disease of prematurity.

Intraventricular hemorrhage (IVH) is a life threatening condition in premature infants characterized by bleeding in and around brain ventricles, leading to acute and chronic brain injury, and possible adverse neurodevelopmental outcomes in the long-term.

IVH is a major complication of prematurity. IVH can be associated with serious complications including periventricular hemorrhagic infarction, posthemorrhagic ventricular dilatation, periventricular leukomalacia, and cerebellar hemorrhage, resulting in high mortality and morbidity. Other complications include posthemorrhagic hydrocephalus, cerebral palsy, and mental retardation, and long-term neurodevelopmental disabilities.

IVH can lead to mortality and morbidity in premature infants. Current standard of care is based on early management and stabilization of cerebral hemodynamics and respiratory support. Prognosis is related to the severity of bleeding, damage to the brain parenchyma, the presence of seizures and severity of periventricular hemorrhagic infarction.

Chronic Lung Disease (CLD) of prematurity is another serious and life threatening condition in premature infants. Extremely premature infants in particular are at very high risk for developing CLD. Bronchopulmonary dysplasia (BPD) at 36 weeks post-menstrual age is typically the earliest manifestation of CLD, but infants without BPD may also develop CLD. The term "chronic lung disease of prematurity" encompasses a broad range of symptoms and characteristics that often overlap with manifestations of BPD.

Current standard of care treatment and prevention options for BPD and CLD include respiratory support (such as supplemental oxygen, via non-invasive nasal flow, with continuous positive airway pressure, and mechanical ventilation with endotracheal intubations, pulmonary surfactant administration, bronchodilators to help open the airways, caffeine to help reduce breathing pauses (apnea) and improve breathing muscle function, and steroids to help reduce inflammation, diuretics to help reduce excess fluid in the lungs, vasodilators to help decrease blood pressure in the lungs and antibiotics to fight infections. The long term trajectory of pulmonary outcomes in infants born extremely premature commonly starts with antenatal risk factors, followed by respiratory distress syndrome (RDS) in the first hours or days of life requiring respiratory support, leading up to BPD, typically diagnosed at 36 weeks post-menstrual age, and finally chronic lung disease during infancy, childhood, adolescence and even adulthood. CLD can result in more frequent re-hospitalizations and ER visits for respiratory causes, the need for respiratory medications or home respiratory support, and many children suffer from a form of reactive airway disease (asthma) that continues to limit their quality of life.

Medical treatment of premature neonates, who sometimes have a body mass of about 1 to 2 lbs (450-900 g), presents many challenges not least because the amounts that can be administered are so small. Delivering accurate doses of proteins by infusion to this patient population, and ensuring compatibility of pharmaceutical compositions with clinical administration components (e.g., infusion equipment) is critical.

What is more this class of patients are extremely weak and vulnerable. Toxicological risks posed by exposure to substances such as excipients in the course of pharmaceutical treatments must be carefully evaluated. Neonates may exhibit different sensitivity to chemicals, such as excipients used in formulations, as compared to adults and older children. This creates a huge challenge for those seeking to treat this patient population.

In addition, each therapeutic protein has different properties, for example a balance of hydrophobic and charged moieties on the surface of the folded molecule. Therefore, each therapeutic protein needs a formulation, which is optimized specifically for its properties, *e.g.,* such that the formulation is compatible with the pharmacological activity and stability of the therapeutic protein, and its suitability for administration. This challenge is even greater when the therapeutic entity is a protein complex.

### SUMMARY

The present disclosure relates to compositions optimized for administration to neonates and/or preterm infants, and methods of treatment employing the same, *e.g.,* use of the composition in treatment (particularly use in said patient population), for example, in the treatment of intraventricular hemorrhage, bronchopulmonary dysplasia and chronic lung disease of prematurity. In a further independent aspect, there is provided processes for manufacturing the formulation of the composition, for example employing a single use bag, in particular minimizing the contact of the formulation with stainless steel, to provide a robust process for achieving formulations having reduced oxidation.

Compositions provided herein are suitable for use in neonates and have one or more (such as all) of the following benefits: minimized excipients (to minimize adverse effects and maximize intolerance/toxicity in the patient population); minimized loss of the protein complex when the composition in administered parenterally (for example using tubing, rubber and/or plastic materials and/or filters, for example, for via intravenous (IV) infusion); are stable (for example physically and/or chemically stable which advantageously gives a shelf-life of at least 3-6 months); and have the ability to deliver active therapeutic at the appropriate concentration levels into the neonate patient population.

Thus, in one aspect there is provided a low concentration pharmaceutical composition suitable for administration of a therapeutic protein complex to a neonate comprising: Insulin-like growth factor 1 (IGF-1), and Insulin-like growth factor binding protein 3 complexed (for example, in a range 0.75 to 1.25: 1 or 1: 0.75-1.25 such as in equimolar amounts); and a non-ionic surfactant (for example, a polysorbate surfactant, such as polysorbate 20 or polysorbate 80, in particular polysorbate 20) in the range of 0.0025% to 0.0075%.

In one embodiment, the pharmaceutical composition is isotonic.

In one embodiment, the pharmaceutical composition is liquid.

In one embodiment, the concentration of the therapeutic complex is in the range 10 to 100 mg/L (for example 15, 20, 25, 30, 35, 40, 45, 50, 55, 60, 65, 70, 75, 80, 85, 90, 95 or 100 mg/L) such as 45-55 mg/L in particular 50 mg/L).

In one embodiment, the pharmaceutical composition is aqueous.

In one embodiment, the formulation is provided as a unit comprising 50 micrograms/mL rhIGF-1/rhIGFBP-3 solution in 50 mM sodium acetate and 105 mM sodium chloride with 0.005% (v/v) polysorbate 20, at pH 5.5, stored at 2°C to 8°C (36°F to 46°F).

In one embodiment, the pharmaceutical composition according to the present disclosure is provided as final product in an infusion bag, or glass vial, containing approximately 6.5 mL extractable volume.

In one embodiment, the pharmaceutical formulation can be administered parenterally without loss of the therapeutic protein complex (such as without loss of the activity of the therapeutic protein complex).

In one embodiment, the composition has a minimal number and/or quantity of excipients, for example 1, 2, 3, 4 or 5 excipients.

In one embodiment, activity or another property of the composition is measured by an assay or method disclosed herein, such as in the examples.

In one embodiment, the polysorbate surfactant, such as polysorbate 20 or polysorbate 80, in particular polysorbate 20 % is in the range of 0.0025% to 0.0075% v/v.

In one embodiment, the pH of the composition is in the range pH 5 to pH 7, or pH 5.3 to pH 5.8, such as pH 5.3, 5.4, 5.5, 5.6, 5.7, 5.8, in particular pH 5.5.

In one embodiment the formulation delivers at least 80% of the dose of the therapeutic protein complex to the neonate in an active form, for example 81, 82, 83, 84, 85, 86, 87, 88, 89, 90, 91, 92, 93, 94, 95, 96, 97, 98, 99 or 100%.

Thus, in a preferred embodiment there is provided a low concentration pharmaceutical composition suitable for administration of a therapeutic protein complex to a neonate (in particular in an active form) comprising 10 to 1000 micrograms per mL (for example, 10 to 100 micrograms/mL, i.e., for example, 15, 20, 25, 30, 35, 40, 45, 50, 55, 60, 65, 70, 75, 80, 85, 90, 95 or 100 micrograms/mL, such as 45-55 micrograms/mL, in particular 50 micrograms/mL) of complexed Insulin-like growth factor 1 (IGF-1), and Insulin-like growth factor binding protein 3 (IGFBP-3) (for example in a range 0.75 to 1.25: 1 or 1: 0.75-1.25 such as in equimolar amounts; and a non-ionic surfactant (for example a polysorbate surfactant, such as polysorbate 20 or polysorbate 80, in particular polysorbate 20) in the range of 0.0025% to *0.0075%* (*e.g.* 0.005%) ; a buffer *(e.g.* 50 mM sodium acetate), salt *(e.g.* 105 mM sodium chloride) wherein the pH of the formulation is in the range pH 5.3 to 5.8 *(e.g.* pH 5.5).

In further embodiments, the present invention provides an effective treatment of diseases and complications of prematurity, including, among others, Intraventricular Hemorrhage (IVH), Bronchopulmonary Dysplasia (BPD), and Chronic Lung Disease (CLD) of prematurity. The invention provides, in part, methods and pharmaceutical compositions comprising a combination of insulin-like growth factor-1 (IGF-1) and insulin-like growth factor binding protein-3 (IGFBP-3), and further comprising a surfactant (*e.g.* polysorbate 20), together which, e.g., when administered for example, intravenously immediately after birth to replace IGF-1 lost from the maternal supply, can ameliorate or reduce Intraventricular Hemorrhage (IVH), Bronchopulmonary Dysplasia and/or Chronic Lung Disease of prematurity. The present invention is based, in part, on the surprising discovery that addition of surfactant (*e.g.* polysorbate 20) at low concentrations (*e.g.* 0.0025% - 0.0075% v/v) to the IGF-1/IGFBP-3 pharmaceutical composition results in improved potency and increased stability. Without wishing to be bound by any particular theory, it is contemplated, for example, that the surfactant (*e.g.* polysorbate 20) at such low concentrations, minimizes adsorption-related product loss, eliminating the need to prime and flush intravenous infusion sets prior to drug product administration, significantly reducing loss of drug product and improving potency. The present invention is also based, in part, on the reduced accumulation of oxidized species and improved stability achieved through the use of a contact surface other than stainless steel, such as a single-use bag during compounding the complex with a formulation solution. Improved stability is achieved during storage at about 25°C *(e.g.* about 23°C to about 27°C), or storage at about 40°C *(e.g.* about 38°C to about 42°C) for example, during storage for about 3 - 6 months. In addition to the advantages of reduced aggregation and increased stability and purity, the formulation comprising surfactant allows for accurate dosing and the administration of small dose volumes to extremely premature neonates (See [0008]) at risk for developing complications of prematurity, for example, Intraventricular Hemorrhage, Bronchopulmonary Dysplasia, and/or Chronic Lung Disease of prematurity. As used herein, Chronic Lung Disease (CLD) and Chronic Lung Disease of prematurity are used interchangeably. The formulation of the present invention is stable and shows reduced aggregation at a range of concentrations from 10 micrograms/mL to 1000 micrograms/mL (*e.g.* 20 micrograms/mL, 30, 40, 50 micrograms/mL). In contrast, in the absence of polysorbate and when exposed to routine stresses, high molecular weight species accumulate that contribute to decreased stability, increased aggregation and potential increased immunogenicity. In summary, the present invention provides, among other things, an improved formulation of IGF-1/IGFBP-3 with advantages of increased potency and improved stability during storage, in particular, with minimized toxicity, providing a safe and efficacious product for administration to neonates in treating diseases of prematurity.

In one aspect, stable formulations of rIGF-1/rIGFBP-3 comprising a surfactant can be prepared and delivered at a dose such as about 400 micrograms/kg/24 hours. In one aspect, stable formulations of rIGF-1/rIGFBP-3 comprising a surfactant can be prepared and delivered at a dose such as about 1000 micrograms/kg/24 hours. The addition of surfactant also increases the stability of the product by reducing protein loss due to adsorption. In one aspect, the methods provided herein result in high effective exposure of serum IGF-1 by administration of the pharmaceutical rIGF-1/rIGFBP-3 composition at a dose of about 250 micrograms/kg/24 hours or lower in the presence of the surfactant.

The present application, is based in part, on the advantages arising from the incorporation of polysorbate surfactants, for example, polysorbate 20 or polysorbate 80, in a pharmaceutical rIGF-1/rIGFBP-3 composition. The present application discloses a highly potent, stable formulation resulting in high rIGF-1 serum exposure levels upon administration of rIGF-1/rIGFBP-3 at a concentration of about 10 micrograms/mL - 1000 micrograms/mL for treatment of diseases and complications of prematurity, for example, intraventricular hemorrhage, bronchopulmonary dysplasia, and chronic lung disease of prematurity.

In one aspect of the present disclosure, provided herein is a pharmaceutical composition comprising a protein complex comprising recombinant insulin-like growth factor-1 (rIGF-1), recombinant insulin-like growth factor binding protein-3 (rIGFBP-3), and a polysorbate surfactant at a concentration of between about 0.0025% v/v and 0.0075% v/v, wherein the rIGF-1 and rIGFBP-3 are complexed in equimolar amounts, and wherein the rIGF-1/IGFBP-3 is at a concentration of about 10 micrograms/mL - 1000 micrograms/mL.

In some aspects, provided herein is a pharmaceutical composition comprising a protein complex comprising recombinant insulin-like growth factor 1 (rIGF-1), recombinant insulin-like growth factor binding protein 3 (rIGFBP-3), and a polysorbate surfactant at a concentration of between about 0.0025% and 0.0075%, wherein the rIGF-1 and rIGFBP-3 are complexed in a range of 0.75 to 1.25: 1 or 1: 0.75-1.25, for example, in equimolar amounts. In some embodiments, the polysorbate surfactant is polysorbate 20 or polysorbate 80.

In some embodiments, the polysorbate surfactant is polysorbate 20. Polysorbate 20 is also known as polyoxyethylene sorbitan monolaurate.

In some embodiments, the polysorbate surfactant is polysorbate 80. Polysorbate 80 is also known as polyoxyethylene sorbitan monooleate.

In some embodiments, polysorbate surfactant is at a concentration of between about 0.001% to 2.4% v/v. In some embodiments, the polysorbate surfactant is at a concentration of between about 0.2% to 0.4% v/v. In some embodiments, polysorbate surfactant is at a concentration of about 0.001%, 0.005%, 0.01%, 0.02%, 0.03%, 0.05%, 0.10%, 0.15%, 0.2%, 0.5%, 0.7%, 1.0%, 1.2%, 1.5%, 1.8%, 2.0%, 2.2% or 2.4% v/v.

In some embodiments, the polysorbate surfactant is at a concentration of between about 0.0025% and 0.0075% v/v. In some embodiments, the polysorbate surfactant is at a concentration of about 0.0025%, about 0.005%, or about 0.0075% v/v. In some embodiments, the polysorbate surfactant is at a concentration of about 0.005% v/v.

In some embodiments, less than about 20% of the IGF-1 in the composition exists as oxidized species after storage for about 6 months at 25 °C. In some embodiments, less than about 15% of the IGF-1 in the composition exists as oxidized species after storage for about 6 months at 25 °C. In some embodiments, less than 10% of the IGF-1 exists as oxidized species after storage for about 6 months at 25 °C. In some embodiments, less than 5% of the IGF-1 exists as oxidized species after storage for about 6 months at 25 °C. In some embodiments, less than 4% of the IGF-1 exists as oxidized species after storage for about 6 months at 25 °C. In some embodiments, less than 2% of the IGF-1 exists as oxidized species after storage for about 6 months at 25 °C. In some embodiments, less than 4% of the IGF-1 exists as oxidized species after storage for about 3 months at 25 °C. In some embodiments, less than 2% of the IGF-1 exists as oxidized species after storage for about 3 months at 25 °C.

In some embodiments, less than 10% of the IGF-1 exists as oxidized species after storage for about 3 months at 40 °C. In some embodiments, less than 8% of the IGF-1 exists as oxidized species after storage for about 3 months at 40 °C. In some embodiments, less than 6% of the IGF-1 exists as oxidized species after storage for about 3 months at 40 °C. In some embodiments, less than 5% of the IGF-1 exists as oxidized species after storage for about 3 months at 40 °C. In some embodiments, less than 4% of the IGF-1 exists as oxidized species after storage for about 3 months at 40 °C. In some embodiments, less than 10% of the IGF-1 exists as oxidized species after storage for about 3 months at 40 °C. In some embodiments, less than 3 % of the IGF-1 exists as oxidized species after storage for about 3 months at 40 °C. In some embodiments, less than 8% of the IGF-1 exists as oxidized species after storage for about 1 month at 40 °C. In some embodiments, less than 6% of the IGF-1 exists as oxidized species after storage for about 1 month at 40 °C. In some embodiments, less than 5 % of the IGF-1 exists as oxidized species after storage for about 1 month at 40 °C. In some embodiments, less than 4% of the IGF-1 exists as oxidized species after storage for about 1 month at 40 °C. In some embodiments, less than 3% of the IGF-1 exists as oxidized species after storage for about 1 month at 40 °C. In some embodiments, less than 2% of the IGF-1 exists as oxidized species after storage for about 1 month at 40 °C.

In some embodiments, the percentage of oxidized species in the composition is determined by Reversed Phase-Ultra Performance Liquid Chromatography (RP-UPLC). In some embodiments, the IGFBP-3 comprises less than 5% of trisulfide variants.

In some embodiments, the composition further comprises a buffer comprising sodium acetate, acetic acid and/or sodium chloride. In some embodiments, the composition further comprises a buffer comprising sodium acetate or acetic acid. In some embodiments, the composition further comprises a buffer comprising sodium chloride.

In some embodiments, the sodium acetate or acetic acid is at a concentration of between about 10 and 100 mM. In some embodiments, the sodium acetate or acetic acid is at a concentration of about 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM. In some embodiments, the sodium acetate or acetic acid is at a concentration of about 50 mM.

In some embodiments, the sodium chloride is at a concentration of about 20 mM and 200 mM. In some embodiments, the sodium chloride is at a concentration of about 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 155 mM, about 160 mM, about 165 mM, about 170 mM, about 175 mM, about 180 mM, about 185 mM, about 190 mM, about 195 mM or about 200 mM. In some embodiments, the sodium chloride is at a concentration of about 105 mM.

In some embodiments, the composition is at a pH between about 5.0 and 7.0. In some embodiments, the pH is about 5.0, about 5.2, about 5.4, about 5.6, about 5.8, about 6.0, about 6.2, about 6.4, about 6.6, about 6.8 or about 7.0. In some embodiments, the composition is at a pH of about 5.1, about 5.3, about 5.5, about 5.7 or about 5.9, about 6.1, about 6.3, about 6.5, about 6.7 or about 6.9. In some embodiments, the composition is at a pH of between about 5.3 and 5.8. In some embodiments, the composition is at a pH of about 5.5.

In some embodiments, the rIGF-1/IGFBP-3 is at a concentration of between about 10 micrograms/mL - 1000 micrograms/mL. In some embodiments, the rIGF-1/IGFBP-3 is at a concentration of between about 10 micrograms/mL - 100 micrograms/mL. In some embodiments, the rIGF-1/IGFBP-3 is at a concentration of between about 20 micrograms/mL - 100 micrograms/mL. In some embodiments, the rIGF-1/IGFBP-3 is at a concentration of about 10 micrograms/mL, about 20 micrograms/mL, about 30 micrograms/mL, about 40 micrograms/mL, about 50 micrograms/mL, about 60 micrograms/mL, about 70 micrograms/mL, about 80 micrograms/mL, about 90 micrograms/mL or about 100 micrograms/mL. In some embodiments, the rIGF-1/IGFBP-3 is at a concentration of about 10 micrograms/mL, 25 micrograms/mL, 50 micrograms/mL, 75 micrograms/mL, 100 micrograms/mL. In some embodiments, the rIGF-1/IGFBP-3 is at a concentration of about 45-55 micrograms/mL. In some embodiments, the rIGF-1/IGFBP-3 is at a concentration of about 50 micrograms/mL. In some embodiments, the rIGF-1/rIGFBP-3 is at a concentration of about 75 micrograms/mL.

In some embodiments, the rIGF-1/IGFBP-3 is at a concentration of between about 100 micrograms/mL - 1000 micrograms/mL. In some embodiments, the rIGF-1/IGFBP-3 is at a concentration of about 100 micrograms/mL, about 200 micrograms/mL, about 300 micrograms/mL, about 400 micrograms/mL, about 500 micrograms/mL, about 600 micrograms/mL, about 700 micrograms/mL, about 800 micrograms/mL, about 900 micrograms/mL, about 1000 micrograms/mL.

In some embodiments, the pharmaceutical composition is suitable for subcutaneous or intravenous injection. In some embodiments, the pharmaceutical composition is suitable for subcutaneous injection. In some embodiments, the pharmaceutical composition is suitable for intravenous injection.

In some embodiments, the pharmaceutical composition has a storage life of 20 months or more at a temperature of between 2-8 °C. In some embodiments, the pharmaceutical composition has a storage life of at least 24 months at a temperature of between 2-8 °C. In some embodiments, the pharmaceutical composition is stable at room temperature and/or ambient light. In some embodiments, the pharmaceutical composition is stable at room temperature and/or under ambient light for at least 8 hours, 12 hours, 24 hours, 36 hours, or 48 hours. In some embodiments, the pharmaceutical composition is stable at room temperature and/or under ambient light for at least 8 hours. In some embodiments, the pharmaceutical composition is stable at room temperature and/or under ambient light for at least 12 hours. In some embodiments, the pharmaceutical composition is stable at room temperature and/or under ambient light for at least 24 hours. In some embodiments, the pharmaceutical composition is stable at room temperature and/or under ambient light for at least 48 hours.

In some embodiments, the pharmaceutical composition comprises rIGF-1 and rIGFBP-3 complexed in a range of 0.75 to 1.25: 1 or 1: 0.75-1.25, for example, in equimolar amounts. In some embodiments, the IGF-1 is recombinantly produced. In some embodiments, the IGFBP-3 is recombinantly produced. In some embodiments, the IGF-1 and the IGFBP-3 are complexed prior to administration to the subject.

In some embodiments, the pharmaceutical composition comprising recombinant insulin-like growth factor 1 (rIGF-1), recombinant insulin-like growth factor binding protein 3 (rIGFBP-3), and polysorbate 20 is more stable at room temperature than a pharmaceutical composition comprising recombinant insulin-like growth factor 1 (rIGF-1), recombinant insulin-like growth factor binding protein 3 (rIGFBP-3) without polysorbate 20.

In some embodiments, the pharmaceutical composition comprising recombinant insulin-like growth factor 1 (rIGF-1), recombinant insulin-like growth factor binding protein 3 (rIGFBP-3), and polysorbate 20 has less oxidation at room temperature than a pharmaceutical composition comprising recombinant insulin-like growth factor 1 (rIGF-1), recombinant insulin-like growth factor binding protein 3 (rIGFBP-3) without polysorbate 20.

In some embodiments, the percentage of oxidized IGF-1 species does not increase more than 25% upon storage at a temperature of about 25 °C after three months. In some embodiments, the percentage of oxidized IGF-1 species does not increase more than 10% upon storage at a temperature of about 25 °C after three months.

In some embodiments, provided herein is a pharmaceutical composition further comprising a pharmaceutically acceptable carrier.

In some embodiments, provided herein is a method of treating or preventing intraventricular hemorrhage (IVH) comprising administering to a subject in need of treatment a composition comprising recombinant insulin-like growth factor 1 (rIGF-1) and recombinant insulin-like growth factor binding protein 3 (rIGFBP-3).

In some aspects, provided herein is a method of treating or preventing intraventricular hemorrhage comprising administering to a subject in need thereof a composition comprising recombinant insulin-like growth factor 1 (rIGF-1), recombinant insulin-like growth factor binding protein 3 (rIGFBP-3), and a polysorbate surfactant. (e.g., polysorbate 20).

In some aspects, provided herein is a method of treating or preventing intraventricular hemorrhage (IVH) comprising administering to a subject in need thereof a pharmaceutical composition comprising recombinant insulin-like growth factor 1 (rIGF-1), recombinant insulin-like growth factor binding protein 3 (rIGFBP-3), and a polysorbate surfactant (e.g., polysorbate 20) at a concentration of between about 0.0025% v/v and 0.0075% v/v, wherein the rIGF-1 and rIGFBP-3 are complexed in a range of 0.75 to 1.25: 1 or 1: 0.75-1.25, for example, in equimolar amounts, and wherein the rIGF-1/IGFBP-3 is at a concentration of about 10 micrograms/mL - 1000 micrograms/mL.

In some aspects, provided herein is a method of treating or preventing intraventricular hemorrhage (IVH) comprising administering to a subject in need thereof a composition comprising recombinant insulin-like growth factor 1 (rIGF-1), recombinant insulin-like growth factor binding protein 3 (rIGFBP-3), wherein the subject in need has gestational age of less than 26 weeks. In some embodiments, provided herein is a method of treating or preventing IVH comprising administering to a subject in need thereof a composition comprising recombinant insulin-like growth factor 1 (rIGF-1), recombinant insulin-like growth factor binding protein 3 (rIGFBP-3), wherein the subject in need has gestational age of between about 23 weeks to 25 weeks + 6 days. In some aspects, provided herein is a method of treating or preventing intraventricular hemorrhage (IVH) comprising administering to a subject in need thereof a composition comprising recombinant insulin-like growth factor 1 (rIGF-1), recombinant insulin-like growth factor binding protein 3 (rIGFBP-3), at a therapeutic amount sufficient to achieve reduced incidence of intraventricular hemorrhage relative to a control.

In some embodiments, the reduced incidence is achieved between about 36 to 40 weeks post-menstrual age (PMA) as assessed by ultrasound or MRI. In some embodiments, the reduced incidence is achieved between about 35 to 42 weeks post-menstrual age as assessed by ultrasound or MRI. In some embodiments, the reduced incidence is achieved by 35 weeks post-menstrual age as assessed by ultrasound or MRI. In some embodiments, the reduced incidence is achieved by 36 weeks post-menstrual age as assessed by ultrasound or MRI. In some embodiments, the reduced incidence is achieved by 37 weeks post-menstrual age as assessed by ultrasound or MRI. In some embodiments, the reduced incidence is achieved by 38 weeks post-menstrual age as assessed by ultrasound or MRI. In some embodiments, the reduced incidence is achieved by 39 weeks post-menstrual age as assessed by ultrasound or MRI. In some embodiments, the reduced incidence is achieved by 40 weeks post-menstrual age as assessed by ultrasound or MRI. In some embodiments, the reduced incidence is achieved by 41 weeks post-menstrual age as assessed by ultrasound or MRI. In some embodiments, the reduced incidence is achieved by 42 weeks post-menstrual age as assessed by ultrasound or MRI.

In some embodiments, the reduced incidence is assessed by ultrasound.

In some embodiments, the reduced incidence for IVH is at least about 30% reduction in incidence of grade II, grade III, or grade IV IVH. In some embodiments, the reduced incidence for IVH is at least about 30% reduction in incidence of grade II IVH. In some embodiments, the reduced incidence for IVH is at least about 30% reduction in incidence of grade III IVH. In some embodiments, the reduced incidence for IVH is at least about 30% reduction in incidence of grade IV IVH.

In some embodiments, provided herein is a method of treating or preventing bronchopulmonary dysplasia comprising administering to a subject in need thereof a composition. In some embodiments, provided herein is a method of treating or preventing chronic lung disease of prematurity comprising administering to a subject in need of treatment a composition.

In one aspect, provided herein is a method of treating or preventing chronic lung disease of prematurity comprising administering to a subject in need thereof a composition comprising recombinant insulin-like growth factor 1 (rIGF-1), recombinant insulin-like growth factor binding protein 3 (rIGFBP-3), and a surfactant.

In some embodiments, the surfactant is a polysorbate surfactant. In some embodiments, the polysorbate surfactant is selected from polysorbate 20 (P20) or polysorbate 80 (P80). In some embodiments, the polysorbate surfactant is P20. In some embodiments, the polysorbate surfactant is P80.

In some embodiments, the polysorbate surfactant is at a concentration of between about 0.001% to 2.4% v/v. In some embodiments, the polysorbate surfactant is at a concentration of between about 0.2% to 0.4% v/v. In some embodiments, polysorbate surfactant is at a concentration of about 0.001%, 0.005%, 0.01%, 0.02%, 0.03%, 0.05%, 0.10%, 0.15%, 0.2%, 0.5%, 0.7%, 1.0%, 1.2%, 1.5%, 1.8%, 2.0%, 2.2% or 2.4% v/v. In some embodiments, the polysorbate surfactant is at a concentration of about 0.0025%, about 0.005%, or about 0.0075% v/v. In some embodiments, the polysorbate is at a concentration of 0.005%.

In some embodiments, the subject in need of treatment is an infant.

In some embodiments, the infant was prematurely born by at least 1 week, 2 weeks, 3 weeks, 4 weeks, 1 month, 2 months or 3 months. In some embodiments, the infant is between about 23 to 34 weeks of postmenstrual age (PMA). In some embodiments, the infant is about 23 weeks, about 24 weeks, about 25 weeks, about 26 weeks, about 27 weeks, about 28 weeks, about 29 weeks, about 30 weeks, about 31 weeks, about 32 weeks, about 33 weeks or about 34 weeks of PMA. In some embodiments, the infant is about 23 weeks of PMA. In some embodiments, the infant is below 36 weeks of PMA. In some embodiments, the infant is below

35 weeks of PMA. In some embodiments, the infant is below 34 weeks of PMA. In some embodiments, the infant is below 33 weeks of PMA. In some embodiments, the infant is below 32 weeks of PMA. In some embodiments, the infant is below 31 weeks of PMA. In some embodiments, the infant is below 30 weeks of PMA. In some embodiments, the infant is below 29 weeks of PMA. In some embodiments, the infant is below 28 weeks of PMA. In some embodiments, the infant is below 27 weeks of PMA. In some embodiments, the infant is below 26 weeks of PMA. In some embodiments, the infant is below 25 weeks of PMA. In some embodiments, the infant is below 24 weeks of PMA. In some embodiments, the infant is below 23 weeks of PMA.

In some embodiments, the infant is born at less than 32 weeks of gestational age (GA). In some embodiments, the infant is born at less than 31 weeks of GA. In some embodiments, the infant is born at less than 30 weeks of GA. In some embodiments, the infant is born at less than 29 weeks of GA. In some embodiments, the infant is born at less than 28 weeks of GA. In some embodiments, the infant is born at between 28-32 weeks of GA.

In some embodiments, the subject in need of the treatment is administered the composition is subcutaneously, intravenously, intramuscularly, or orally. In some embodiments, the method comprises a composition administered subcutaneously or intravenously. In some embodiments, the composition is administered intravenously.

In some embodiments, the composition is administered at a dosage of about 100 to 1000 micrograms/kg/24 hours. In some embodiments, the composition is administered at a dosage of about 100 to 800 micrograms/kg/24 hours. In some embodiments, the composition is administered at a dosage of about 100 to 500 micrograms/kg/24 hours. In some embodiments, the composition is administered at a dosage of between 100 micrograms/kg/24 hours and 450 micrograms/kg/24 hours. In some embodiments, the composition is administered at a dosage of between 100 micrograms/kg/24 hours and 400 micrograms/kg/24 hours. In some embodiments, the composition is administered at a dosage of between 150 micrograms/kg/24 hours and 400 micrograms/kg/24 hours. In some embodiments, the composition is administered at a dosage of between 200 micrograms/kg/24 hours and 400 micrograms/kg/24 hours. In some embodiments, the composition is administered at a dosage of about 250 to 1000 micrograms/kg/24 hours. In some embodiments, the composition is administered at a dosage of about 800 micrograms/kg/24 hours. In some embodiments, the composition is administered at a dosage of about 250 to 800 micrograms/kg/24 hours. In some embodiments, the composition is administered at a dosage of between 250 micrograms/kg/24 hours and 400 micrograms/kg/24 hours. In some embodiments, the composition is administered at a dosage of about 250 micrograms/kg/24 hours. In some embodiments, the composition is administered at a dosage of about 400 micrograms/kg/24 hours.

In some embodiments, the composition is administered once, twice, three times, or four times in a 24 hour period. In some embodiments, the composition is administered once in a 24 hour period. In some embodiments, the composition is administered by continuous infusion for the 24 hour period.

In some embodiments, the composition is administered from the time of birth up to post-menstrual age (PMA) of about 23 to 34 weeks. In some embodiments, the composition is administered from the time of birth up to PMA of about 23 to 32 weeks. In some embodiments, the composition is administered from the time of birth up to PMA of about 29 weeks plus 6 days. "PMA" or "postmenstrual age" refers to gestational age plus chronological age. In some embodiments, treating or preventing Chronic Lung Disease (CLD) of prematurity results in reduced incidence of bronchopulmonary dysplasia (BPD), right ventricular hypertrophy (RVH), pulmonary hypertension (PH), necrotizing enterocolitis, or intraventricular hemorrhage. The method provided herein comprises embodiments where the administration of the IGF-I or agonist or analog results in reduced incidence of Chronic Lung Disease (CLD) of prematurity through 12 months corrected age (CA). The corrected age of an infant is the adjusted age of the infant based on his or her delivery due date. Taking the term of the pregnancy to be 40 weeks (i.e., due date), a prematurely born infant gets a corrected age (CA) where the excess time the infant has existed outside the mother's body (i.e. the number of weeks the infant was born premature) is subtracted from the infant's real age. In some embodiments, the administration of the pharmaceutical composition results in reduced incidence of chronic lung disease of prematurity through postmenstrual age (PMA) 23 weeks to 12 months. For example, the administration of the pharmaceutical composition results in reduced incidence of CLD through at least PMA 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, 50 weeks, 51 weeks or 52 weeks. In some other embodiments, the administration of the pharmaceutical composition results in reduced incidence of CLD through at least PMA 6 months, 7 months, 8 months, 9 months, 10 months, 11 months, or 12 months. In some embodiments, the administration of the composition results in reduced incidence of Bronchopulmonary Dysplasia (BPD) through postmenstrual age (PMA) 23 weeks to 12 months. For example, the administration of the pharmaceutical composition results in reduced incidence of BPD through at least PMA 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, 50 weeks, 51 weeks or 52 weeks. In some other embodiments, the administration of the pharmaceutical composition results in reduced incidence of BPD through at least PMA 6 months, 7 months, 8 months, 9 months, 10 months, 11 months, or 12 months. In some embodiments, the administration of the pharmaceutical composition results in reduced incidence of Severe Intraventricular Hemorrhage (IVH) Grade II, III or IV through at least postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of right ventricular hypertrophy (RVH) through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 8 months, 10 months, 12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of pulmonary hypertension (PH) through (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 8 months, 10 months, 12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of necrotizing enterocolitis through (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 8 months, 10 months, 12 months, 13 months, 14 months, 15 months, 16 months, 17 months, 18 months, 19 months, 20 months, 21 months, 22 months, 23 months or 24 months.

In some embodiments, the subject has reduced IGF-1 serum levels. In some embodiments, the subject has IGF-1 levels of below 60 micrograms/L. In some embodiments, the subject has IGF-1 levels of below 50 micrograms/L. In some embodiments, the subject has IGF-1 levels of below 40 micrograms/L. In some embodiments, the subject has IGF-1 levels of below 30 micrograms/L. In some embodiments, the subject has IGF-1 levels of between about 30-50 micrograms/L.

In some embodiments, administration of the pharmaceutical composition results in elevated IGF-1 serum levels in comparison to a baseline value. In some embodiments of the disclosure, the baseline value of IGF-1 serum levels in an untreated infant is below 60 micrograms/L. In some embodiments, the baseline value of IGF-1 serum levels is below 50 micrograms/L. In some embodiments, the baseline value of IGF-1 serum levels is below 40 micrograms/L. In some embodiments, the baseline value of IGF-1 serum levels is below 30 micrograms/L. In some embodiments, the baseline value of IGF-1 serum levels is about 30 to 50 micrograms/L.

In some embodiments, the IGF-1 serum levels are elevated by at least about 25% - 50% in comparison to a baseline value. In some embodiments, the IGF-1 serum levels are elevated by at least about 10%, 15%, 20%, 25%, 30%, 35%, 40%, 45%, 50%, 55%, or 60% in comparison to a baseline value. In some embodiments, the IGF-1 serum levels are elevated by at least about 10% in comparison to a baseline value. In some embodiments, the IGF-1 serum levels are elevated by at least about 15% in comparison to a baseline value. In some embodiments, the IGF-1 serum levels are elevated by at least about 20% in comparison to a baseline value. In some embodiments, the IGF-1 serum levels are elevated by at least about 25% in comparison to a baseline value. In some embodiments, the IGF-1 serum levels are elevated by at least about 30% in comparison to a baseline value. In some embodiments, the IGF-1 serum levels are elevated by at least about 35% in comparison to a baseline value. In some embodiments, the IGF-1 serum levels are elevated by at least about 40% in comparison to a baseline value. In some embodiments, the IGF-1 serum levels are elevated by at least about 45% in comparison to a baseline value. In some embodiments, the IGF-1 serum levels are elevated by at least about 50% in comparison to a baseline value. In some embodiments, the IGF-1 serum levels are elevated by at least about 55% in comparison to a baseline value. In some embodiments, the IGF-1 serum levels are elevated by at least about 60% in comparison to a baseline value.

In some embodiments, the IGF-1 serum levels remain elevated for at least 24 hours following administration. In some embodiments, the IGF-1 serum levels remain elevated for about 48 hours. In some embodiments, the IGF-1 serum levels remain elevated for about 72 hours. In some embodiments, the IGF-1 serum levels remain elevated for at least about 7 days. In some embodiments, the IGF-1 serum levels remain elevated through postmenstrual age (PMA) of at least about 23 weeks to 34 weeks. In some embodiments, the IGF-1 serum levels remain elevated through postmenstrual age (PMA) of at least about 23 weeks to 12 months.

In some embodiments, the administration of the pharmaceutical composition results in reduced incidence of chronic lung disease of prematurity, bronchopulmonary dysplasia, right ventricular hypertrophy (RVH), pulmonary hypertension (PH), necrotizing enterocolitis, or intraventricular hemorrhage through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months,12 months, 13 months, 14 months, 15 months, 16 months, 17 months, 18 months, 19 months, 20 months, 21 months, 22 months, 23 months or 24 months. The method provided herein comprises embodiments where the administration of the pharmaceutical composition results in reduced incidence of Chronic Lung Disease (CLD) of prematurity through 12 months corrected age (CA). The corrected age of an infant is the adjusted age of the infant based on his or her due date. Taking the term of the pregnancy to be 40 weeks (i.e., due date), a prematurely born infant gets a corrected age where the excess time it has existed outside the mother's body is subtracted from its real age. In some embodiments, the administration of the pharmaceutical composition in reduced incidence of Bronchopulmonary Dysplasia (BPD) through postmenstrual age (PMA) 23 weeks to 24 months. For example, the administration of the pharmaceutical composition results in reduced incidence of BPD through PMA 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38, or 40 weeks, 45 weeks, 50 weeks, or 52 weeks. In some other embodiments, the administration of the pharmaceutical composition results in reduced incidence of BPD through PMA 6 months, 7 months, 8 months, 9 months, 10 months, 11 months,12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of Intraventricular Hemorrhage (IVH) Grade I, II, III or IV through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of right ventricular hypertrophy (RVH) through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months or 12 months. In some embodiments, the administration of the pharmaceutical composition results in reduced incidence of pulmonary hypertension (PH) through (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months,12 months, 13 months, 14 months, 15 months, 16 months, 17 months, 18 months, 19 months, 20 months, 21 months, 22 months, 23 months or 24 months. In some embodiments, the administration of the pharmaceutical composition results in reduced incidence of necrotizing enterocolitis through (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 7 months, 8 months, 9 months, 10 months, 11 months,12 months, 13 months, 14 months, 15 months, 16 months, 17 months, 18 months, 19 months, 20 months, 21 months, 22 months, 23 months or 24 months.

In some embodiments, the administration of the pharmaceutical composition results in increased Functional Status as Assessed by PREMature Infant Index (PREMII) through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks or 40 weeks.

In some embodiments, the administration of the pharmaceutical composition results in elevated IGF-1 serum target levels of between about 28-109 micrograms/mL (µg/ml). In some embodiments, the administration of the pharmaceutical composition results in elevated IGF-1 serum target levels of about 30 µg/ml, about 40 µg/ml, about 50 µg/ml, about 60 µg/ml, about 70 µg/ml, about 80 µg/ml, about 90 µg/ml, about 100 µg/ml or about 110 µg/ml.

In some embodiments, the reduced incidence is at least about 20% - 50% relative to an untreated control. In some embodiments, the reduced incidence is about 10%, about 20%, about 30%, about 40%, about 50%, about 60%, or about 70% relative to an untreated control.

In some embodiments, the reduced incidence is at least about 20 - 50% relative to a control receiving standard of care. In some embodiments, the reduced incidence is about 10%, about 20%, about 30%, about 40%, about 50%, about 60%, or about 70% relative to a control receiving standard of care.

In some embodiments, the increased functional status is at least about 20% - 50% relative to an untreated control. In some embodiments, the increased functional status is about 10%, about 20%, about 30%, about 40%, about 50%, about 60%, or about 70% relative to a control receiving standard of care.

In some embodiments, the increased functional status is at least about 20 - 50% relative to a control receiving standard of care. In some embodiments, the increased functional status is about 20%, about 30%, about 40%, about 50%, about 60%, or about 70% relative to a control receiving standard of care.

In some embodiments, provided herein is a method of treating or preventing chronic lung disease of prematurity comprising administering to a subject in need thereof a composition comprising recombinant insulin-like growth factor 1 (rIGF-1), recombinant insulin-like growth factor binding protein 3 (rIGFBP-3), and a polysorbate 20 surfactant.

The method disclosed herein comprises embodiments where the administration of the composition results in increased Functional Status as Assessed by PREMature Infant Index (PREMII) through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks or 40 weeks.

In some aspects, provided herein is a method for manufacturing a protein complex comprising insulin-like growth factor 1 (IGF-1) and insulin-like growth factor binding protein 3 (IGFBP-3), wherein the method comprises a use of a contact surface other than stainless steel, such as a single-use bag, during compounding the complex with a formulation solution, wherein, in the protein complex manufactured by such a method, an increase is oxidized species of the IGF-1 is less than 20 % upon storage for 6 months at 25 °C. In some embodiments, the increase in oxidized species of the IGF-1 is less than 18%. In some embodiments, the increase in oxidized species of the IGF-1 is less than 16%. In some embodiments, the increase in oxidized species of the IGF-1 is less than 15% upon storage for 6 months at 25°C. In some embodiments, the increase in oxidized species of the IGF-1 is less than 12% upon storage for 6 months at 25°C. In some embodiments, the increase in oxidized species of the IGF-1 is less than 10% upon storage for 6 months at 25°C. In some embodiments, the increase in oxidized species of the IGF-1 is less than 8% upon storage for 6 months at 25°C. In some embodiments, the increase in oxidized species of the IGF-1 is less than 6% upon storage for 6 months at 25°C. In some embodiments, the increase in oxidized species of the IGF-1 is less than 4% upon storage for 6 months at 25°C. In some embodiments, the increase in oxidized species of the IGF-1 is less than 3% upon storage for 6 months at 25°C. In some embodiments, the increase in oxidized species of the IGF-1 is less than 2% upon storage for 6 months at 25°C. In some embodiments, the increase in oxidized species of the IGF-1 is less than 1 % upon storage for 6 months at 25°C. In some embodiments, the increase in oxidized species of the IGF-1 is less than 0.5% upon storage for 6 months at 25°C.

In some embodiments, the increase in oxidized species of the IGF-1 is less than 12% upon storage for 3 months at 40 °C. In some embodiments, the increase in oxidized species of the IGF-1 is less than 10% upon storage for 3 months at 40 °C. In some embodiments, the increase in oxidized species of the IGF-1 is less than 8% upon storage for 3 months at 40 °C. In some embodiments, the increase in oxidized species of the IGF-1 is less than 6% upon storage for 3 months at 40°C. In some embodiments, the increase in oxidized species of the IGF-1 is less than 5% upon storage for 3 months at 40 °C. In some embodiments, the increase in oxidized species of the IGF-1 is less than 4% upon storage for 3 months at 40 °C. In some embodiments, the increase in oxidized species of the IGF-1 is less than 3% upon storage for 3 months at 40 °C. In some embodiments, the increase in oxidized species of the IGF-1 is less than 2% upon storage for 3 months at 40 °C. In some embodiments, the increase in oxidized species of the IGF-1 is less than 1% upon storage for 3 months at 40 °C.

In some embodiments, a single-use bag is further used during a drug product filtration step. In some embodiments, the method comprises filling at least 30% of the single-use bag with the protein complex during the compounding step and/or the filtration step. In some embodiments, the method comprises filling at least 40% of the single-use bag with the protein complex during the compounding step and/or the filtration step. In some embodiments, the method comprises filling 40% of the single-use bag with the protein complex during the compounding step and/or the filtration step. In some embodiments, the method comprises filling 50% of the single-use bag with the protein complex during the compounding step and/or the filtration step. In some embodiments, the method comprises filling 60% of the single-use bag with the protein complex during the compounding step and/or the filtration step. In some embodiments, the method comprises filling 70% of the single-use bag with the protein complex during the compounding step and/or the filtration step. In some embodiments, the method comprises filling at least 75% of the single-use bag with the protein complex during the compounding step and/or the filtration step. In some embodiments, the method comprises filling 80% of the single-use bag with the protein complex during the compounding step and/or the filtration step. In some embodiments, the method comprises filling 85% of the single-use bag with the protein complex during the compounding step and/or the filtration step. In some embodiments, the method comprises filling 90% of the single-use bag with the protein complex during the compounding step and/or the filtration step. In some embodiments, the method comprises filling 95% of the single-use bag with the protein complex during the compounding step and/or the filtration step.

In some embodiments, the method comprises filling 75-95% of the single-use bag with the protein complex during the compounding step and/or the filtration step. In some embodiments, the method comprises filling 75-90% of the single-use bag with the protein complex during the compounding step and/or the filtration step. In some embodiments, the method comprises filling 75-85% of the single-use bag with the protein complex during the compounding step and/or the filtration step. In some embodiments, the method comprises filling 75-80% of the single-use bag with the protein complex during the compounding step and/or the filtration step.

In some embodiments, the method comprises filling 25-80% of the single-use bag with the protein complex during the compounding step and/or the filtration step. In some embodiments, the method comprises filling 25-75% of the single-use bag with the protein complex during the compounding step and/or the filtration step. In some embodiments, the method comprises filling 25-70% of the single-use bag with the protein complex during the compounding step and/or the filtration step. In some embodiments, the method comprises filling 25-65% of the single-use bag with the protein complex during the compounding step and/or the filtration step. In some embodiments, the method comprises filling 25-60% of the single-use bag with the protein complex during the compounding step and/or the filtration step. In some embodiments, the method comprises filling 25-55% of the single-use bag with the protein complex during the compounding step and/or the filtration step. In some embodiments, the method comprises filling 25-50% of the single-use bag with the protein complex during the compounding step and/or the filtration step.

In some embodiments, the single-use bag is about or greater than 500L. In some embodiments, the single-use bag is about or greater than 10L. In some embodiments, the single-use bag is about or greater than 50L. In some embodiments, the single-use bag is about or greater than 100L. In some embodiments, the single-use bag is about or greater than 150L. In some embodiments, the single-use bag is about or greater than 200L. In some embodiments, the single-use bag is about or greater than 250L. In some embodiments, the single-use bag is about or greater than 300L. In some embodiments, the single-use bag is about or greater than 350L. In some embodiments, the single-use bag is about or greater than 400L. In some embodiments, the single-use bag is about or greater than 450L. In some embodiments, the single-use bag is about or greater than 500L. In some embodiments, the single-use bag is about or greater than 550L.

In some embodiments, less than 25 % of the protein complex exists as low molecular weight species upon storage for 6 months at 40 °C. In some embodiments, less than 20 % of the protein complex exists as low molecular weight species upon storage for 6 months at 40 °C. In some embodiments, less than 18 % of the protein complex exists as low molecular weight species upon storage for 6 months at 40 °C. In some embodiments, less than 15 % of the protein complex exists as low molecular weight species upon storage for 6 months at 40 °C. In some embodiments, less than 12 % of the protein complex exists as low molecular weight species upon storage for 6 months at 40 °C. In some embodiments, less than 10 % of the protein complex exists as low molecular weight species upon storage for 6 months at 40 °C. In some embodiments, less than 5 % of the protein complex exists as low molecular weight species upon storage for 6 months at 40 °C.

In some embodiments, less than 15 % of the protein complex exists as low molecular weight species upon storage for 3 months at 40 °C. In some embodiments, less than 10 % of the protein complex exists as low molecular weight species upon storage for 3 months at 40 °C. In some embodiments, less than 5 % of the protein complex exists as low molecular weight species upon storage for 3 months at 40 °C.

In some embodiments, less than 15 % of the protein complex exists as low molecular weight species upon storage for 1 month at 40 °C. In some embodiments, less than 15 % of the protein complex exists as low molecular weight species upon storage for 8 months at 40 °C. In some embodiments, less than 15 % of the protein complex exists as low molecular weight species upon storage for 10 months at 40 °C. In some embodiments, less than 15 % of the protein complex exists as low molecular weight species upon storage for 12 months at 40 °C.

In some embodiments, a pharmaceutical composition comprising an isolated protein complex comprising insulin-like growth factor 1 (IGF-1) and insulin-like growth factor binding protein 3 (IGFBP-3) is manufactured by the method of aspects or embodiments described above. In some embodiments, the composition comprises a surfactant, e.g., a polysorbate surfactant.

In some aspects, provided herein is a pharmaceutical composition comprising an isolated protein complex comprising insulin-like growth factor 1 (IGF-1) and insulin-like growth factor binding protein 3 (IGFBP-3) manufactured by a method comprising use of a single-use bag.

In some embodiments, the pharmaceutical composition comprises 50 mM sodium acetate, 105 mM sodium chloride, and 0.005% (v/v) P20 at pH 5.5.

In some embodiments, the sodium acetate or acetic acid is at a concentration of between about 10 and 100 mM. In some embodiments, the sodium acetate or acetic acid is at a concentration of about 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM. In some embodiments, the sodium acetate or acetic acid is at a concentration of about 50 mM.

In some embodiments, the sodium chloride is at a concentration of about 20 mM and 200 mM. In some embodiments, the sodium chloride is at a concentration of about 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 155 mM, about 160 mM, about 165 mM, about 170 mM, about 175 mM, about 180 mM, about 185 mM, about 190 mM, about 195 mM or about 200 mM. In some embodiments, the sodium chloride is at a concentration of about 105 mM.

In some embodiments, the pharmaceutical composition is at a pH between about 5.0 and 7.0. In some embodiments, the pH is about 5.0, about 5.2, about 5.4, about 5.6, about 5.8, about 6.0, about 6.2, about 6.4, about 6.6, about 6.8 or about 7.0. In some embodiments, the composition is at a pH of about 5.1, about 5.3, about 5.5, about 5.7 or about 5.9, about 6.1, about 6.3, about 6.5, about 6.7 or about 6.9. In some embodiments, the composition is at a pH of between about 5.3 and 5.8. In some embodiments, the composition is at a pH of about 5.5.

In some aspects, provided herein is a pharmaceutical composition comprising a protein complex comprising recombinant insulin-like growth factor 1 (rIGF-1) and recombinant insulin-like growth factor binding protein 3 (rIGFBP-3) in equimolar amounts, polysorbate 20 surfactant at a concentration of about 0.005%, and a buffer comprising sodium acetate, acetic acid, and/or sodium chloride, wherein the pharmaceutical composition has a pH of about 5.3-5.8, wherein the rIGF-1/IGFBP-3 is at a concentration of about 50 micrograms/mL, and wherein less than 2 % of the IGF-1 exists as oxidized species.

In some aspects, a pharmaceutical composition is provided comprising a protein complex comprising recombinant insulin-like growth factor 1 (rIGF-1) and recombinant insulin-like growth factor binding protein 3 (rIGFBP-3) in a range of 0.75 to 1.25: 1 or 1: 0.75-1.25, for example, in equimolar amounts, polysorbate 20 surfactant at a concentration of about 0.005%, and a buffer comprising sodium acetate, acetic acid, and/or sodium chloride, wherein the composition has a pH of about 5.3-5.8, wherein the rIGF-1/IGFBP-3 is at a concentration of about 50 micrograms/mL, and wherein less than 1.5 % of the IGF-1 exists as oxidized species. It is to be understood that while the invention has been described in conjunction with the preferred specific embodiments thereof that the foregoing description as well as the examples that follow are intended to illustrate and not limit the scope of the invention. Other aspects, advantages and modifications within the scope of the invention will be apparent to those skilled in the art to which the invention pertains.

### BRIEF DESCRIPTION OF THE DRAWING

Drawings are for illustration purposes only; not for limitation.
**FIG. 1** is a photograph of components of the Swedish Model Infusion Set used in the IGF-1/IGFBP-3 infusion studies.
**FIG. 2** is a graph of protein concentration of IGF-1/IGFBP-3 drug product with and without 0.005% polysorbate 20 before and after buffer correction.
**FIG. 3A** is a graph that shows purity of samples measured by SE-HPLC in the absence of polysorbate 20. **FIG. 3B** depicts SE-HPLC profiles of samples in the presence of polysorbate 20. Percent peak areas are shown for an average of two infusion sets.
**FIG. 4** is a graph of oxidation of IGF-1 in the IGF-1/IGFBP-3 formulation in the presence and absence of P20 over 48 hours, as measured by RP-UPLC.
**FIG. 5** is a graph showing the percentage of oxidized IGF-1 as a function of time, concentration of the drug product and material used.
**FIG. 6** is a graph of percent oxidized IGF-1 as a function of time and head space for single-use bag and glass containers.
**FIG. 7A** is a graph showing stability (percent main peak) of the IGF-1/IGFBP-3 as a function of time at between 2°C -8°C (e.g. 5°C). **FIG. 7B** is a graph showing stability (percent main peak) of the IGF-1/IGFBP-3 as a function of time at 23°C to 27°C (25°C). **FIG. 7C** is a graph showing stability (percent main peak) of the IGF-1/IGFBP-3 as a function of time at between 38°C to 42°C.
**FIG. 8A** is a graph of percent oxidized IGF-1 plotted as function of time at 5°C. **FIG. 8B** is a graph of percent oxidized IGF-1 plotted as function of time at 25°C. **FIG. 8C** is a graph of percent oxidized IGF-1 plotted as function of time at 40°C.
**FIG. 9** depicts graphs of IGF-1 concentration over time for samples treated with either 250 micrograms/kg/24 hours or 400 micrograms/kg/24 hours of IGF-1/IGFBP-3 as compared to subjects receiving standard of care.
**FIG. 10** shows graphs that validate the simulation model based on simulated and observed IGF-1 concentration in untreated controls, subjects receiving standard of care, and subjects receiving treatment with rIGF-1/IGFBP-3 at a low dose or at 250 micrograms/kg/24hrs.
**FIG. 11** is a graph that shows results of a clinical trial simulation wherein the probability of subjects developing no bronchopulmonary dysplasia (BPD) or mild BPD was determined based on serum IGF-1 exposure levels as a result of treatment with either 250 micrograms/kg/24 hours or 400 micrograms/kg/24 hours of IGF-1/IGFBP-3 as compared to subjects receiving standard of care.
**FIG. 12** is a model of simulated 95% prediction intervals of IGF-1 PK concentrations over duration of treatment to predict where the mean and 5th and 95th intervals would fall with respect to target therapeutic range (28-109 ng/ml) in subjects treated with either 250 micrograms/kg/24 hours or 400 micrograms/kg/24 hours of IGF-1/IGFBP-3 as compared to subjects receiving standard of care at day 7 and at end of infusion.
**FIG. 13A** is a graph of serum IGF-1 exposure at day 7 and probability of BPD outcome (mild or no BPD). **FIG. 13B** is a graph of serum IGF-1 exposure at 40 weeks PMA and probability of BPD outcome (mild or no BPD).

### DETAILED DESCRIPTION

The present invention provides, in some aspects, a composition optimized for administration to neonates and/or preterm infants, and methods for treating diseases and complications of prematurity, including, among others, Intraventricular Hemorrhage (IVH), Bronchopulmonary Dysplasia (BPD), and Chronic Lung Disease of prematurity. The compositions and methods provided herein are particularly effective in treating Intraventricular Hemorrhage, Bronchopulmonary Dysplasia and/or Chronic Lung Disease in premature infants, especially extremely premature infants. In some embodiments of the invention, provided herein is a low concentration pharmaceutical composition suitable for administration of a therapeutic complex to a neonate and/or preterm infant comprising an insulin-like growth factor-1 (IGF-1), an insulin-like growth factor binding protein-3 (IGFBP-3) complexed, and a non-ionic surfactant (*e.g.* a polysorbate surfactant such as polysorbate 20 or polysorbate 80, in particular polysorbate 20, at a concentration of between about 0.0025% v/v and 0.0075% v/v (*e.g*. 0.005% v/v)). In some embodiments, the polysorbate surfactant is polysorbate 20 (P20) or polysorbate 80 (P80). In some embodiments, the rIGF-1 and rIGFBP-3 are complexed in a range of 0.75 to 1.25: 1 or 1: 0.75-1.25, for example, equimolar amounts, and the rIGF-1/IGFBP-3 is at a concentration of about 10 micrograms/mL - 1000 micrograms/mL (*e.g.* 50 micrograms/mL). In some aspects, a method of the invention involves administering to a subject in need of treatment (*e.g.,* a premature infant) aforesaid pharmaceutical composition. In further independent aspect, provided herein is a process for manufacturing the formulation of the composition, for example, employing a single use bag, in particular, minimizing the contact of the formulation with stainless steel, to provide a robust process for achieving formulations having reduced oxidation.

Various aspects of the invention are described in detail in the following sections. The use of sections is not meant to limit the invention. Each section can apply to any aspect of the invention. In this application, the use of "or" means "and/or" unless stated otherwise.

### Definitions

"Preterm" or "preterm birth" or "prematurity" or "premature infant" or "premature baby", or grammatical equivalents, refers to birth of an infant prior to 37 weeks of gestation or weighing 10% less than the average for the infant's gestational age. For example, infants born between 22-37 weeks would be considered preterm. "GA" or "Gestational age" is a common term to describe how far along a pregnancy has progressed, measured in weeks from the first day of the woman's last menstrual cycle to the current date. In some embodiments, a premature infant refers to an infant that was prematurely born by at least 1 week, 2 weeks, 3 weeks, 4 weeks, 1 month, 2 months, or 3 months. In some embodiments, a premature infant refers to an infant born at less than 32 weeks of gestational age (GA). In some embodiments, a premature infant refers to an infant born at less than 28 weeks of gestational age (GA). "PMA" or "postmenstrual age" refers to gestational age plus chronological age. "CA" or "corrected age" is the chronological age reduced by the number of weeks born before 40 weeks of gestation.

As used herein, the term "Gestational age" (completed weeks) means: time elapsed between the first day of the last menstrual period and the day of delivery. If pregnancy was achieved using assisted reproductive technology, gestational age is calculated by adding 2 weeks to the conceptional age.

As used herein, the term "Chronological age" (days, weeks, months, or years) means: time elapsed from birth.

As used herein, the term "Postmenstrual age" (weeks) means: gestational age plus chronological age.

As used herein, the term "Corrected age" (weeks or months) means: chronological age reduced by the number of weeks born before 40 weeks of gestation; the term should be used only for children up to 3 years of age who were born preterm.

During the perinatal period neonatal hospital stay, "postmenstrual age" is preferred to describe the age of preterm infants. After the perinatal period, "corrected age" is the preferred term.

"IGF-I" refers to insulin-like growth factor I from any species, including bovine, ovine, porcine, equine, and human, preferably human, and, if referring to exogenous administration, from any source, whether natural, synthetic, or recombinant, provided that it will bind IGF binding protein at the appropriate site. IGF-I can be produced recombinantly, for example, as described in PCT publication WO 95/04076.

An "IGFBP" or an "IGF binding protein" refers to a protein or polypeptide from the insulin-like growth factor binding protein family and normally associated with or bound or complexed to IGF-I whether or not it is circulatory (i.e., in serum or tissue). Such binding proteins do not include receptors. This definition includes IGFBP-1, IGFBP-2, IGFBP-3, IGFBP-4, IGFBP-5, IGFBP-6, Mac 25 (IGFBP-7), and prostacyclin-stimulating factor (PSF) or endothelial cell-specific molecule (ESM-1), as well as other proteins with high homology to IGFBPs. Mac 25 is described, for example, in Swisshelm et al., Proc. Natl. Acad. Sci. USA, 92: 4472-4476 (1995) and Oh et al., J. Biol. Chem., 271: 30322-30325 (1996). PSF is described in Yamauchi et al., Biochemical Journal, 303: 591-598 (1994). ESM-1 is described in Lassalle et al., J. Biol. Chem., 271: 20458-20464 (1996). For other identified IGFBPs, see, e.g., EP 375,438 published Jun. 27, 1990; EP 369,943 published May 23, 1990; WO 89/09268 published Oct. 5, 1989; Wood et al., Molecular Endocrinology, 2: 1176-1185 (1988); Brinkman et al., The EMBO J., 7: 2417-2423 (1988); Lee et al., Mol. Endocrinol., 2: 404-411 (1988); Brewer et al., BBRC, 152: 1289-1297 (1988); EP 294,021 published Dec. 7, 1988; Baxter et al., BBRC, 147: 408-415 (1987); Leung et al., Nature, 330: 537-543 (1987); Martin et al., J. Biol. Chem., 261: 8754-8760 (1986); Baxter et al., Comp. Biochem. Physiol., 91B: 229-235 (1988); WO 89/08667 published Sep. 21, 1989; WO 89/09792 published Oct. 19, 1989; and Binkert et al., EMBO J, 8: 2497-2502 (1989).

"IGFBP-3" refers to insulin-like growth factor binding protein 3. IGFBP-3 is a member of the insulin-like growth factor binding protein family. IGFBP-3 may be from any species, including bovine, ovine, porcine and human, in native-sequence or variant form, including but not limited to naturally-occurring allelic variants. IGFBP-3 may be from any source, whether natural, synthetic or recombinant, provided that it will bind IGF-I at the appropriate sites. IGFBP-3 can be produced recombinantly, as described in PCT publication WO 95/04076.

A "therapeutic composition," as used herein, is defined as comprising IGF-I, an analog thereof, or IGF-I in combination with its binding protein, IGFBP-3 (IGF-I/IGFBP-3 complex). The therapeutic composition may also contain other substances such as water, minerals, carriers such as proteins, and other excipients known to one skilled in the art. In some embodiments, the therapeutic composition comprises a surfactant such as polysorbate 20 (P20) or polysorbate 80 (P80).

"Analogs" of IGF-I are compounds having the same therapeutic effect as IGF-I in humans or animals. These can be naturally occurring analogs of IGF-I (e.g., truncated IGF-I) or any of the known synthetic analogs of IGF-I. See, for example, U.S. Pat. No. 5,473,054 for analog compounds of IGF-I.

"Agonists" of IGF-I are compounds, including peptides, which are capable of increasing serum and tissue levels of IGF, especially IGF-I, in a mammal and particularly in a human. See, for example, U.S. Pat. No. 6,251,865 for IGF agonist molecules.

"Developmental delay" as used herein shall mean abnormal neurogenesis which has the potential of leading to slowed mental progression in achieving developmental milestones. Developmental delay can, in some cases, be determined by means of electroencephalogram.

"Subject" as used herein means any mammal, including humans. In certain embodiments of the present invention the subject is an adult, an adolescent or an infant. Also contemplated by the present invention are the administration of the pharmaceutical compositions and/or performance of the methods of treatment in-utero.

As used herein, the term "treatment" (also "treat" or "treating") refers to any administration of a therapeutic composition (e.g., IGF-1/IGFBP-3) that partially or completely alleviates, ameliorates, relieves, inhibits, delays onset of, prevents, reduces severity of and/or reduces incidence of one or more symptoms or features of a particular disease, disorder, and/or condition (e.g., Intraventricular Hemorrhage, Bronchopulmonary Dysplasia, Chronic lung disease of prematurity). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. Accordingly, in some embodiments, treatment means preventing onset or progression of a disease.

"Baseline levels of IGF-1" refers to the serum levels of IGF-1 in a subject before receiving any treatment.

"Storage life" or "shelf life" refers to a period of storage of drug product when it retains the characteristics and properties making it suitable for therapeutic use. For example, in some embodiments, during the storage life of the drug product, there are reduced amounts (e.g. below 10%) of undesirable species including oxidized species, high-molecular weight species, degradation products. Storage life is described, for example, at particular temperatures of storage (e.g. 5 °C, 25 °C, 40 °C, *etc*.).

"Stability" or "stable" refers to the extent to which a drug product retains the characteristics and properties making it suitable for therapeutic use possessed at the time of manufacture during its period of storage and use. Stability includes aspects with regard to its formulation, the stability of IGF-1 and IGFBP3 ingredients, the integrity and stability of the IGF-1/IGFBP3 complex, container and closure, manufacturing and processing conditions, packaging components, storage and shipping conditions, temperature, light, and humidity, and the anticipated duration and conditions of pharmacy shelf-life and patient use. In some embodiments, stability refers to reduced amounts of undesirable species including oxidized species, reduced high molecular weight species that result in aggregation and/or reduced low molecular weight species represented by degradation products. In some embodiments, stability refers to less than 10% of undesirable species. In some embodiments, stability refers to less than 5% of undesirable species. In some embodiments, stability refers to less than 2% of undesirable species.

"Standard Neonatal Care" or "Standard of Care" refers to standard care at a level III neonatal intensive care unit (NICU) described by the American Academy of Pediatrics for infants born below 32 weeks gestational age.

"Standard of Care for CLD and/or BPD" refers to treatment measures including respiratory support, such as supplemental oxygen, continuous positive airway pressure, and mechanical ventilation with endotracheal intubation. Symptoms are managed with medications such as bronchodilators to help open the airways, steroids to help reduce inflammation, diuretics to help reduce excess fluid in the lungs, vasodilators to help decrease blood pressure in the lungs and antibiotics to fight an infection.

"Standard of Care for IVH" refers to preventive and/or treatment measures including antenatal steroids and the early management and stabilization of cerebral hemodynamics and respiratory support. IVH treatment refers to supportive management of symptoms with medications to reduce bleeding and damage to the brain, and prevent seizures.

As used herein, the terms "improve," "increase" or "reduce," or grammatical equivalents, indicate values that are relative to a baseline measurement, such as a measurement in the same individual prior to initiation of the treatment described herein, or a measurement in a control individual (or multiple control individuals) in the absence of the treatment described herein, or historical reference or data. A "control individual" is an individual afflicted with the same form of disease (e.g., IVH, BPD, CLD, among others) of prematurity as the individual being treated, who is about the same age as the individual being treated (to ensure that the stages of the disease in the treated individual and the control individual(s) are comparable).

### IGF-1/IGFBP-3

IGF-1 or an agonist or an analog thereof may be used to practice the present invention. IGF-I is a well-known regulator of postnatal growth and metabolism. See, Baker J, Liu J P, Robertson E J, Efstratiadis A. It has a molecular weight of approximately 7.5 kilodaltons (Kd). Most circulating IGF is bound to the IGF-binding protein, and more particularly to IGFBP-3. IGF-I may be measured in blood serum to diagnose abnormal growth-related conditions.

Typically, a therapeutic composition suitable for treatment of the diseases described herein, including for example IVH, BPD or CLD, contains an IGF-1 and an IGF-1 binding protein such as IGF binding-proteins (IGFBPs). At least six distinct IGF binding-proteins (IGFBPs) have been identified in various tissues and body fluids. In some embodiments, a suitable therapeutic composition according to the present invention contains IGF-1 and IGFBP-3. IGF-1 and IGFBP-3 may be used as a protein complex or separately. In some embodiments, IGF-1 and IGFBP-3 are complexed in equimolar amounts. In some embodiments, a therapeutic composition comprises mecasermin rinfabate. In some embodiments, a therapeutic composition comprises mecasermin rinfabate and a surfactant. In some embodiments, a therapeutic composition comprises mecasermin rinfabate and polysorbate 20. In some embodiments, a therapeutic composition comprises mecasermin rinfabate and polysorbate 80.

IGF-I and IGF-I binding proteins such as IGFBP-3 may be purified from natural sources or produced by recombinant means. For instance, purification of IGF-I from human serum is well known in the art (Rinderknecht et al. (1976) Proc. Natl. Acad. Sci. USA 73:2365-2369). Production of IGF-I by recombinant processes is shown in EP 0128733, published in December of 1984. IGFBP-3 may be purified from natural sources using a process such as that shown by Baxter et al. (1986, Biochem. Biophys. Res. Comm. 139:1256-1261). Alternatively, IGFBP-3 may be synthesized recombinantly as discussed by Sommer et al., pp. 715-728, Modern Concepts Of Insulin-Like Growth Factors (E. M. Spencer, ed., Elsevier, N.Y., 1991). Recombinant IGFBP-3 binds IGF-I in a 1:1 molar ratio (equimolar amounts).

### Pharmaceutical composition and therapeutic use

The present invention provides a pharmaceutical composition suitable for administration of a therapeutic protein complex to a neonate comprising insulin-like growth factor 1 and insulin-like growth factor binding protein 3 complexed (for example in a range 0.75 to 1.25: 1 or 1: 0.75-1.25 such as in equimolar amounts), and a non-ionic surfactant (for example, a polysorbate surfactant, such as polysorbate 20 or polysorbate 80, in particular polysorbate 20) in the 0.0025% to 0.0075%. In one embodiment, the pharmaceutical composition is isotonic. In one embodiment, the pharmaceutical composition is liquid. In one embodiment, the pharmaceutical composition is aqueous.

The present invention also provides methods for treating (e.g. ameliorating or reducing risk of) diseases and complications of prematurity, for example, Intraventricular Hemorrhage (IVH), Bronchopulmonary Dysplasia (BPD) or Chronic Lung Disease (CLD), such as for example, CLD associated with prematurity. For example, in some embodiments, the present invention may be used to treat a premature infant, e.g., to ameliorate or reduce the risk of IVH or complication associated with IVH. In some other embodiments, the present invention may be used to treat a premature infant, e.g., to ameliorate or reduce the risk of CLD or complication associated with CLD. In further embodiments, the present invention may be used to treat a premature infant, e.g., to ameliorate or reduce the risk of BPD or complication associated with BPD. In some embodiments, the present invention may be used to treat an infant who is prematurely born by at least 1 week, 2 weeks, 3 weeks, 4 weeks, 1 month, 2 months, or 3 months. In some embodiments, the present invention may be used to treat an extremely premature infant.

In one embodiment of the invention, a pharmaceutical composition comprising IGF-I is administered in combination with IGF binding protein capable of binding IGF-I, and a surfactant. In some embodiments, the IGF binding protein capable of binding IGF-I is IGF binding protein 3 (IGFBP-3). In some embodiments, the composition comprises a surfactant selected from polysorbate 20 or polysorbate 80.

In some embodiments, the concentration of the therapeutic complex comprising rIGF-1/IGFBP-3 is in the range of 10 to 1000 micrograms/mL (for example, 10 to 100 micrograms/mL, 15, 20, 25, 30, 35, 40, 45, 50, 55, 60, 65, 70, 75, 80, 85, 90, 95 or 100 micrograms/mL) such as 45-55 micrograms/mL in particular 50 micrograms/mL.

In some embodiments, a pharmaceutical composition comprising rIGF-1/IGFBP-3 is at a concentration of between about 10 micrograms/mL - 1000 micrograms/mL. In some embodiments, a pharmaceutical composition comprising rIGF-1/IGFBP-3 is at a concentration of between about 10 micrograms/mL - 100 micrograms/mL. For example, in some embodiments, a pharmaceutical composition comprising rIGF-1/IGFBP-3 is at a pharmaceutical concentration of between about 45 - 55 micrograms/mL. In some embodiments, a pharmaceutical composition comprising rIGF-1/IGFBP-3 is at a concentration of about 50 micrograms/mL. In some embodiments, a pharmaceutical composition comprising rIGF-1/IGFBP-3 at a pharmaceutical concentration of about 10 - 100 micrograms/mL is suitable for intravenous administration. In some embodiments, a pharmaceutical composition comprising rIGF-1/IGFBP-3 at a concentration of about 45 - 55 micrograms/mL is suitable for intravenous administration. In some embodiments, a pharmaceutical composition comprising rIGF-1/IGFBP-3 at a concentration of about 50 micrograms/mL is suitable for intravenous administration.

In some embodiments, a pharmaceutical composition comprising rIGF-1/IGFBP-3 is at a concentration of between about 1000 micrograms/mL - 5000 micrograms/mL. For example, in some embodiments, a pharmaceutical composition comprising rIGF-1/IGFBP-3 is at a concentration of between 2000 micrograms/mL - 3000 micrograms/mL. In some embodiments, a pharmaceutical composition comprising rIGF-1/IGFBP-3 is at a concentration of about 2500 micrograms/mL. In some embodiments, a pharmaceutical composition comprising rIGF-1/IGFBP-3 at a concentration of between about 1000 - 5000 micrograms/mL is suitable for subcutaneous administration. In some embodiments, a pharmaceutical composition comprising rIGF-1/IGFBP-3 at a concentration of between about 2000 micrograms/mL - 3000 micrograms/mL is suitable for subcutaneous administration. In some embodiments, a pharmaceutical composition comprising rIGF-1/IGFBP-3 at a concentration of about 2500 micrograms/mL is suitable for subcutaneous administration.

In some embodiments, a pharmaceutical composition comprising IGF-1 and IGFBP-3, in a range of 0.75 to 1.25: 1 or 1: 0.75-1.25, for example, equimolar amounts of IGF-I and IGF-binding protein 3 may be used. In some embodiments, the IGF-I and IGF binding protein 3 are complexed prior to administration. The complex may be formed by mixing approximately equimolar amounts of IGF-I and IGF binding protein 3 dissolved in physiologically compatible carriers such as normal saline, or phosphate buffered saline solution. In some embodiments, a concentrated solution of recombinant human IGF-I and a concentrated solution of recombinant human IGF binding protein 3 are mixed together for a sufficient time to form a complex in a range of 0.75 to 1.25: 1 or 1: 0.75-1.25, for example, in an equimolar complex. In some embodiments, recombinant human IGF-I and recombinant human IGF binding protein 3 are combined to form a complex during purification as described in International Patent Application No. WO 96/40736.

In some embodiments, a pharmaceutical composition is provided comprising the composition described herein and one or more suitable pharmaceutical excipients. In one embodiment, the composition has a minimal number and/or quantity of excipients, for example, 1, 2, 3, 4 or 5 excipients.

In some embodiments, the pharmaceutical composition comprises polysorbate 20. In some embodiments, the polysorbate 20 surfactant is at a concentration of between about 0.001% to 2.4% v/v. In some embodiments, the polysorbate 20 surfactant is at a concentration of between about 0.2% to 0.4% v/v. In some embodiments, polysorbate 20 surfactant is at a concentration of about 0.001%, 0.005%, 0.01%, 0.02%, 0.03%, 0.05%, 0.10%, 0.15%, 0.2%, 0.5%, 0.7%, 1.0%, 1.2%, 1.5%, 1.8%, 2.0%, 2.2% or 2.4% v/v. In some embodiments, the polysorbate 20 surfactant is at a concentration of about 0.0025%, about 0.005%, or about 0.0075% v/v. In some embodiments, the polysorbate 20 is at a concentration of 0.005%.

In some embodiments, the pharmaceutical composition comprises polysorbate 80. In some embodiments, the polysorbate 80 surfactant is at a concentration of between about 0.001% to 2.4% v/v. In some embodiments, the polysorbate 80 surfactant is at a concentration of between about 0.2% to 0.4% v/v. In some embodiments, polysorbate 80 surfactant is at a concentration of about 0.001%, 0.005%, 0.01%, 0.02%, 0.03%, 0.05%, 0.10%, 0.15%, 0.2%, 0.5%, 0.7%, 1.0%, 1.2%, 1.5%, 1.8%, 2.0%, 2.2% or 2.4% v/v. In some embodiments, the polysorbate 80 surfactant is at a concentration of about 0.0025%, about 0.005%, or about 0.0075% v/v. In some embodiments, the polysorbate 80 is at a concentration of 0.005%.

In some embodiments, the surfactant % is w/w.

In one embodiment, the surfactant % is w/v, i.e., weight of the composition to volume of the surfactant or weight of the surfactant to volume of the composition.

In one embodiment, the surfactant % is v/v.

In some embodiments, the pharmaceutical composition further comprises a buffer comprising sodium acetate, acetic acid and/or sodium chloride. In some embodiments, the composition further comprises a buffer comprising sodium acetate or acetic acid. In some embodiments, the composition further comprises a buffer comprising sodium chloride.

In some embodiments, the sodium acetate or acetic acid is at a concentration of between about 10 and 100 mM. In some embodiments, the sodium acetate or acetic acid is at a concentration of about 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM or 100 mM. In some embodiments, the sodium acetate or acetic acid is at a concentration of about 50 mM.

In some embodiments, the sodium chloride is at a concentration of about 20 mM and 200 mM. In some embodiments, the sodium chloride is at a concentration of about 20 mM, about 25 mM, 30 mM, 35 mM, 40 mM, 45 mM, 50 mM, 55 mM, 60 mM, 65 mM, 70 mM, 75 mM, 80 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 155 mM, about 160 mM, about 165 mM, about 170 mM, about 175 mM, about 180 mM, about 185 mM, about 190 mM, about 195 mM, or about 200 mM. In some embodiments, the sodium chloride is at a concentration of about 105 mM.

In some embodiments, the composition is at a pH between about 5.0 and 6.0. In some embodiments, the pH is about 5.0, about 5.2, about 5.4, about 5.6, about 5.8, about 6.0, about 6.2, about 6.4, about 6.6, about 6.8 or about 7.0. In some embodiments, the composition is at a pH of about 5.1, about 5.3, about 5.5, about 5.7, about 5.9, about 6.1, about 6.3, about 6.5, about 6.7, or about 6.9. In some embodiments, the composition is at a pH of about 5.3 to 5.8, *e.g.* 5.3, 5.4, 5.5, 5.6, 5.7, 5.8, in particular, pH 5.5.

The pharmaceutical compositions of the invention include those suitable for oral, nasal, topical (including buccal and sublingual), or parenteral (including subcutaneous, intramuscular, intravenous and intradermal) administration. The formulations may conveniently be presented in unit dosage form, e.g., tablets and sustained release capsules, and in liposomes, and may be prepared by any methods well known in the art of pharmacy. See, for example, Remington's Pharmaceutical Sciences, Mack Publishing Company, Philadelphia, Pa. (17th ed. 1985). In some embodiments, the composition is administered intravenously.

Such preparative methods include the step of bringing into association with the molecule to be administered ingredients such as the carrier which constitutes one or more accessory ingredients. In general, the compositions are prepared by uniformly and intimately bringing into association the active ingredients with liquid carriers, liposomes or finely divided solid carriers or both, and then if necessary shaping the product.

Compositions of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion, or packed in liposomes and as a bolus, etc.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets optionally may be coated or scored and may be formulated so as to provide slow or controlled release of the active ingredient therein.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

In some embodiments, the methods disclosed herein provide for the parenteral administration of a low concentration isotonic pharmaceutical composition suitable for administration of a therapeutic protein complex to a neonate (in particular in an active form) comprising IGF-I complexed with IGF binding protein 3 in the presence of a non-ionic surfactant, e.g., 0.005% polysorbate 20 or polysorbate 80 to infants in need of such treatment.

In one embodiment, the pharmaceutical formulation can be administered parenterally without loss of the therapeutic protein complex (such as without loss of the activity of the therapeutic protein complex). In one embodiment, activity is measured by an assay disclosed herein, such as in the examples. In one embodiment, the formulation delivers at least 80% of the dose of the therapeutic protein complex to the neonate in an active form, for example, 81, 82, 83, 84, 85, 86, 87, 88, 89, 90, 91, 92, 93, 94, 95, 96, 97, 98, 99 or 100%.

Parenteral administration includes, but is not limited to, intravenous (IV), intramuscular (IM), subcutaneous (SC), intraperitoneal (IP), intranasal, and inhalant routes. In some embodiments, the IGF-I/IGFBP-3 pharmaceutical composition is administered intravenously. IV, IM, SC, and IP administration may be by bolus or infusion, and may also be by slow release implantable device, including, but not limited to pumps, slow release formulations, and mechanical devices. The formulation, route and method of administration, and dosage will depend on the disorder to be treated and the medical history of the patient. Accordingly, in some embodiments, the methods disclosed herein provide for the intravenous administration of a pharmaceutical composition comprising IGF-I complexed with IGF binding protein 3 in the presence of a surfactant, e.g., 0.005% v/v polysorbate 20 or polysorbate 80 to infants in need of such treatment. In some embodiments, the methods disclosed herein provide for the subcutaneous administration of a pharmaceutical composition comprising IGF-I complexed with IGF binding protein 3 in the presence of a surfactant, e.g., 0.005% v/v polysorbate 20 or polysorbate 80 to infants in need of such treatment.

A pharmaceutical composition according to the present invention may be administered at various doses. For example, a suitable dosage may range from about 100 to 1000 micrograms/kg/24 hours. In some embodiments, a suitable dosage may be or greater than about 100 micrograms/kg/24 hours, 150 micrograms/kg/24 hours, 200 micrograms/kg/24 hours, 250 micrograms/kg/24 hours, 300 micrograms/kg/24 hours, 350 micrograms/kg/24 hours, 400 micrograms/kg/24 hours, 450 micrograms/kg/24 hours, 500 micrograms/kg/24 hours, 550 micrograms/kg/24 hours, 600 micrograms/kg/24 hours, 650 micrograms/kg/24 hours, 700 micrograms/kg/24 hours, 750 micrograms/kg/24 hours, 800 micrograms/kg/24 hours, 850 micrograms/kg/24 hours, 900 micrograms/kg/24 hours, 950 micrograms/kg/24 hours or 1000 micrograms/kg/24 hours. In some embodiments, a suitable dosage is about 250 micrograms/kg/24 hours. In some embodiments, a suitable dosage is about 400 micrograms/kg/24 hours. In some embodiments, a suitable dosage is about 750 micrograms/kg/24 hours. In some embodiments, a suitable dosage is about 1000 micrograms/kg/24 hours. In some embodiments, a pharmaceutical composition according to the invention is administered from the time of birth up to postmenstrual age (PMA) of about 23 to 34 weeks, up to PMA of about 28 to 32 weeks, up to PMA of about 29 weeks plus 6 days.

The method provided herein comprises embodiments where the administration of the pharmaceutical IGF-I/IGFBP-3 composition results in reduced incidence of chronic lung disease of prematurity through 12 months corrected age (CA). In some embodiments, the administration of the pharmaceutical IGF-I/IGFBP-3 composition results in reduced incidence of Bronchopulmonary dysplasia (BPD) through postmenstrual age (PMA) 23 weeks, 26 weeks, 30 weeks, 34 weeks, 36 weeks, 40 weeks, 6 months, 8 months, 10 months, or 12 months. In some embodiments, the administration of the pharmaceutical IGF-I/IGFBP-3 composition results in reduced incidence of Severe Intraventricular Hemorrhage (IVH) Grade III or IV through postmenstrual age (PMA) 23 weeks, 26 weeks, 30 weeks, 34 weeks, 36 weeks, 40 weeks, 6 months, 8 months, 10 months, or 12 months. In some embodiments, the administration of the pharmaceutical IGF-I/IGFBP-3 composition results in reduced incidence of right ventricular hypertrophy (RVH) through postmenstrual age (PMA) 23 weeks, 26 weeks, 30 weeks, 34 weeks, 36 weeks, 40 weeks, 6 months, 8 months, 10 months, or 12 months. In some embodiments, the administration of the pharmaceutical IGF-I/IGFBP-3 composition results in reduced incidence of pulmonary hypertension (PH) through postmenstrual age (PMA) 23 weeks, 26 weeks, 30 weeks, 34 weeks, 36 weeks, 40 weeks, 6 months, 8 months, 10 months, or 12 months. In some embodiments, the administration of the pharmaceutical IGF-I/IGFBP-3 composition results in reduced incidence of necrotizing enterocolitis through postmenstrual age (PMA) 23 weeks, 26 weeks, 30 weeks, 34 weeks, 36 weeks, 40 weeks, 6 months, 8 months, 10 months, or 12 months.

In some embodiments, the methods disclosed herein comprise embodiments where the administration of the pharmaceutical IGF-I/IGFBP-3 composition results in increased Functional Status as Assessed by PREMature Infant Index (PREMII) through postmenstrual age (PMA) 36 weeks, or 40 weeks.

For parenteral or oral administration, compositions of the complex may be semisolid or liquid preparations, such as liquids, suspensions, and the like. Physiologically compatible carriers are those that are non-toxic to recipients at the dosages and concentrations employed and are compatible with other ingredients of the formulation. For example, the formulation preferably does not include oxidizing agents and other compounds that are known to be deleterious to polypeptides. Hence, physiologically compatible carriers include, but are not limited to, normal saline, serum albumin, 5% dextrose, plasma preparations, and other protein-containing solutions. Optionally, the carrier may also include detergents or surfactants.

In yet another aspect of the invention there is provided use of the IGF-I/IGFBP-3 pharmaceutical composition in the manufacture of a therapeutic composition for treating a complication of preterm birth.

In one preferred embodiment, there is provided a low concentration isotonic pharmaceutical composition suitable for administration of a therapeutic protein complex to a neonate (in particular in an active form) comprising 10 to 1000 micrograms per mL (for example, 10 to 100 micrograms/mL, i.e., for example, 15, 20, 25, 30, 35, 40, 45, 50, 55, 60, 65, 70, 75, 80, 85, 90, 95 or 100 micrograms/mL, such as 45-55 micrograms/mL, in particular 50 micrograms/mL) of complexed Insulin-like growth factor 1 (IGF-1), and Insulin-like growth factor binding protein 3 (IGFBP-3) (for example in a range 0.75 to 1.25: 1 or 1: 0.75-1.25 such as in equimolar amounts; and a non-ionic surfactant (for example a polysorbate surfactant, such as polysorbate 20 or polysorbate 80, in particular polysorbate 20) in the range of 0.0025% to *0.0075% (e.g.* 0.005%) ; a buffer *(e.g.* 50 mM sodium acetate), salt *(e.g.* 105 mM sodium chloride) wherein the pH of the formulation is in the range pH 5.3 to 5.8 *(e.g.* pH 5.5).

### Chronic Lung Disease

### Chronic Lung Disease of Prematurity

Extremely premature infants are at very high risk for developing chronic lung disease of prematurity. Premature babies may need a breathing machine (ventilator) and extra oxygen to breathe. Chronic Lung Disease of prematurity may occur when a breathing machine or oxygen injures a premature baby's lungs. With a lung injury, the tissues inside a baby's lungs get inflamed. The tissue can break down, causing scarring. The scarring can cause trouble breathing, and the baby may need more oxygen. Lung injury may be caused by:
- *Prematurity:* A premature baby's lungs aren't fully formed. This is especially true of the air sacs.
- *Low amounts of surfactant*: This is a substance in the lungs that helps keep the tiny air sacs open.
- *Oxygen use:* High amounts of oxygen can harm the cells in the lungs.
- *Breathing machine (mechanical ventilation):* Air pressure can harm the lungs.
This pressure may come from breathing machines, suctioning of the airways, and use of an endotracheal (ET) tube. An ET tube is a tube placed in your baby's windpipe (trachea) and connected to a breathing machine.

The long term trajectory of pulmonary outcomes in infants born extremely premature commonly starts with antenatal risk factors, followed by respiratory distress syndrome (RDS) in the first hours or days of life requiring respiratory support, often leading up to a diagnosis of BPD in those who survive to term equivalence, and finally chronic lung disease of prematurity as they grow into infancy, early childhood and often even school age or adolescence that results in more frequent re-hospitalizations and ER visits for respiratory causes, the need for respiratory medication or home respiratory support, and many suffer from a form of reactive airway disease that continues to limit their quality of life.

Chronic lung disease of prematurity is a broad term that includes infants diagnosed with bronchopulmonary dysplasia, but may also include infants that never received a diagnosis for bronchopulmonary dysplasia after extremely premature birth and manifest with persistent lung disease. Chronic lung disease of prematurity may be diagnosed by prolonged time to weaning off of oxygen support, but clinical endpoints of chronic lung disease of prematurity can include, in practice, but are not limited to ongoing oxygen dependence, symptoms of cough and wheezing, respiratory illnesses and hospitalizations, dependence on continuous airway pressure, mechanical ventilation and dependence on respiratory medications, and diagnosis of pulmonary hypertension (PH). In some embodiments, Chronic Lung Disease (CLD) of prematurity may persist into adulthood. In some embodiments, CLD involves a spectrum of diseases and disorders, including but not limited to COPD (emphysema and chronic bronchitis), asthma, cystic fibrosis, restrictive lung disease, and persistent infections.

In some embodiments, the compositions and methods described herein result in reduced incidence of CLD. In some embodiments, the compositions and methods described herein are used to treat CLD.

In some aspects, described herein are methods for treating or reducing incidence of CLD comprising administering to a subject in need thereof a composition comprising recombinant insulin-like growth factor 1 (rIGF-1), recombinant insulin-like growth factor binding protein 3 (rIGFBP-3), and a surfactant (e.g., a polysorbate surfactant, e.g., polysorbate 20).

In some aspects, described herein are methods of treating or reducing incidence of CLD comprising administering to a subject in need thereof a composition comprising recombinant insulin-like growth factor 1 (rIGF-1), recombinant insulin-like growth factor binding protein 3 (rIGFBP-3), and a polysorbate surfactant at a concentration of between about 0.0025% and 0.0075% *(e.g.* 0.005%), wherein the rIGF-1 and rIGFBP-3 are complexed in a range of 0.75 to 1.25: 1 or 1: 0.75-1.25, for example, equimolar amounts, and wherein the rIGF-1/IGFBP-3 is at a concentration of about 10 micrograms/mL - 1000 micrograms/mL (*e.g.* 50 micrograms/mL). In some aspects described herein are methods of treating or reducing incidence of CLD comprising administering to a subject in need thereof a composition comprising recombinant insulin-like growth factor 1 (rIGF-1), recombinant insulin-like growth factor binding protein 3 (rIGFBP-3), at a therapeutic amount sufficient to achieve reduced incidence of CLD relative to a control.

In some embodiments, reduced incidence or treatment of CLD is assessed by the Time to Final Weaning off Respiratory Technology Support (RTS) from Day 1 through 12 Months Corrected Age (CA) (Time Frame: Baseline through 12 months Corrected Age (CA) ). In this context, RTS is defined as any one of the following: (1) supplemental oxygen less than (<) 2 Liter per minute (L/min) without positive pressure (including nasal cannula), (2) positive pressure support (including continuous positive airway pressure [CPAP], nasal cannula oxygen greater than (>) 2 L/min), (3) positive pressure ventilation (high frequency oscillation ventilation and technologies with positive pressure tidal volume breaths, such as mechanical ventilation, nasal intermittent positive pressure ventilation [NIPPV]). In some embodiments, the compositions and methods described herein result in reduced incidence of CLD as assessed by the Time to Final Weaning off RTS as compared to a baseline control.

In some embodiments, reduced incidence or treatment of CLD is assessed by the Incidence of Bronchopulmonary Dysplasia (BPD) or Death through Postmenstrual age (PMA) 36 Weeks (Time Frame: Baseline through 36 weeks postmenstrual age (PMA)). BPD can be assessed, for example, by modified National Institute of Child Health and Human Development (NICHD) severity grading and standardized by oxygen challenge testing. In some embodiments, the compositions and methods described herein result in reduced incidence of CLD as assessed by Incidence of Bronchopulmonary Dysplasia (BPD) or Death through Postmenstrual age (PMA) 36 Weeks as compared to a baseline control.

In some embodiments, reduced incidence or treatment of CLD is assessed by the Total Number of Days on Respiratory Technology Support (RTS) from birth through 12 Months corrected age (CA) (time frame: birth through 12 months CA). In this context, RTS can be defined as any one of the following: (1) any fraction of inspired oxygen (FiO2) greater than (>) 21 percent (%), (2) noninvasive respiratory support delivered via a nasal interface (e.g., continuous positive airway pressure [CPAP], bilevel positive airway pressure [BiPAP], high flow therapy, nasal intermittent positive pressure ventilation [NIPPV], nasal cannula), (3) invasive respiratory support (mechanical ventilation) via an endotracheal tube or tracheostomy. Total number of days on RTS from birth through 12 months CA will be reported. In some embodiments, the compositions and methods described herein result in reduced incidence of CLD as assessed by Total Number of Days on Respiratory Technology Support (RTS) from birth through 12 Months corrected age (CA) (time frame: birth through 12 months CA) as compared to a baseline control.

In some embodiments, reduced incidence or treatment of CLD is assessed by the duration of re-hospitalizations (Time Frame: From neonatal intensive care unit (NICU) discharge through 12 months CA). In some embodiments, the compositions and methods described herein result in reduced incidence of CLD as assessed by the duration of re-hospitalizations as compared to a baseline control.

In some embodiments, reduced incidence or treatment of CLD is assessed by the number of emergency room visits (Time Frame: From NICU discharge through 12 months CA). In some embodiments, the compositions and methods described herein result in reduced incidence of CLD as assessed by the number of emergency room visits.

In some embodiments, the compositions and methods described herein result in reduced incidence of CLD as assessed by the number of days of respiratory medication use (Time Frame: From NICU discharge through 12 months CA). In some embodiments, the compositions and methods described herein result in reduced incidence of CLD as assessed by the number of days of respiratory medication use as compared to a baseline control. Respiratory medication use can include for example bronchodilators, steroids, leukotriene inhibitors, diuretics.

In some embodiments, reduced incidence or treatment of CLD is assessed by the incidence of signs and/or symptoms of respiratory disease as Assessed by a 28-day Caregiver-administered Diary ending at 12 Months Corrected age (CA) (Time Frame: 11 months CA through 12 months CA). In some embodiments, the compositions and methods described herein result in reduced incidence of CLD as assessed by a 28-day Caregiver-administered Diary ending at 12 Months Corrected age (CA) as compared to a baseline control.

In some embodiments, reduced incidence or treatment of CLD is assessed by the incidence of Chronic Respiratory Morbidity (CRM1) through 12 Months Corrected age (CA) (Time Frame: From NICU discharge through 12 months CA). A subject is defined as having CRM1 if he or she experienced/required at least 1 of the 3 following clinical/treatment events, as reported by parents/caregivers and captured by the pulmonary morbidity assessment on at least two 3-month quarters over a 12-month time period: 1. Emergency room visit or hospitalization associated with a respiratory diagnosis, 2. Home RTS, 3. Daily use of respiratory medications (e.g., bronchodilators, steroids, leukotriene inhibitors, diuretics) as reported by caregivers on the pulmonary morbidity assessment. Incidence of CRM1 through 12 months CA will be reported. In some embodiments, the compositions and methods described herein result in reduced incidence of CLD as assessed by the incidence of Chronic Respiratory Morbidity (CRM1) through 12 Months Corrected age (CA) as compared to a baseline control.

In some embodiments, reduced incidence or treatment of CLD is assessed by the incidence of chronic respiratory morbidity including symptoms of respiratory disease (CRM2) through 12 Months Corrected age (CA) (Time Frame: From NICU discharge through 12 months CA). A subject is defined as having CRM2 if he or she experienced/required at least 1 of the 4 following clinical/treatment events, as reported by parents/caregivers and captured by the pulmonary morbidity assessment on at least two 3-month quarters over a 12-month time period: 1. Emergency room visit or hospitalization associated with a respiratory diagnosis, 2. Home RTS, 3. Daily use of respiratory medications (e.g., bronchodilators, steroids, leukotriene inhibitors, diuretics) as reported by caregivers on the pulmonary morbidity assessment, 4. Symptoms of respiratory disease as defined by presence of cough without cold, or wheeze at least once per week. Incidence of CRM2 through 12 months CA will be reported. In some embodiments, the compositions and methods described herein result in reduced incidence of CLD as assessed by the incidence of chronic respiratory morbidity including symptoms of respiratory disease (CRM2) through 12 Months Corrected age (CA) as compared to a baseline control.

In some embodiments, reduced incidence or treatment of CLD is assessed by the severity of chronic respiratory morbidity (CRM3) through 12 Months Corrected age (CA), as determined by the Chronic Lung Disease (CLD) of Infancy Severity Score (Time Frame: From NICU discharge through 12 months CA). This is determined by, for example, the CLD of infancy severity score that will include components such as respiratory hospitalizations, RTS use, and use of respiratory medications. In some embodiments, the compositions and methods described herein result in reduced incidence of CLD as assessed by the severity of chronic respiratory morbidity (CRM3) through 12 Months Corrected age (CA), as determined by the Chronic Lung Disease (CLD) of Infancy Severity Score as compared to a baseline control.

In some embodiments, reduced incidence or treatment of CLD is assessed by the incidence of Intraventricular Hemorrhage (IVH) through postmenstrual age (PMA) 40 weeks, as assessed by cranial ultrasound (Time Frame: Baseline through 40 Weeks PMA). In some embodiments, the compositions and methods described herein result in reduced incidence of CLD as assessed by the incidence of Intraventricular Hemorrhage (IVH) through postmenstrual age (PMA) 40 weeks, as assessed by cranial ultrasound as compared to a baseline control.

In some embodiments, reduced incidence or treatment of CLD is assessed by the incidence of Intraventricular Hemorrhage (IVH) through postmenstrual age (PMA) of between about 36-40 weeks, as assessed by cranial ultrasound (Time Frame: Baseline through 36-40 weeks Weeks PMA). In some embodiments, the compositions and methods described herein result in reduced incidence of CLD as assessed by the incidence of Intraventricular Hemorrhage (IVH) through postmenstrual age (PMA) 36-40 weeks, as assessed by cranial ultrasound as compared to a baseline control.

In some embodiments, reduced incidence or treatment of CLD is assessed by the incidence of Intraventricular Hemorrhage (IVH) through postmenstrual age (PMA) 36 weeks, as assessed by cranial ultrasound (Time Frame: Baseline through 36 Weeks PMA). In some embodiments, the compositions and methods described herein result in reduced incidence of CLD as assessed by the incidence of Intraventricular Hemorrhage (IVH) through postmenstrual age (PMA) 36 weeks, as assessed by cranial ultrasound as compared to a baseline control.

In some embodiments, reduced incidence or treatment of CLD is assessed by the motor function at 12 Months Corrected age (CA), as measured by Alberta Infant Motor Scales (AIMS) (Time Frame: At 12 months CA). The AIMS is a measure of early motor maturation used for assessment of infants at risk for motor delay, focusing on attainment of motor milestones and development of postural control. It consists of 58 items, including assessments in 4 postural positions: prone (21 items), supine (9 items), sitting (12 items) and standing (16 items). Each item is scored as 'observed' or 'not observed'. The scorer identifies the least and most mature item observed. In some embodiments, the compositions and methods described herein result in reduced incidence of CLD as assessed by the motor function at 12 Months Corrected age (CA), as measured by Alberta Infant Motor Scales (AIMS) as compared to a baseline control.

In some embodiments, reduced incidence or treatment of CLD is assessed by the functional status as assessed by PREMature Infant Index (PREMII) at Postmenstrual Age (PMA) 36 Weeks (Time Frame: At 36 weeks PMA). PREMII is a Clinician-Reported Outcome (ClinRO) assessment used to capture overall functional maturation of extremely preterm neonates. Functional Status is defined as what the infant can do with respect to 8 key functional areas (feeding, weight gain, thermoregulation, respiratory support, apnea, bradycardia, desaturation events, and oxygen administration), as a reflection of the infant's overall health and development. In some embodiments, the compositions and methods described herein result in reduced incidence of CLD as assessed by the functional status as assessed by PREMature Infant Index (PREMII) at Postmenstrual Age (PMA) 36 Weeks as compared to a baseline control.

In some embodiments, reduced incidence or treatment of CLD is assessed by the incidence of mortality (Time Frame: From birth through 12 months CA). In some embodiments, the compositions and methods described herein result in reduced incidence of CLD as assessed by the incidence of mortality as compared to a baseline control.

In some embodiments, reduced incidence or treatment of CLD is assessed by the exposure-response of Insulin-like Growth Factor-1 (IGF-1) (Time Frame: Up to 12 months CA). IGF-1 is evaluated for example using (Pharmacokinetic/Pharmacodynamic [PK/PD]) relationship of IGF-1 between respiratory and neurologic endpoints. In some embodiments, the compositions and methods described herein result in reduced incidence of CLD as assessed by the exposure-response of Insulin-like Growth Factor.

### Intraventricular Hemorrhage

Intraventricular hemorrhage (IVH) is a life threatening condition in premature infants characterized by bleeding in and around brain ventricles, the spaces in the brain containing cerebrospinal fluid. Intraventricular hemorrhage is most common in premature babies, especially those born before 28 weeks gestational age.

Babies with respiratory problems, such as respiratory distress syndrome (RDS), or other complications of prematurity, are more likely to have IVH.

IVH is associated with serious complications including periventricular hemorrhagic infarction, post hemorrhagic ventricular dilatation, periventricular leukomalacia, and cerebellar hemorrhage, resulting in mortality and morbidity. Other complications include posthemorrhagic hydrocephalus, cerebral palsy, and mental retardation, and long-term neurodevelopmental disorders.

Current standard of care for prevention and/or treatment of IVH is based on antenatal steroids and the early management and stabilization of cerebral hemodynamics and respiratory support. Prognosis is related to the severity of bleeding, damage to the brain parenchyma, the presence of seizures and severity of periventricular hemorrhagic infarction. IVH treatment comprises supportive management of symptoms with medications to reduce bleeding and damage to the brain, and prevent seizures.

In some aspects, described herein are methods for treating or preventing IVH comprising administering to a subject in need thereof a composition comprising recombinant insulin-like growth factor 1 (rIGF-1), recombinant insulin-like growth factor binding protein 3 (rIGFBP-3), and a surfactant (*e.g.,* a polysorbate surfactant, *e.g.,* polysorbate 20).

In some aspects, described herein are methods of treating or preventing intraventricular hemorrhage (IVH) comprising administering to a subject in need thereof a composition comprising recombinant insulin-like growth factor 1 (rIGF-1), recombinant insulin-like growth factor binding protein 3 (rIGFBP-3), and a polysorbate surfactant at a concentration of between about 0.0025% and 0.0075%, wherein the rIGF-1 and rIGFBP-3 are complexed in equimolar amounts, and wherein the rIGF-1/IGFBP-3 is at a concentration of about 10 micrograms/mL - 1000 micrograms/mL. In some aspects described herein are methods of treating or preventing intraventricular hemorrhage (IVH) comprising administering to a subject in need thereof a composition comprising recombinant insulin-like growth factor 1 (rIGF-1), recombinant insulin-like growth factor binding protein 3 (rIGFBP-3), at a therapeutic amount sufficient to achieve reduced incidence of intraventricular hemorrhage relative to a control.

IVH grades are qualified as follows: grade I - hemorrhage in the region of germinal matrix, very little hemorrhage or no hemorrhage in the ventricle; grade II - hemorrhage filling 10-50% of the ventricle; grade III - hemorrhage filling more than 50% of the ventricle; grade IV (PVHI) - periventricular echodensity. This hemorrhage, which was named grade IV previously, is currently classified as periventricular hemorrhagic infarction (PVI). Grade I and II are defined as mild grades and grade III and PVHI are defined as advanced grades. Table 1 shows cranial ultrasound image grading for IVH.

**Table 1: Cranial ultrasound image grading for IVH**

| ***IVH Grades by Volpe Method*** | |
|---|---|
| Severity | Description |
| Grade I | GMH with no or minimal IVH (<10% of ventricular area on parasagittal view) |
| Grade II | IVH in 10-50% of ventricular area on parasagittal view |
| Grade III | IVH >50% of ventricular area on parasagittal view; usually distends lateral ventricle |
| Grade IV | IVH compounded by hemorrhagic venous infarction in the periventricular white matter |

In some embodiments, the compositions and methods described herein result in reduced incidence of Grade II IVH, Grade III IVH and Grade IV IVH or PVHI. In some embodiments, the compositions and methods described herein result in reduced incidence of Grade II IVH. In some embodiments, the compositions and methods described herein result in reduced incidence of Grade III IVH. In some embodiments, the compositions and methods described herein result in reduced incidence of PVHI. In some embodiments, the compositions and methods described herein are used to treat Grade II IVH, Grade III IVH and Grade IV IVH or PVHI. In some embodiments, the compositions and methods described herein are used to treat Grade II IVH. In some embodiments, the compositions and methods described herein are used to treat Grade III IVH. In some embodiments, the compositions and methods described herein are used to treat Grade IV IVH or PVHI.

In some embodiments, incidence of IVH is assessed by cranial ultrasonography or ultrasound. Screening is performed at various intervals to detect and assess cerebral hemorrhage, including, for example, in some embodiments, on day 0, 1, 2, 3, 7, 14, and 21 days. In some embodiments, ultrasound is further carried out at 32 weeks PMA, 33 weeks PMA, 34 weeks PMA, 35 weeks PMA, 36 weeks PMA, 37 weeks PMA, 38 weeks PMA, 39 weeks PMA or 40 weeks PMA. In some embodiments, ultrasound is further carried out at 36 weeks PMA. In some embodiments, ultrasound is further carried out at 40 weeks PMA. In some embodiments, a single reader (masked to treatment) is used to evaluate all ultrasound images for the highest grade of GMH-IVH, according to Papile and Bowerman methods. (Papile LA, et al. Incidence and evolution of subependymal and intraventricular hemorrhage: a study of infants with birth weights less than 1,500 gm. J. Pediatr. (1978). 92: 529-34; Bowerman RA, et al. Natural history of neonatal periventricular/intraventricular hemorrhage and its complications: sonographic observations. AJR Am J. Roentgenol. (1984) 143: 1041-52.)

In some embodiments, no IVH and grade I IVH are grouped together. In some embodiments, two independent readers are used for a post-hoc analysis which is also masked to treatment to assess periventricular hemorrhagic infarction (PVHI), and discrepancies were resolved by consensus agreement. IVH was graded based on severity according to Volpe. In some embodiments, PHI is graded by Dudink method by assessing the localization of caudate vein, temporal vein, anterior terminal vein and complete terminal vein infarction. (Dudink J. et al., Venous subtypes of preterm periventricular haemorrhagic infarction. Arch Dis Child Fetal Neonatal Ed. (2008) 93:F201-6.)

In some embodiments, a limited PHI is assessed when only caudate vein or temporal vein is affected or a small anterior terminal vein infarction is observed in cranial ultrasounds. In some embodiments, extensive PHI is assessed with complete terminal vein infarction or combination of caudate vein, temporal vein and anterior vein infarctions. Cranial ultrasound images are also used to detect post-hemorrhagic ventricular dilatation (PHVD), and white matter injury (WMI). (Davies, M.W. et al., Reference ranges for the linear dimensions of the intracranial ventricles in preterm neonates. Arch. Dis. Child Fetal Neonatal Ed., 2000 82:F218-23; Govaert P. et al. An Atlas of Neonatal Brain Sonography. 2nd Ed. London: Mac Keith Press, 2010).). In some embodiments, WMI is scored using a four-grade scale as shown in Table 2.

**Table 2. Grades of Post-Hemorrhagic Ventricular Dilatation (PHVD) and White Matter Injury**

| ***PHVD Grades by Davies Method*** | |
|---|---|
| Severity | Description |
| 0 | Normal, Anterior Horn Width < 3mm |
| 1 | Mild, Anterior Horn Width 3 to < 5mm |
| 2 | Moderate, Anterior Horn Width 5-10 mm |
| 3 | Severe, Anterior Horn Width >10 mm |

| ***White Matter Injury by Govaert and deVries Method*** | |
|---|---|
| Scoring | Description |
| 0 | Persistent periventricular hyperechogenicity or punctate lesions, no cysts, no obvious white matter loss (white matter loss was identified as ventricular dilatation without hemorrhage) |
| 1 | Persistent periventricular hyperechogenicity or extensive punctate lesions evolving into diffuse white matter loss without cysts |
| 2 | Persistent periventricular hyperechogenicity evolving into small localized frontoparietal cystic lesions (limited cystic PVL) |
| 3 | Persistent periventricular hyperechogenicity evolving into extensive cystic lesions (extensive cystic PVL) |

| ***Brain Injury Severity Score*** | |
|---|---|
| Scoring | Description |
| 0 | No brain abnormalities |
| 1 | GMH, periventricular hyperechogenicity without white or gray matter loss, and mild-moderate cerebral injury |
| 2 | IVH II, mild PHVD, stroke of a perforating artery |
| 3 | IVH III, persistent moderate PHVD without shunt or Rickham device, persistent periventricular hyperechogenicities with diffuse white and/or gray matter loss at term cranial ultrasound |
| 4 | Limited PHI, limited PVL, PHVD with shunt or Rickham device, anterior cerebral artery stroke, posterior cerebral artery stroke, severe cerebellar injury |
| 5 | Unilateral extensive PHI, extensive cystic PVL, mild cerebral artery stroke, severe global brain atrophy |
| 6 | Bilateral extensive PHI |

In some embodiments, IVH is assessed by magnetic resonance imaging (MRI). MRI evaluates white matter injury and dimensions and localization of hemorrhage associated with neurodevelopmental outcomes.

In some embodiments, a consensus IVH grade is allotted to each subject based on the highest grade of IVH observed by single, or in the case of multiple readers, joint masked readers.

In some embodiments, reduced incidence of IVH relates to assessing a subject as manifesting a lower grade of IVH as compared to the grade of IVH in the absence of treatment. In some embodiments, for example, reduced incidence refers to Grade I IVH instead of Grade II, Grade III or Grade IV IVH prior to treatment. In some embodiments, reduced incidence refers to Grade II IVH instead of Grade III or Grade IV IVH prior to treatment. In some embodiments, reduced incidence refers to Grade III IVH instead of Grade IV IVH prior to treatment.

In some embodiments, the severity of IVH according to treatment group was determined from the maximum-grade hemorrhage observed for each infant in the population. In some embodiments, reduced incidence of IVH refers to a reduction of incidence of IVH in the population by 20-50%. In some embodiments, reduced incidence of IVH refers to a reduction of incidence of IVH in the population by 20%. In some embodiments, reduced incidence of IVH refers to a reduction of incidence of IVH in the population by 30%. In some embodiments, reduced incidence of IVH refers to a reduction of incidence of IVH in the population by 40%. In some embodiments, reduced incidence of IVH refers to a reduction of incidence of IVH in the population by 50%.

In some embodiments, reduced incidence of IVH refers to a reduction of incidence of Grade IV IVH (or PHVI) in the population by 20-50%. In some embodiments, reduced incidence of IVH refers to a reduction of incidence of Grade IV IVH (or PHVI) in the population by 20%. In some embodiments, reduced incidence of IVH refers to a reduction of incidence of Grade IV IVH (or PHVI) in the population by 30%. In some embodiments, reduced incidence of IVH refers to a reduction of incidence of Grade IV IVH (or PHVI) in the population by 40%. In some embodiments, reduced incidence of IVH refers to a reduction of incidence of Grade IV IVH (or PHVI) in the population by 50%.

In some embodiments, reduced incidence of IVH refers to a reduction of incidence of Grade III IVH in the population by 20-50%. %. In some embodiments, reduced incidence of IVH refers to a reduction of incidence of Grade III IVH in the population by 20%. In some embodiments, reduced incidence of IVH refers to a reduction of incidence of Grade III IVH in the population by 30%. In some embodiments, reduced incidence of IVH refers to a reduction of incidence of Grade III IVH in the population by 40%. In some embodiments, reduced incidence of IVH refers to a reduction of incidence of Grade III IVH in the population by 50%.

In some embodiments, reduced incidence of IVH refers to a reduction of incidence of Grade II IVH in the population by 20-50%. In some embodiments, reduced incidence of IVH refers to a reduction of incidence of Grade II IVH in the population by 20%. In some embodiments, reduced incidence of IVH refers to a reduction of incidence of Grade II IVH in the population by 30%. In some embodiments, reduced incidence of IVH refers to a reduction of incidence of Grade II IVH in the population by 40%. In some embodiments, reduced incidence of IVH refers to a reduction of incidence of Grade II IVH in the population by 50%.

In some embodiments, reduced incidence of IVH refers to a reduction of incidence of Grade I IVH in the population by 20-50%. %. In some embodiments, reduced incidence of IVH refers to a reduction of incidence of Grade I IVH in the population by 20%. In some embodiments, reduced incidence of IVH refers to a reduction of incidence of Grade I IVH in the population by 30%. In some embodiments, reduced incidence of IVH refers to a reduction of incidence of Grade I IVH in the population by 40%. In some embodiments, reduced incidence of IVH refers to a reduction of incidence of Grade I IVH in the population by 50%.

In some embodiments, the treatment of IVH refers to administration of a therapeutic composition (e.g., IGF-1/IGFBP-3) that partially or completely alleviates, ameliorates, relieves, inhibits, delays onset of, prevents, reduces severity of and/or reduces incidence of one or more symptoms or features of Intraventricular Hemorrhage. In some embodiments, the subject receiving treatment does not exhibit signs of IVH and/or exhibits only early or mild signs of IVH. In some other embodiments, the subject receiving treatment exhibits one or more established signs of IVH. Accordingly, in some embodiments, treatment refers to preventing onset or progression of IVH from mild to severe forms.

In some embodiments, the compositions and methods described herein result in reduced incidence of IVH. In some embodiments, the compositions and methods described herein are used to treat IVH.

### Bronchopulmonary Dysplasia

Bronchopulmonary dysplasia (BPD) is a serious lung condition that affects newborns. BPD mostly affects premature infants and is a breathing disorder where an infant's lungs become irritated and do not develop normally. BPD mostly affects premature newborns who need oxygen therapy, which is oxygen given through nasal prongs, a mask, or a breathing tube. It occurs most often in low-weight infants born more than 10 weeks early or weigh less than 2 pounds at birth.

Since it was first described in 1967, the definition for BPD has been evolving. The original definition included infants with oxygen requirement for 28 days with associated radiographic changes. This definition of BPD was then refined as infants requiring oxygen support at 36 weeks PMA in infants born with birth weight < 1,500 g. In 2001, the NIH defined BPD as "For those born at gestational age < 32 weeks, BPD referred to requirement of oxygen support (>21%) for at least 28 days and a subsequent assessment at 36 weeks PMA or discharge, whichever comes first. In those born with GA >32 weeks, BPD referred to the requirement of supplemental oxygen (<21%) for at least 28 days and a subsequent assessment at 56 days postnatal age or discharge, whichever comes first." Infants with no oxygen requirement were classified as having mild BPD. Moderate BPD was diagnosed in those requiring <30% oxygen and severe BPD in those with a need for positive pressure ventilation/continuous positive pressure and/or oxygen requirement >30%. The "physiologic definition" of BPD proposed in 2003 classified as infants as having BPD that "required positive pressure and oxygen ≥0.3 at 36 weeks PMA" In contrast, those that required FiO2 <0.3, required an oxygen reduction test for upto 2 weeks based on oxygen saturation. However, the physiologic definition remained less popular than the functional criterion that if premature newborns still require oxygen therapy by the time they reach 36 weeks of post menstrual age, they are diagnosed with BPD.

Babies who develop BPD are often born with respiratory distress syndrome (RDS). Bronchopulmonary dysplasia is often referred to interchangeably with chronic lung disease of premature babies, but BPD is in fact a single time-point assessment at 36 weeks, while CLD broadly refers to chronic and prolonged or persistent lung disease, sometimes in infants previously diagnosed with BPD, or in infants that have not yet received a diagnosis for BPD. For practical purposes, BPD provides a relevant, easily measured clinical endpoint to measure outcomes on CLD as it is more well-defined. Accordingly, in some embodiments, the methods described herein result in reduced incidence of BPD. In some embodiments, the methods described herein are used to treat BPD. In some embodiments, the methods described herein result in no, mild or less severe BPD.

Some newborns may need long-term oxygen or breathing support from nasal continuous positive airway pressure (NCPAP) machines, ventilators, and medicines like bronchodilators. They may continue to have breathing problems due to chronic lung disease of prematurity throughout childhood and even into adulthood.

In some embodiments, the compositions and methods described herein result in reduced incidence of bronchopulmonary dysplasia. In some embodiments, the compositions and methods described herein are used to treat bronchopulmonary dysplasia.

### Pulmonary hypertension

Antenatal factors, such as chorioamnionitis (CA) and preeclampsia (PE), and postnatal injury, including exposure to high levels of supplemental oxygen, are strongly associated with an increased risk for bronchopulmonary dysplasia (BPD) and pulmonary hypertension (PH) after preterm birth.

Persistent pulmonary hypertension of the newborn occurs when a newborn's circulation system doesn't adapt to breathing outside the womb. While in the womb, the fetus receives oxygen through the umbilical cord, so the lungs need little blood supply. This can lead to symptoms such as fast breathing, difficulty breathing, and/or low oxygen saturation.

In some embodiments, the compositions and methods described herein result in reduced incidence of pulmonary hypertension. In some embodiments, the compositions and methods described herein are used to treat pulmonary hypertension.

### Right Ventricular Hypertrophy

Right ventricular hypertrophy (RVH) is an abnormal enlargement or pathologic increase in muscle mass of the right ventricle in response to pressure overload, most commonly due to severe lung disease. The right ventricle is considerably smaller than the left ventricle and produces electrical forces that are largely obscured by those generated by the larger left ventricle.

In some embodiments, the compositions and methods described herein result in reduced incidence of RVH. In some embodiments, the compositions and methods described herein are used to treat RVH.

### Necrotising enterocolitis

Necrotising enterocolitis (NEC) is a comorbidity of prematurity that may also occur in infants manifesting chronic lung disease of prematurity. Necrotizing enterocolitis affects mostly the intestine of premature infants. The wall of the intestine is invaded by bacteria, which cause local infection and inflammation that can ultimately destroy the wall of the bowel (intestine).

In some embodiments, the compositions and methods described herein result in reduced incidence of NEC. In some embodiments, the compositions and methods described herein are used to treat NEC.

In yet another aspect, a method is provided for manufacturing a protein complex comprising insulin-like growth factor 1 (IGF-1) and insulin-like growth factor binding protein 3 (IGFBP-3), wherein the method comprises a use of a contact surface other than stainless steel, such as a single-use bag, during compounding the complex with a formulation solution, wherein, in the protein complex manufactured by such a method, an increase is oxidized species of the IGF-1 is for example, less than 20 % upon storage for 6 months at 25 °C. In some embodiments, the increase in oxidized species of IGF-1 is less than 5% upon storage for 6 months at 25 °C. In some embodiments, the increase in oxidized species of IGF-1 is less than 2% upon storage for 6 months at 25 °C. In some embodiments, the increase in oxidized species of the IGF-1 is, for example, less than 10% upon storage for 3 months at 40 °C. In some embodiments, the increase in oxidized species of the IGF-1 is, for example, less than 2 % upon storage for 3 months at 40 °C. In some embodiments, less than about 5-25 % *(e.g.* less than about 10%) of the protein complex exists as low molecular weight species upon storage for 6 months at 40 °C. In some embodiments, less than 5-15 % *(e.g.* less than about 10 %) of the protein complex exists as low molecular weight species upon storage for 3 months at 40 °C. In some embodiments, the formulation solution comprises a surfactant (*e.g.* polysorbate 20) at a concentration of between about 0.0025 v/v to 0.0075 v/v *(e.g.* 0.0025 % v/v).

Single-use bags are disposable process containers comprising an inert contact surface other than stainless steel. In some embodiments, a single-use bag is made of highly pure, medical grade composite film which is strong, flexible and provides excellent gas barrier performance, making it suitable for bioprocessing of drug product under sterile conditions, resulting in a reduction of oxidized species, improving potency and stability of the drug product. In some embodiments, a single-use bag is used during compounding, wherein the mixing, blending, and preparation of the IGF-1/IGFBP-3 drug substance in a formulation solution, comprising excipients (sodium chloride, sodium acetate, acetic acid and polysorbate 20) is carried out to yield IGF-1/IGFBP-3 drug product, wherein the composition has a pH of about 5.3-5.8. In some embodiments, a single-use bag is further used during a drug product filtration step. The method comprises, for example, filling between about 20-80% of a single-use bag with the protein complex during the compounding step and/or the filtration step. In some embodiments, the single-use bag is about or greater than 500 L.

In some embodiments, storage and transport are additionally carried out in a single-use bag. In some embodiments, single-use bags range in thickness between 0.1 mm to about 0.5 mm. In some embodiments, single-use bags range in thickness between 0.25 mm to about 0.35 mm.

In some embodiments, a single-use bag comprises ultra-low-density polyethylene (ULDPE) contacting the fluid. In some embodiments, a single-use bag comprises a gas barrier comprising polyethylene vinyl alcohol copolymer (EVOH). In some embodiments, the outer layers of a single-use bag comprise polyethylene vinyl acetate (EVA) and ULDPE. In some embodiments, a single-use bag comprises polyethylene vinyl acetate (EVA) contacting the fluid surface. In some embodiments, the gas barrier comprises polyethylene vinyl alcohol copolymer (EVOH). In some embodiments, the outer layer of a single-use bag comprises linear low-density polyethylene (LLDPE). In some embodiments, a kit is provided for carrying out the methods described herein. In certain embodiments, the kit comprises a composition comprising rIGF-1, rIGFBP-3, and a polysorbate surfactant, such as polysorbate 20 or polysorbate 80. In some embodiments, the kit contains the composition described herein in a vial of glass or other suitable material.

In some embodiments, there is also provided an article of manufacture comprising packaging material and a pharmaceutical agent contained within the packaging material. The packaging material comprises a label which indicates that the pharmaceutical may be administered, for a sufficient term at an effective dose, for treating and/or preventing complications associated with preterm birth. The pharmaceutical agent comprises the IGF-I/IGFBP-3 composition together with a pharmaceutically acceptable carrier.

In one embodiment, the formulation is provided as a unit comprising 50 micrograms/mL rhIGF-1/rhIGFBP-3 solution in 50 mM sodium acetate and 105 mM sodium chloride with 0.005% (v/v) polysorbate 20, at pH 5.5, stored at 2°C to 8°C (36°F to 46°F).

In one embodiment, the pharmaceutical composition according to the present disclosure is provided as final product in an infusion bag, or glass vial, containing approximately 6.5 mL extractable volume.

The invention will be further characterized by the following examples which are intended to be exemplary of the invention.

Comprising in the context of the present specification is intended to mean "including".

Where technically appropriate, embodiments of the invention may be combined, in particular where methods describe composition elements this combination of features may be employed to define the composition per se, and vice versa.

The background contains technical information and may be used as basis for amendments.

Embodiments are described herein as comprising certain features/elements. The disclosure also extends to separate embodiments consisting or consisting essentially of said features/elements.

Technical references such as patents and applications are incorporated herein by reference.

Any embodiments specifically and explicitly recited herein may form the basis of a disclaimer either alone or in combination with one or more further embodiments.

The present application claims from priority from U.S. Provisional Application No. 63/093,696, filed on October 19, 2020, incorporated herein by reference. These documents may be used as the basis for corrections.

The invention will now be described with reference to the following examples, which are merely illustrative and should not be construed as limiting the scope of the present invention.

### EXAMPLES

While certain compounds, compositions and methods described herein have been described with specificity in accordance with certain embodiments, the following examples serve only to illustrate the compounds of the invention and are not intended to limit the same.

### Example 1. Assessing the in-use stability of Mecasermin Rinfabate Drug Product in the presence and absence of 0.005% (v/v) polysorbate 20 (P20)

This example illustrates the in-use stability and/or compatibility of the rhIGF-1/rhIGFBP-3 drug product in the presence and absence of 0.005% polysorbate 20 (P20) during a worse case administration simulation in a Phase 2 Swedish Model infusion set over a period of 48 hours at room temperature.

Briefly, an assessment was conducted with two drug product formulations: the Phase 2 (Process A) drug product formulation (50 µg/mL mecasermin rinfabate in 50 mM sodium acetate with 105 mM sodium chloride, at pH 5.5) in the absence of polysorbate 20, and the Phase 3 (Process B) drug product formulation that consisted of the Phase 2 formulation containing 0.005% (v/v) polysorbate 20.

During Phase 2 clinical studies, infusion sets had to be primed and flushed with the drug product for at least one hour to prevent adsorption-related product loss to the infusion sets, and ensure that the premature neonates received the administered dose of 250 µg/kg/24 hours of drug product. It was observed that when "dry" infusion sets were used without any priming or flushing with the drug product, or any other solution, prior to administration, absorption-related product loss occurred to the infusion set ("worse case administration").

As the concentration of the drug product is extremely low (50 µg/mL) and the Phase 2 formulation buffer does not contain any surfactant, which prevents or reduces protein adsorption to different polymeric surfaces, a study was carried out in order to investigate the effect of inclusion of 0.005% P20 in the drug product for adsorption-related product loss and overall quality attributes of the drug product such as purity, pH, appearance and potency during administration.

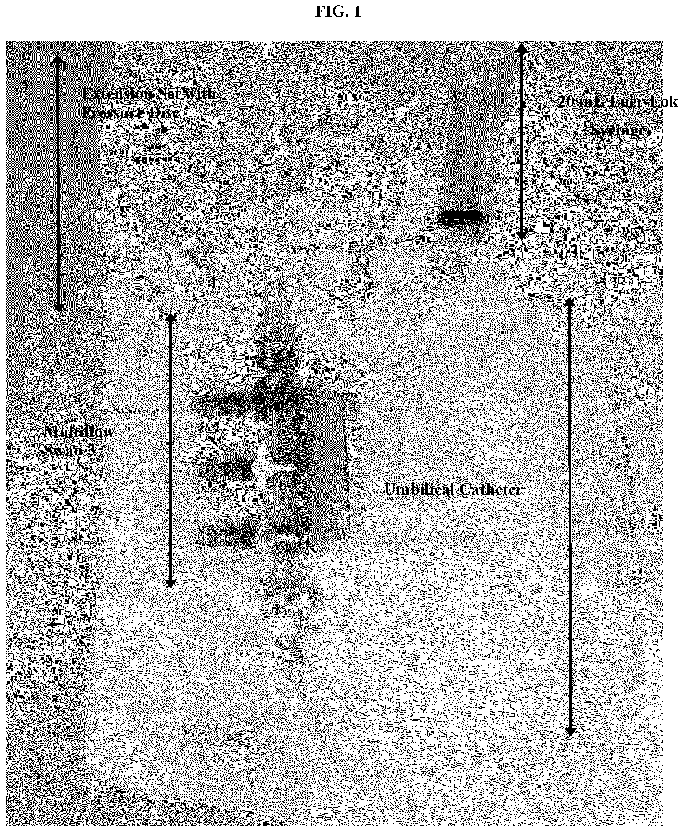

The experimental infusion set that was used as a model for this study was previously used in Karolinska, Sweden in the Phase 2 studies. Approximately 19 mL of the drug product in either formulation, with and without P20, was placed in a 20 mL syringe and each infusion set was labeled with the appropriate formulation; the drug product volume in the syringe was selected to enable the testing requirements. The syringe was then subsequently connected to the already assembled (dry) infusion set (FIG. 1). Approximately 3 mL of the drug product was immediately dispensed from the infusion set and was collected in a clean glass vial; this sample served as the baseline drug product that did not spend an extended amount of time in the infusion set.

For each drug product (with or without P20), two independent infusion sets were prepared. Two control infusion sets were prepared to serve as the buffer background, which were used for protein concentration correction. Due to the low protein concentration of the drug product and use of a Variable Path length Technology for protein concentration determination, absorption interferences, possibly from leachates, must be overcome. In order to do this, corresponding buffer controls for the study were generated and treated exactly as the drug product; the buffers were placed in the syringe and held in the infusion sets for 0, 1, 4, 8, 24, and 48 hours. The buffer controls were then used to correct the protein concentration for the unknown light absorbing species responsible for background.

The data demonstrated that the addition of 0.005% (v/v) P20 reduced adsorption-related product loss as well as improved other quality attributes (for example, potency) of drug product during administration in the model infusion set over 48 hours, and that there was no need to prime and flush the infusion sets prior to the drug product administration.

The results of the study demonstrated that the quality of drug product in the Phase 3 formulation was maintained for up to 48 hours in the infusion set when stored at room temperature with exposure to ambient light and temperature after the inclusion of 0.005% P20 in the drug product formulation buffer.

Following the collection of the syringe-exposed drug product baseline sample, the drug product was held in the infusion set and sampled at 1, 4, 8, 24, and 48 hours (Table 3) with exposure to ambient light and temperature. The samples were visually observed immediately after sampling. The samples were refrigerated until ready to be tested at the end of the 48-hour study for pH, protein concentration (A276 nm), RP-UPLC, and SE-HPLC. The samples were also selectively tested for potency (at baseline and 48 hours), using a low throughput cell proliferation potency assay. Additional drug product vials were prepared and set aside to serve as the non-syringe exposed drug product control (baseline) for all assays.

**Table 3. Properties of Drug Product tested in the absence and presence of P20.**

| **Sampling Time (hr)** | **Drug Product Formulation with no P20** | | | | | **Drug Product Formulation with P20** | | | | |
|---|---|---|---|---|---|---|---|---|---|---|
| | **Visual observation** | **pH** | **Conc. Solo VPE** | **RP-UPL C** | **SE-HP L C** | **Visual observation** | **pH** | **Conc. Solo VPE** | **RP - UP LC** | **SE-HPL C** |
| Syringe-exposed Baseline Control | + | + | + | + | + | + | + | + | + | + |
| 1 | + | + | + | + | + | + | + | + | + | + |
| 4 | + | + | + | + | + | + | + | + | + | + |
| 8 | + | + | + | + | + | + | + | + | + | + |
| 24 | + | + | + | + | + | + | + | + | + | + |
| 48 | + | + | + | + | + | + | + | + | + | + |

Visual observation of the drug product as well as the formulation buffer controls for both formulations (with and without P20) was performed at each time point. Drug product and formulation samples demonstrated no trend with regard to the presence of particulates or fibers. No change in the pH of either drug product formulation (with and without P20) was observed at any time during the study.

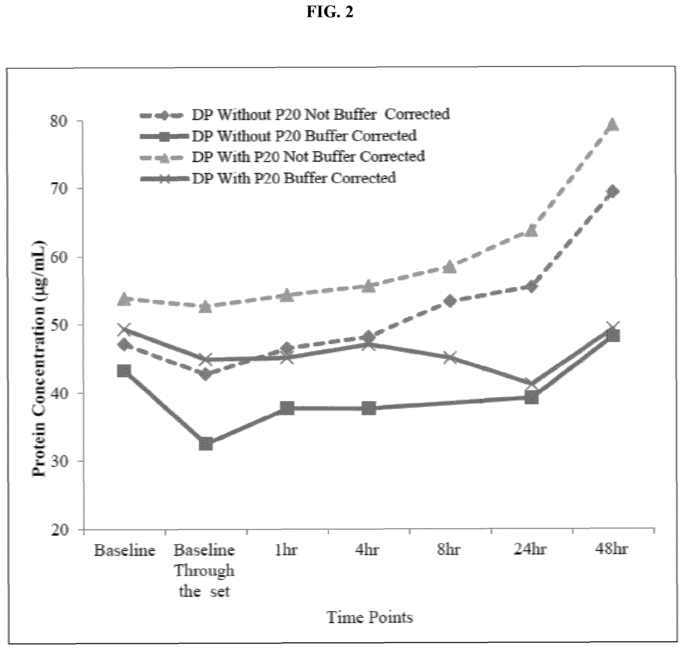

The protein concentration of the drug product was measured over the 48-hour study, before and after buffer correction (FIG. 2). During protein concentration measurements, an increase in the concentration at the later time points was observed that was attributed to potential unknown species that interfered with the protein concentration measurements. Therefore, formulation controls with and without P20 were also generated to correct the protein concentration for the unknown species.

The data showed that less adsorption-related protein loss was observed when P20 was present in the formulation. For example, over the initial infusion period of 4 hours, and after protein content correction for the unknown light absorbing species, a 3 fold less protein loss was observed for the drug product in the formulation that contained P20 as compared with the drug product in the formulation that did not contain P20. The increase in protein concentration at the 48 hour time point even after buffer correction is attributed to background due to leachates.

Overall, the results demonstrated that the addition of P20 prevented the adsorption related product loss in the model infusion set over 48 hours at room temperature. The improvement was seen when the drug product was first exposed to the "dry" infusion set for up to 4 hours. There is no need to prime and flush the infusion sets prior to the drug product administration after the inclusion of 0.005% polysorbate 20 in the drug product formulation.

In-line filter compatibility was assessed for two exemplary in-line filters. An average dose of about 5.5 mL for about 1 kg neonate was tested.

**Table 4. Protein Content Assessment Before and After In-line Filtration**

| Filter | Drug Product | Protein Concentration (micrograms/mL) | |
|---|---|---|---|
| | | Before Filtration | After Filtration |
| MX1480 Filter Assembly | Drug product | 48.1 | 32.1 |
| | Drug product with 0.005% (v/v) polysorbate 20 | 48.6 | 45.5 |
| Neonatal/Pediatric 0.22 micrometers, 96 hour IV filter | Drug product | 46.6 ± 1.1 | 31.5 ± 0.6 |
| | Drug product with 0.005% (v/v) polysorbate 20 | 50.8 ± 0.8 | 53.8 ± 0.4 |

In the absence of polysorbate 20, a significant loss of drug product was observed in both instances of exemplary in-line filters.

Loss of protein content observed in the absence of P20 is undesirable since increasing dose volumes is not feasible in neonates to deliver higher effective doses due to daily limited fluid intake in neonates.

### Example 2. Measuring purity of the Mecasermin Rinfabate drug product in formulations in the presence and absence of 0.005% (v/v) polysorbate 20 (P20) by SE-HPLC and RP-UPLC

This example illustrates the purity of drug product in formulations in the presence and absence of 0.005% (v/v) polysorbate 20 (P20).

In order to compare the purity of the drug product in the presence and absence of 0.005% (v/v) polysorbate 20 (P20), the Size Exclusion High Performance Liquid Chromatography (SE-HPLC) assay was performed. The assay was characterized with the optimization of the column loading for the best profile and signal detection.

**Table 5. Stability Summary of Drug Product in the Infusion Set without Polysorbate 20 at the Ambient Temperature¹**

| ***Assay*** | ***Proposed Target Criteria*** | | ***Baseline²*** | ***Syringe Exposed Baseline*** | ***1 hour*** | ***4 hours*** | ***8 hours*** | **24 *hours*** | ***48 hours*** |
|---|---|---|---|---|---|---|---|---|---|
| ***Appearance and Description*** | | | | | | | | | |
| Appearance | Clear to slightly opalesce nt, colorless to slightly colored, essentiall y free of particles. | **T E S T** | Conforms | Clear, colorless, small and large fibers³ | Conforms | Clear, colorles s, 1 small white flake | Clear, colorles s with 1 short fiber | Confor ms | Clear, colorless, with a few small fibers |
| | | **B U F F E R** | NA | Clear, colorless, small and large fibers | Clear, colorless, small and 1 large fiber | NT | NT | NT | Clear, colorless with 1 small fiber |

| ***Purity and Impurities*** | | | | | | | | | |
|---|---|---|---|---|---|---|---|---|---|
| Size exclusion HPLC | ≥92% main peak area | | 93.9% | 94.4% | 94.5% | 94.8% | 94.6% | 94.9% | 95.3% |
| | ≤8% high molecular weight peak area⁴ | | 4.1% | 3.8% | 3.8% | 3.7% | 3.7% | 3.7% | 3.1% |
| RP-UPLC | ≥95% main peak area | | 97.3% | 97.3% | 97.0% | 96.1% | 96.0% | 95.3% | 95.6% |
| | Report results for oxidized rhIGF1 peak area | | 2.1% | 2.1% | 2.3% | 3.2% | 3.2% | 3.4% | 3.5% |

| ***Potency*** | | | | | | | | | |
|---|---|---|---|---|---|---|---|---|---|
| Cell Proliferation Assay | 50% to 150% Relative Potency | | 76% | NT | | | | | 57% |

| ***Content*** | | | | | | | | | |
|---|---|---|---|---|---|---|---|---|---|
| Protein concentratio n | 45 to 55 µg/ml | 43⁵ | 32 | 38 | 38 | NT | 39 | 48 | |

| ***General*** | | | | | | | | | |
|---|---|---|---|---|---|---|---|---|---|
| pH | 5.5±0.3 | 5.5 | 5.5 | 5.5 | 5.5 | 5.5 | 5.5 | 5.5 | |
| NT: Not Tested | | | | | | | | | |
| NA: Not Applicable | | | | | | | | | |
| 1. Average data from two infusion sets is shown except appearance and selected time points for concentration. | | | | | | | | | |
| 2. Baseline sample is the drug product in the vial (n=1). | | | | | | | | | |
| 3. The data is reported for the worse case when the particle was observed in at least 1 of the 2 vials. Although the receiving vials were washed and depyrogenated, a controlled study of purified water in the receiving vials (3 ml) demonstrated that small and large fibers were observed with water. The receiving vials were changed to clean 5 ml. receiving vials for time points 4 to 48 hours. | | | | | | | | | |
| 4. High molecular weight species are the sums of peaks 4, 5 and 6 in the SE-HPLC profile. The reported value is the average of data from two independent in-use studies. | | | | | | | | | |
| 5. The concentration value is reported after buffer correction: the value without buffer correction is 47 µg/ml. | | | | | | | | | |

**Table 6. Stability Summary of Drug Product in the Infusion Set with Polysorbate 20 at the Ambient Temperature¹**

| ***Assay*** | ***Propose d Target Criteria*** | | ***Baseline²*** | ***Syringe Exposed Baseline 1*** | ***1 hour*** | ***4 hours*** | ***8 hours*** | ***24 hours*** | ***48 hours*** |
|---|---|---|---|---|---|---|---|---|---|
| ***Appearance and Description*** | | | | | | | | | |
| Appearance | Clear to slightly opalesc ent, colorles s to slightly colored, essentia lly free of particles . | Tes t sets | Conforms | Clear, colorless, small and large fibers³ | Clear, colorles s, small and large fibers³ | Clear, colorles s, 1-2 small fibers | Clear, colorles s, 1 long fiber | Confor ms | Clear, colorless, small and large fibers |
| | | Buf fer sets | NA | Clear, colorless, 1 small and 1 large fiber⁴ | Clear, colorles s, 1 large fiber³ | Clear, colorles s, 1 small fiber, 1 small flake | Clear, colorles s, large, medium and small fibers⁴ | Clear, colorles s, 1 long fiber, 1 short fiber | Conform s |

| ***Purity and Impurities*** | | | | | | | | | |
|---|---|---|---|---|---|---|---|---|---|
| Size Exclusion HPLC | ≥92% main peak area | | 95% | 94.6% | 93.8% | 94.4% | 94.6% | 94.7% | 95.3% |
| | ≤8% high molecular weight peak area⁵ | | 3.7% | 3.7% | 3.8% | 3.6% | 3.5% | 3.5% | 3.1% |
| RP-UPLC | ≥95% main peak area | | 98% | 97.7% | 97.5% | 97.1% | 96.3% | 95.8% | 95.6% |
| | Report results for oxidised rhIGF1 peak area | | 1.7% | 1.7% | 1.9% | 2.3% | 2.9% | 3.4% | 3.5% |

| ***Potency*** | | | | | | | | | |
|---|---|---|---|---|---|---|---|---|---|
| Cell Proliferation Assay | 50 to 150% Relative Potency | | 90% | Not Tested | | | | | 83% |

| ***Appearance and Description*** | | | | | | | | | |
|---|---|---|---|---|---|---|---|---|---|
| Protein concentratio n | 45 to 55 µg/ml | | 49 | 45 | 45 | 47 | 45 | 41 | 49 |

| ***General*** | | | | | | | | | |
|---|---|---|---|---|---|---|---|---|---|
| pH | 5.5±0.3 | | 5.5 | 5.5 | 5.5 | 5.5 | 5.5 | 5.5 | 5.5 |
| NA: Not Applicable | | | | | | | | | |
| NT: Not Tested | | | | | | | | | |
| 1. Average data from two infusion sets is shown except appearance and selected time points for concentration. | | | | | | | | | |
| 2. Baseline sample is the drug product in the vial (n=1). | | | | | | | | | |
| 3. The data is reported for the worse case when the particle was observed in at least 1 of the 2 vials. Although the receiving vials were washed and depyrogenated, a controlled study of purified water in the receiving vials (3 ml) demonstrated that small and large fibers were observed with water. The receiving vials were changed to clean 5 ml. receiving vials for time points 4 to 48 hours. | | | | | | | | | |
| 4. A separate infusion step was set up to observe the appearance of the formulation buffer with P20 4 days after the original study. | | | | | | | | | |
| 5. High molecular weight species are the sums of peaks 4, 5 and 6 in the SE-HPLC profile. The reported value is the average of data from two independent in-use studies. | | | | | | | | | |

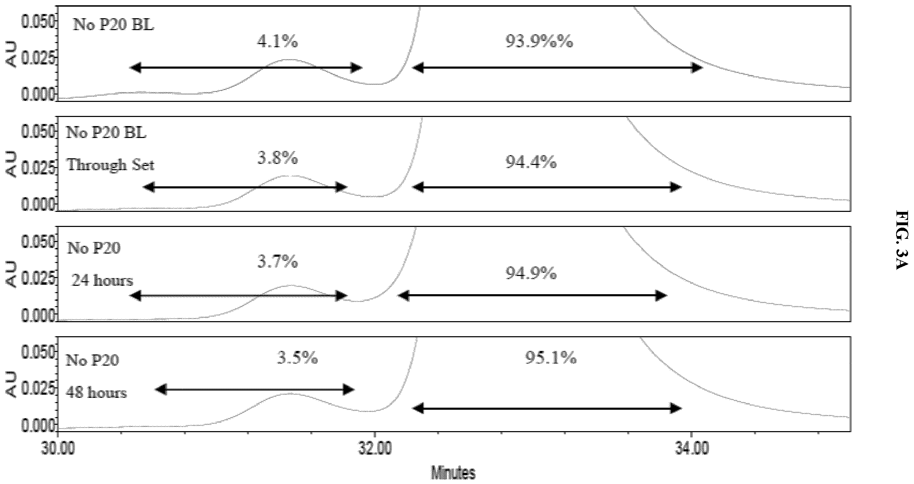

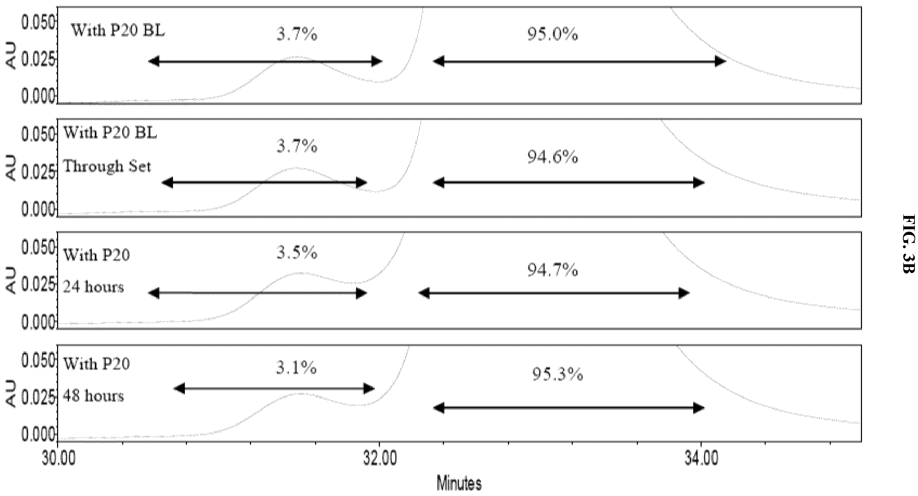

The data showed that less adsorption-related protein loss was observed when P20 SE-HPLC data demonstrated a similar purity (percent complex peak area) for the drug product in formulations held in the infusions set for up to 48 hours (Tables 5
and 6). Three high molecular weight (HMW) species were observed, which were summed to report a qualitative value for HMW species in formulations in the absence of P20 (FIG. 3A) and in the presence of P20 (FIG. 3B). HMW are undesirable because they contribute to decreased stability of the product, increased aggregation and increased immunogenicity.

### Example 3. Measuring degradation of the Mecasermin Rinfabate drug product in formulations in the presence and absence of 0.005% (v/v) polysorbate 20 (P20) by RP-UPLC

This example illustrates exemplary degradation of mecasermin rinfabate by oxidation.

To investigate the extent of degradation of mecasermin rinfabate, Reversed Phase-Ultra-High Performance Chromatography (RP-UPLC) was performed to monitor the oxidation of IGF1 in the IGF-1/IGF1-BP3 protein complex.

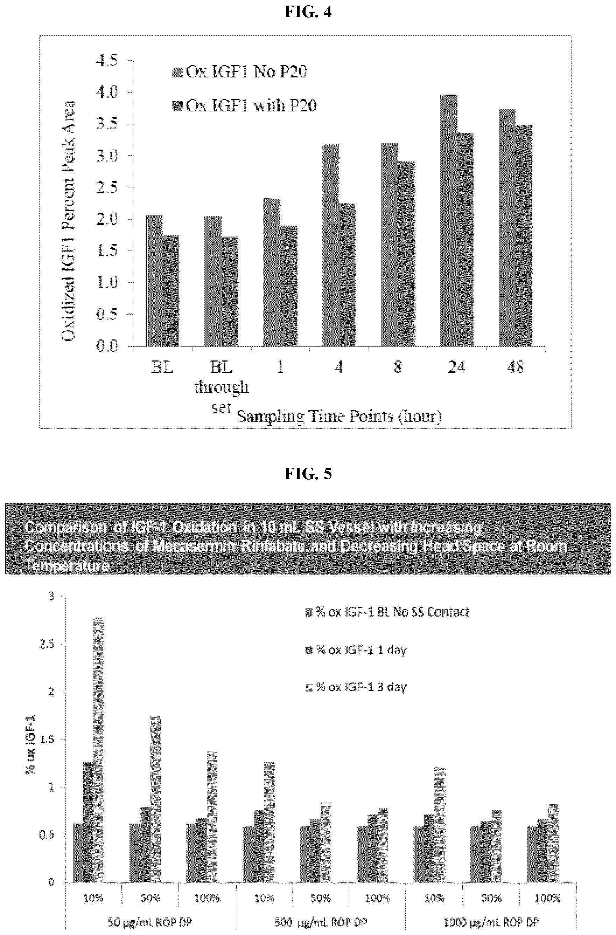

The RP-UPLC data demonstrated a small increase in the percent oxidized IGF1 after 48 hours hold time in the infusion sets when compared to baseline in both formulations (FIG. 4).

This assay also monitors the percent peak areas for IGF-1 and IGFBP-3 proteins separately; the increase in the IGF-1 oxidation coincided with the decrease in the percent IGF-1 peak area while the percent IGFBP-3 did not change significantly. The percent decrease in IGFBP-3 was approximately 0.6% for non-P20 containing formulation as compared with 0.1% when there was P20 present in the formulation. Tables 5 and 6 report the percent oxidized IGF-1 and the sum of the IGF-1 and IGFBP-3 peak areas; the latter is reported as the percent main peak area.

### Example 4. Measuring potency of the Mecasermin Rinfabate drug product in formulations in the presence and absence of 0.005% (v/v) polysorbate 20 (P20)

This example illustrates exemplary effects of the presence of 0.005% (v/v) polysorbate 20 (P20) in a mecasermin rinfabate formulation on potency of the drug product.

To investigate the effects of the presence or absence of 0.005% (v/v) polysorbate 20 (P20) in a formulation on the potency of mecasermin rinfabate drug product, selected samples, at baseline and 48-hour hold time, in formulations containing 0.005% (v/v) P20 or in the absence of 0.005% P20 were tested for potency.

In the worst case scenario where the infusion sets were not primed and flushed with the drug product, there was a 25% potency reduction when the formulation buffer did not contain P20 as compared with only 8% potency reduction when polysorbate 20 was present (Table 7). Although both values may be considered in the range of a typical cell based assay variation (70% to 130%), an approximately 3 fold lower potency is observed when no polysorbate 20 was present in the drug product formulation.

**Table 7. Percent Relative Potency of Drug Product after 48 hours of Hold Time in Formulation with and without Polysorbate 20**

| **Drug Product Sample** | **Percent Relative Potency¹** | |
|---|---|---|
| | **No Polysorbate 20** | **With Polysorbate 20** |
| Baseline | 76% | 90% |
| Drug product samples after 48 hours hold time | 57% | 83% |

| | | |
|---|---|---|
| ¹Relative potency is expressed as percent of the Drug Substance Demo run | | |

Addition of polysorbate 20 improved the overall quality of the product as a higher potency was observed in the presence of polysorbate 20 in the drug product.

Collectively, the results of these studies demonstrated that the quality of the Phase 3 drug product is maintained for up to 48 hours in the infusion set when stored at room temperature with exposure to ambient light and temperature.

### Example 5. Mecasermin Rinfabate drug product in formulations in the presence and absence of 0.005% (v/v) polysorbate 20 (P20) or polysorbate 80 (P80)

In this example, the safety and toxicity of polysorbate 20 and polysorbate 80 exposure in formulations of mecasermin rinfabate drug product were compared.

The nonclinical data for polysorbates 20 and 80 were used to derive IV permitted exposure values of 0.02 mg/kg body weight/day for premature neonates and 0.08 mg/kg body weight/day for full-term infants and children up to 1 year of age (Table 8).

**Table 8. Summary of Exposures to Polysorbate 20 from the rhIGF-1/rhIGFBP-3 Drug Product and Other Sources**

| Patient weight | Daily P20 exposure from rhIGF-1/rhIGFBP-3 | Daily P20 exposure from TPN | Daily P20 exposure from rhIGF-1/rhIGFBP-3 and TPN | Daily P80 exposure from TPN | Daily total P20 + P80 exposure from TPN | Daily P20 exposure with no signs of E-Ferol Toxicity | Daily P80 exposure with no signs of E-Ferol Toxicity |
|---|---|---|---|---|---|---|---|
| 0.4 kg | 0.11 mg | 0.24 mg | 0.36 mg | 15 mg | 15.24 mg | 3.2 mg | 28.8 mg |
| 1.7 kg | 0.47 mg | 0.52 mg | 0.99 mg | 32.5 mg | 33.02 mg | 13.6 mg | 122.4 mg |

| | | | | | | | |
|---|---|---|---|---|---|---|---|
| TPN = Total parenteral nutrition | | | | | | | |

Based on these comparisons, there are no toxicological concerns related to the minor additional amount of polysorbate 20 that patients would receive from the rhIGF-1/rhIGFBP-3 drug product, relative to the amount of polysorbate 20 and polysorbate 80 that patients already would be receiving from total parenteral nutrition products.

### Example 6. Manufacture of Mecasermin Rinfabate in a single-use bag

This example demonstrates the role of contact material in percent oxidation and stability of IGF-1/IGFBP-3.

The drug product manufactured using stainless steel containers or single use bag were analyzed for oxidation. Briefly, the excipients (sodium chloride, sodium acetate, acetic acid and polysorbate 20) were mixed. The excipients were subsequently filtered and mixed with a thawed drug substance which was compounded to drug product, IGF-1/IGFBP-3 complex. The buffer compounded bulk drug product was taken in either a 1000L stainless steel container or 500L single use bag (Mobius MIX0500L bag Gold including magnetic stirrer) and filtered through a filter (Opticap XL10 0.22 µm, Durapore PVDF membrane) into a receiving stainless steel container or bag (including magnetic stirrer). The two batches of the drug product were analyzed for percent of oxidized IGF-1 by RP-UPLC at release and during storage.

**FIG. 5** shows the percentage of oxidized IGF-1 as a function of time and concentration of the drug product in stainless steel in a small-scale model. It was observed that drug product that did not have any contact with the stainless steel was less prone to oxidation of IGF-1, across all concentrations.

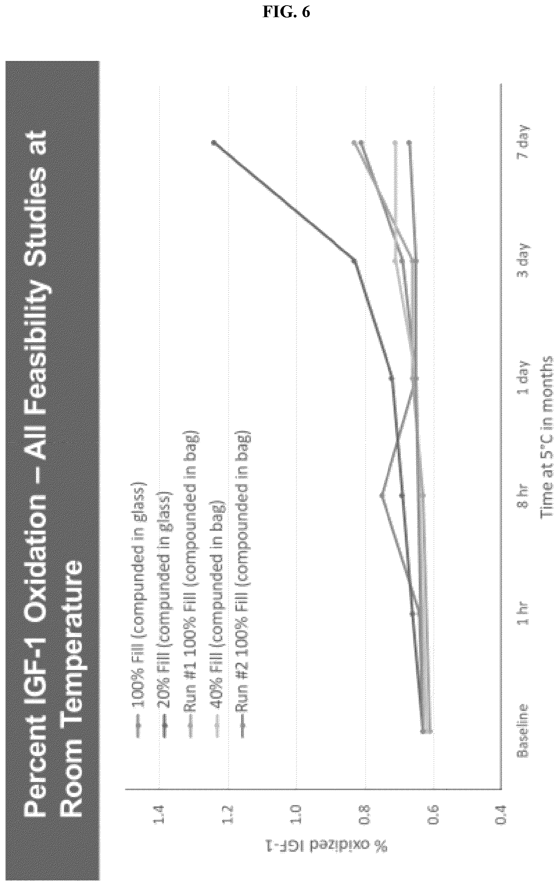

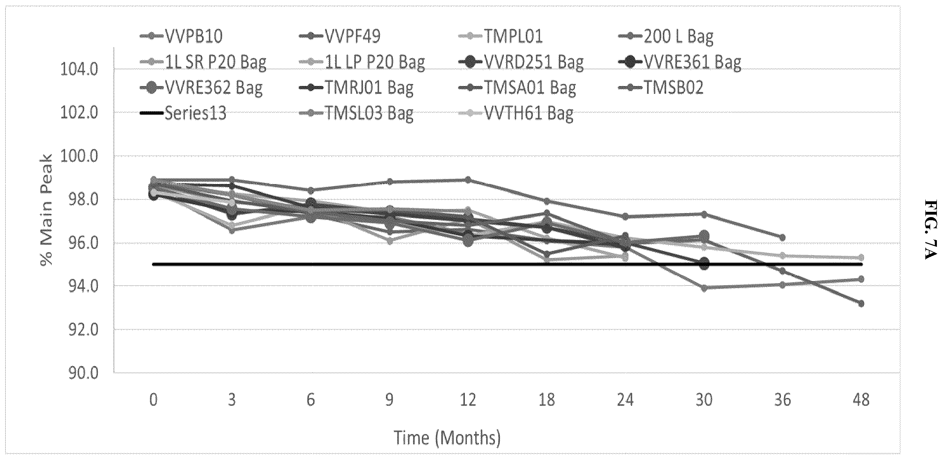

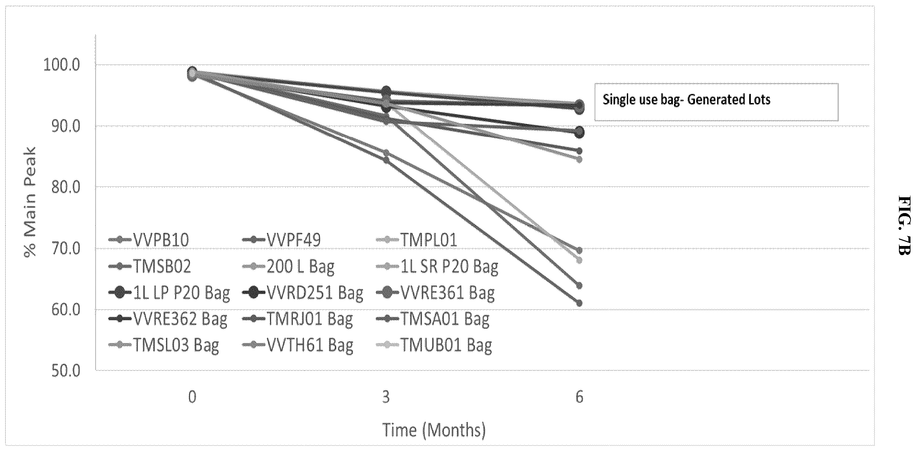

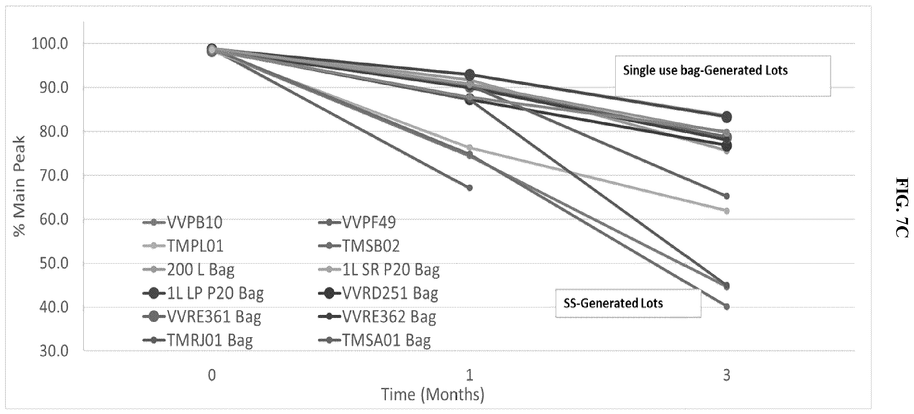

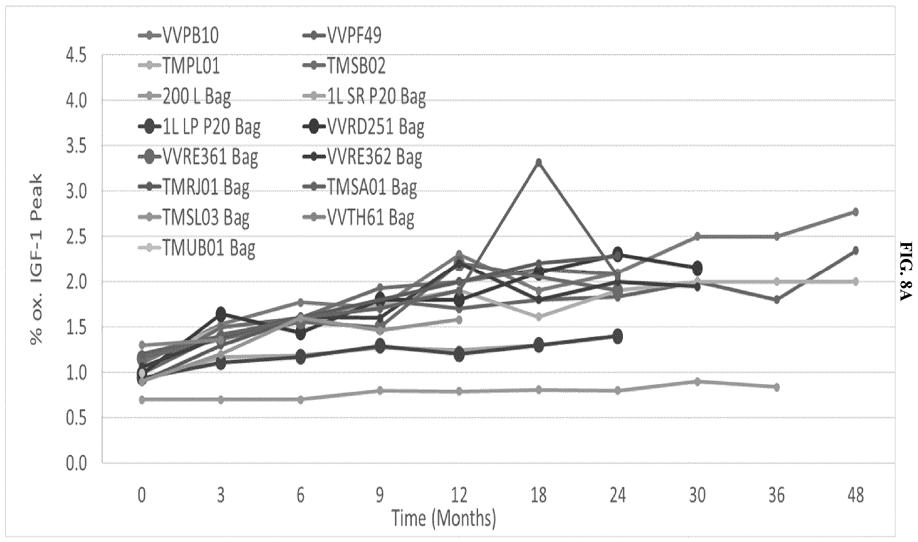

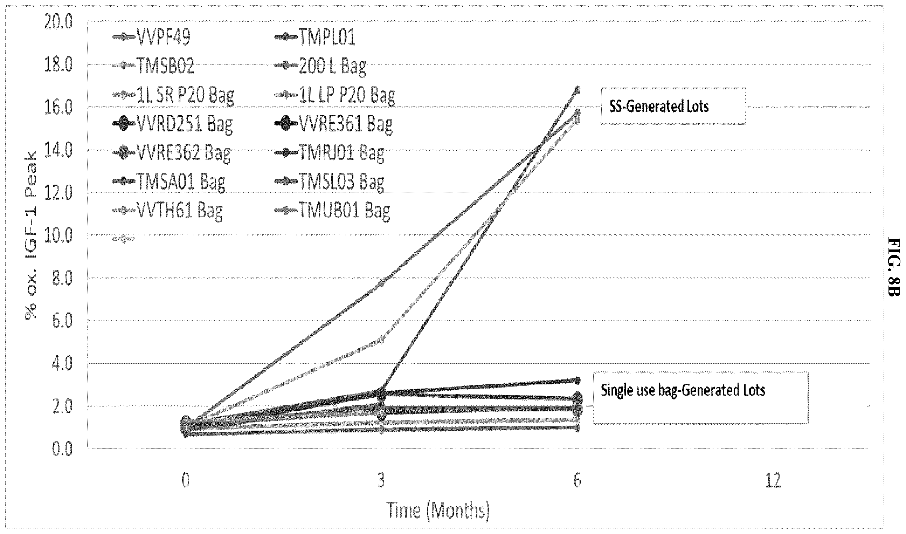

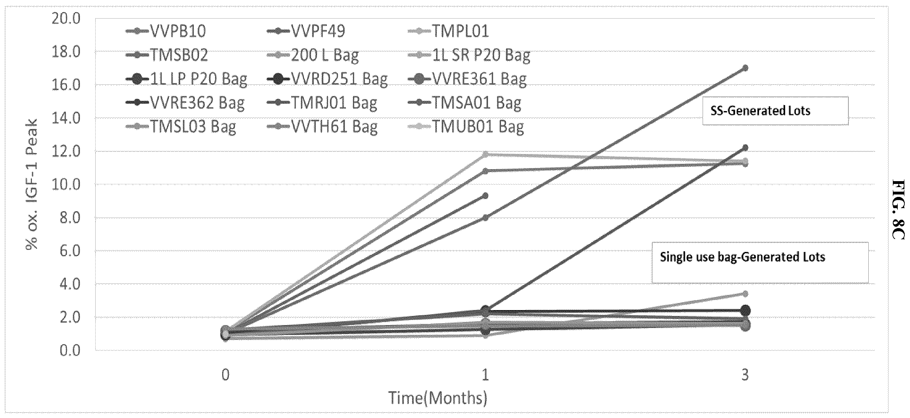

Similar experiment was repeated by compounding the drug product in a glass container and compared to a compounded drug product in a single-use bag. **FIG. 6** summarizes the percent oxidized IGF-1 as a function of time and head space for single-use bag and glass containers. It was observed that drugs compounded in single-use bags yielded the lowest oxidation, followed by the formulations compounded in glass having 20% fill. Next, the stability of the IGF-1 was tested by plotting the percentage of main peak (un-oxidized IGF-1) as a function of time, at different temperatures. It was observed that single- use bag generated drug product preserved the stability of IGF-1 across all temperatures tested, compared to stainless steel vessel-generated drug product. The stability (plotted as percent main peak) are shown in **FIG. 7A****-****FIG. 7C****.** Additionally, the percent oxidized IGF-1 was plotted as a function of time. It was observed that single use bag generated drug product had lower percent oxidized IGF-1 across all temperatures tested, compared to stainless steel vessel-generated drug product. The percent oxidized IGF-1 plotted as function of time are shown in **FIG. 8A****-****FIG. 8C****.**

### Example 7. Treatment of CLD in extremely premature infants

An investigational drug comprising insulin like growth factor -1/insulin-like growth factor binding protein-3 (rhIGF-1/rhIGFBP-3) complex was studied for therapeutic effect in CLD. The drug product formulation used was 50 µg/mL mecasermin rinfabate in 50 mM sodium acetate with 105 mM sodium chloride, at pH 5.5, containing 0.005% (v/v) polysorbate 20.

It is designed as a multicenter, randomized, open-label, controlled, 3-arm study to evaluate the clinical efficacy and safety of the therapeutic composition in preventing human chronic lung disease of prematurity. This study is undertaken on subjects through 12 months corrected age (CA) compared to standard neonatal care in extremely premature infants. The study is reviewed and approved by the institutional review board (IRB)/independent ethics committee (IEC) of the responsible institution.

Purpose: The purpose of this study is to determine if an investigational drug comprising rhIGF-1/rhIGFBP-3 (henceforth, the therapeutic composition) can reduce respiratory complications in extremely premature babies through 12 months corrected age (CA), as compared to extremely premature babies receiving standard neonatal care alone.

Study Subjects: The subjects are between gestational age (GA) of 23 weeks + 0 days and 27 weeks +6 days. Subjects include both sexes. At least fifty subjects are included in the study.

Exclusion criteria: The exclusion criteria include detectable gross malformation, known or suspected chromosomal abnormality, genetic disorder or syndrome, according to the investigator's opinion. The exclusion criteria also include persistent blood glucose level less than (<) 2.5 millimoles per liter (mmols/L) at the baseline visit to exclude severe congenital abnormalities of glucose metabolism; clinically significant neurological disease according to the investigator's opinion; monozygotic multiples; and any other condition that may pose risk to the subject or interfere with the subject's ability to be compliant with the protocol or interfere with the interpretation of results. If the subject is participating or plans to participate in a clinical study of another investigational study drug, device, or procedure (participation in observational studies is permitted on a case-by-case basis) are excluded. If the subject or subject's parent or legally authorized representative(s) is unable to comply with the protocol or is unlikely to be available for long-term follow-up as determined by the investigator, the subject is also excluded.

Details of the Study Design: The primary purpose of the study is prevention of Bronchopulmonary Dysplasia and Chronic Lung Disease of prematurity. It is an open label study, and the intervention model will be Parallel Assignment. The conditions monitored will be BPD and CLD.

250 micrograms/kg/24 hours of the therapeutic composition is administered to one group of participants (Group A) by intravenous administration (IV) from birth up to postmenstrual age (PMA) 29 weeks +6 days. To another group of participants (Groups B), 400 micrograms/kg/24 hours of the therapeutic composition is administered by intravenous administration (IV) from birth up to postmenstrual age (PMA) 29 weeks +6 days. To the third group (Group C or control group), standard neonatal care alone is provided.

The primary outcomes measured is incidence of chronic lung disease (CLD) of prematurity through 12 Months Corrected Age (CA) [Time Frame: Baseline through 12 Months Corrected Age (CA)] CLD is a common adverse outcome of premature birth resulting in recurrent respiratory symptoms requiring treatment with pulmonary medications such as bronchodilators, need for supplementary home oxygen, frequent emergency room visits or hospital readmissions, especially during the first year of life. CLD will be measured by respiratory health care utilization and respiratory symptoms.

The secondary outcomes include incidence of Bronchopulmonary Dysplasia (BPD) at Postmenstrual Age (PMA) 36 Weeks [Time Frame: PMA Week 36]. BPD is a chronic lung disorder characterized by pulmonary immaturity, undifferentiated alveoli with the presence of hyaline membrane and atelectasis, dilated capillaries immersed in the mesenchyme, and a distorted deposition of the extracellular matrix. BPD results in residual effects on pulmonary function and is linked to neurodevelopmental problems during later childhood.

The secondary outcomes also include
- Incidence of Severe Intraventricular Hemorrhage (IVH) Grade III or IV Through Postmenstrual Age (PMA) 40 Weeks [Time Frame: Baseline Through PMA 40 Weeks]
- Incidence of Bronchopulmonary Dysplasia (BPD) at Postmenstrual Age (PMA) 40 Weeks [Time Frame: PMA Week 40]
- Incidence of Chronic Lung Disease (CLD) or Death Through 6 Months Corrected Age (CA) [Time Frame: Baseline through 6 Months Corrected Age (CA)] CLD is a common adverse outcome of premature birth resulting in recurrent respiratory symptoms requiring treatment with pulmonary medications such as bronchodilators, need for supplementary home oxygen, frequent emergency room visits or hospital readmissions, especially during the first year of life. CLD will be measured by respiratory health care utilization and respiratory symptoms.
- Functional Status as Assessed by PREMature Infant Index (PREMII) at Postmenstrual Age (PMA) 40 Weeks [Time Frame: PMA Week 36] PREMII is a Clinician-Reported Outcome (ClinRO) assessment used to capture overall functional maturation of extremely preterm neonates. Functional Status is defined as what the infant can do with respect to 8 key functional areas (feeding, weight gain, thermoregulation, respiratory support, apnea, bradycardia, desaturation events, and oxygen administration), as a reflection of the infant's overall health and development.

### Example 8. Intraventricular Hemorrhage (IVH) treatment in extremely premature low gestational age infants

An investigational drug comprising insulin like growth factor -1/insulin-like growth factor binding protein-3 (rhIGF-1/rhIGFBP-3) complex (50 µg/mL mecasermin rinfabate in 50 mM sodium acetate with 105 mM sodium chloride, at pH 5.5, comprising 0.005% (v/v) polysorbate 20) is studied for therapeutic effect in intraventricular hemorrhage (IVH). It is designed as a multicenter, randomized, open-label, controlled, 3-arm study to evaluate the clinical efficacy and safety of the therapeutic composition in preventing intraventricular hemorrhage. This study is undertaken on subjects through 36 weeks PMA compared to standard neonatal care (SNC) in extremely premature infants with gestational age <26 weeks. The study is reviewed and approved by the institutional review board (IRB)/independent ethics committee (IEC) of the responsible institution.

Purpose: The purpose of this study is to determine if an investigational drug comprising rhIGF-1/rhIGFBP-3 (henceforth, the therapeutic composition) can reduce incidence of intraventricular hemorrhage in extremely premature babies through 36 weeks PMA, as compared to extremely premature babies receiving standard neonatal care alone.

Study Subjects: The subjects are infants with gestational age (GA) of less than 26 weeks or < 25 weeks + 6 days. Subjects include both sexes. At least fifty subjects are included in the study.

Study Design: 250 micrograms/kg/24 hours of the therapeutic composition is administered to one group of participants (Group A) by intravenous administration (IV) from birth up to postmenstrual age (PMA) 29 weeks +6 days. To another group of participants (Groups B), 400 micrograms/kg/24 hours of the therapeutic composition is administered by intravenous administration (IV) from birth up to postmenstrual age (PMA) 29 weeks +6 days. To the third group (Group C or control group), standard neonatal care alone is provided.

In lower gestational age infants, where the incidence of IVH is higher, the incidence of IVH II/III/PHVI is assessed.

The primary outcomes measured are incidence and severity of intraventricular hemorrhage at Postmenstrual Age (PMA) 36 Weeks [Time Frame: PMA Week 36]. IVH is a common adverse outcome of premature birth and will be monitored by cranial ultrasound.

The secondary outcomes include severity and incidence of Bronchopulmonary Dysplasia (BPD) at Postmenstrual Age (PMA) 36 Weeks [Time Frame: PMA Week 36]. BPD is a chronic lung disorder histologically characterized by pulmonary immaturity, undifferentiated alveoli with the presence of hyaline membrane and atelectasis, dilated capillaries immersed in the mesenchyme, and a distorted deposition of the extracellular matrix. BPD results in residual effects on pulmonary function and is associated with neurodevelopmental disabilities during later childhood.

Efficacy analysis would be carried out on infants <26 week GA at 36 week PMA. Relative reduction in IVH grades II/III/IV (PHVI) is assessed. In some embodiments, subjects are followed out to about 40 weeks, 12 months or 24 months CA.

### Example 9. BPD prevention in extremely premature infants

A randomized study for effect of IGF-1/IGFBP-3 in complications of extreme prematurity, including BPD prevention, was undertaken with an intervention model of parallel assignment. The study was conducted in multiple centers in Italy, the Netherlands, Poland, Sweden, the United Kingdom and the United States between 18 Jun 2010 and 30 March 2016.

The drug Mecasermin Rinfabate, that is IGF-1/IGFBP-3, was administered as continuous intravenous infusion in subjects from Study Day 0 (day of birth) up to and including PMA 29 weeks + 6 days, when the subject's endogenous production of IGF-1 is considered sufficient to maintain physiologic serum IGF-1 levels. After discontinuation of study drug infusion, each subject will be followed to PMA 40 weeks ± 4 days. The study was intended to determine the rhIGF-1/rhIGFBP-3 Dose, Administered as a Continuous Infusion (CI), required to establish and maintain longitudinal serum IGF-1 levels within physiological levels in premature infants, to prevent retinopathy of prematurity. This was a Phase 2, Randomized Controlled, Assessor-blind, dose confirming, pharmacokinetic, safety and efficacy of rhIGF-1/rhIGFBP-3. 61 Participants received insulin-like growth factor (rhIGF-I)/insulin-like growth factor binding protein-3 (rhIGFBP-3) 250 micrograms per kilogram (mcg/kg) for 24 hours through continuous intravenous (IV) infusion from Day 0 up to 29 weeks 6 days of post-menstrual age (PMA). As a control group, 60 participants received standard of care alone. Table 3 illustrates the participant flow of the overall study.

**Table 9 Participant Flow: Overall Study**

| | rhIGF-1/rhIGFBP-3 | Standard of Care (Control) |
|---|---|---|
| STARTED | 61 | 60 |
| COMPLETED | 46 | 46 |
| NOT COMPLETED | 15 | 14 |
| Withdrawal by Subject | 2 | 1 |
| Adverse Event | 11 | 9 |
| Protocol Deviation | 2 | 2 |
| Administrative Decision | 0 | 1 |
| Other Unspecified | 0 | 1 |

**Table 10 illustrates the population in the study.**

| | | rhIGF-1/rhIGFBP-3 | | Standard of Care (Control) | | Total | |
|---|---|---|---|---|---|---|---|
| Overall Participants Analyzed | | 61 | | 60 | | 121 | |
| [Units: Participants] | | | | | | | |
| Age | | 25.60 (1.207) | | 25.62 (1.397) | | 25.61 (1.300) | |
| [Units: Weeks] | | | | | | | |
| Mean (Standard Deviation) | | | | | | | |
| Sex: Female, Male | | | | | | | |
| [Units: Participants] | | | | | | | |
| Count of Participants | | | | | | | |
| | Female | 22 | 36.1% | 21 | 35.0% | 43 | 35.5% |
| | Male | 39 | 63.9% | 39 | 65.0% | 78 | 64.5% |

The secondary outcomes included among other parameters:
- Time to Discharge From Neonatal Intensive Care (TDNIC) [ Time Frame: Day 0 to 40 Weeks Post Menstrual Age (EOS)]
- Number of Participants With Bronchopulmonary Dysplasia (BPD) [Time Frame: At 36 Weeks Post Menstrual Age]
- Severity of BPD as mild, moderate and severe were based on the National Institute of Child Health and Human Development (NICHD) guidelines for preterm infants born at gestational age (GA) less than (<) 32 weeks.
- Mild: oxygen requirement during the first 28 days but in room air at PMA 36 weeks or discharge to home, whichever comes first.
- Moderate BPD: oxygen requirement during the first 28 days and oxygen <30 percent (%) at PMA 36 weeks or discharge to home, whichever comes first.
- Severe BPD: oxygen requirement during the first 28 days and oxygen greater than equal (≥)30% through head hood or nasal canula, or continuous positive airway pressure, or mechanical ventilation, or high flow nasal cannula ≥2 L/min at PMA 36 weeks or discharge to home, whichever comes first.
- Rate of Change in Body Weight [Time Frame: Day 0 to 40 Weeks Post Menstrual Age (EOS) ] The rate of change is the rate of specific body weight change per day in kilogram (kg).
- Rate of Change in Length [ Time Frame: Day 0 to 40 Weeks Post Menstrual Age (EOS) ]
- The rate of change is the length change per day in centimeter (cm). Number of Participants With Treatment Emergent Adverse Event (TEAE) and Treatment Emergent Serious Adverse Event (TESAE) [ Time Frame: Day 0 to 40 Weeks Post Menstrual Age (EOS) ]
- An adverse event (AE) was any untoward medical occurrence in a participant who received study drug without regard to possibility of causal relationship. A serious adverse event (SAE) was an AE resulting in any of the following outcomes or deemed significant for any other reason: death; initial or prolonged in-patient hospitalization; life-threatening experience (immediate risk of dying); persistent or significant disability/incapacity; congenital anomaly.
- Treatment-emergent adverse event was defined as the onset of any AE or if the severity of a pre-existing AE worsened any time on or after the date of first dose of investigational product.
- Percentage of Serum IGF-1 Concentrations Falling Within Target Range After Infusion of rhIGF-1/rhIGFBP-3 [Time Frame: Day 0 to 40 Weeks Post Menstrual Age (EOS)]
- Serum samples were collected from treated and control participants for quantitation of IGF-1 using validated immunoassays. Target range of serum IGF-1 was 28-109 mcg/L. The percentage of serum IGF-1 levels across treated participants that fall within the range was reported.
- Serum Concentrations of IGFBP-3 After Intravenous (IV) Infusion of rhIGF-1/rhIGFBP-3 [Time Frame: Day 0 and Week 40 Post Menstrual Age]
- Serum Concentrations of Acid Labile Sub-unit (ALS) After Intravenous (IV) Infusion of rhIGF-1/rhIGFBP-3 [Time Frame: Day 7 and Week 40 Post Menstrual Age]

**Table 11 illustrates the measured values of BPD as secondary outcome.**

| | | rhIGF-1/rhIGFBP-3 | Standard of Care (Control) |
|---|---|---|---|
| Participants Analyzed | | 47 | 49 |
| [Units: Participants] | | | |
| Number of Participants With Bronchopulmonary Dysplasia (BPD) | | | |
| [Units: Participants] | | | |
| | No BPD | 4 | 4 |
| | Mild | 23 | 16 |
| | Moderate | 9 | 5 |
| | Severe | 10 | 22 |
| | Unable to determine | 1 | 2 |

No statistical analysis provided for Number of Participants With Bronchopulmonary Dysplasia (BPD).

### Example 10. Exposure Response Analysis of IGF-1 Following Administration of IGF-1 and IGFBP-3 in Premature Infants

This example illustrates the exposure response of IGF-1 in serum in extremely premature infants treated with IGF-1/IGFBP-3.

Briefly, a human recombinant form of the naturally occurring protein complex of IGF-1 and insulin-like growth factor binding protein-3 (rhIGF-1/rhIGFBP-3) is administered to premature infants.

About 96 premature infants were administered rhIGF-1/rhIGFBP-3 daily in an intravenous infusion, starting within 24 hours of birth, and ending at 30 weeks post-menstrual age (PMA). Control subjects received standard neonatal care (SOC). The development of BPD was assessed by oxygen challenge testing at PMA of 36 weeks.

The median gestational age at birth was 25.9 weeks (and ranged from 23.1-27.9 weeks). Median birth weight was 0.790 (ranged from 0.425 - 1.22). Logistic regression models were constructed to assess the relationship between IGF-1 exposure metrics and the probability of developing BPD (i.e., No/mild BPD vs. Moderate/Severe BPD). Sources of variability in the BPD response such as gestational age, birth weight, sex, oxygen saturation on day 1, APGAR score at 1 min., and study arm were assessed and considered.

Clinical trial simulations were then performed to identify the dosing regimen that would result in serum IGF-1 levels that correlated with BPD prevention or amelioration, i.e. provided high probability of No/mild BPD symptoms in the premature infants receiving treatment relative to standard of care. A total of 500 trials of 75 subjects treated with 250 and 400 micrograms/kg/day rhIGF-1/rhIGFBP-3 or no dose (SOC) in a 1:1:1 ratio were simulated based on a population PK model.

The results from these simulations showed that higher observed average IGF-1 levels at day 7 (CavD7) were associated with a greater probability of No/Mild BPD, showing a statistically significant exposure-response relationship. The probability of No/Mild or Moderate/Severe BPD was determined based on simulated CavD7 in each trial according to the above exposure-response model.

In addition, effects of other variables were observed. For example, statistically significant effects of weight at birth and sex were observed, whereby larger females are expected to have a higher probability of No/Mild BPD. Neither oxygen saturation at birth nor APGAR score at 1 min were predictors of No/Mild BPD.

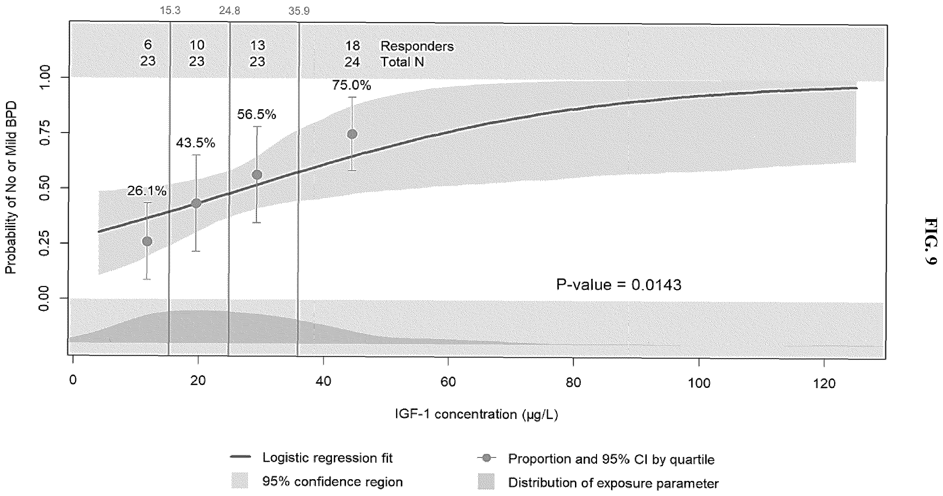

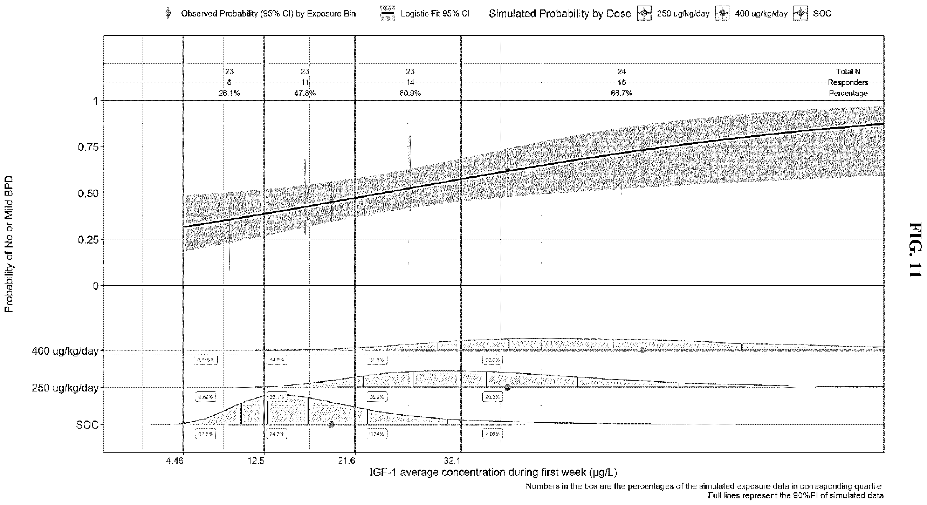

The results from the simulations showed that treatment with rhIGF-1/rhIGFBP-3 treatments resulted in a higher probability of developing no BPD or mild BPD relative to standard of care for treating CLD (FIG. 9, Table 12). A comparison between subjects treated with 250 and 400 micrograms/kg/day rhIGF-1/rhIGFBP-3 or no dose (SOC) is seen at FIG. 11.

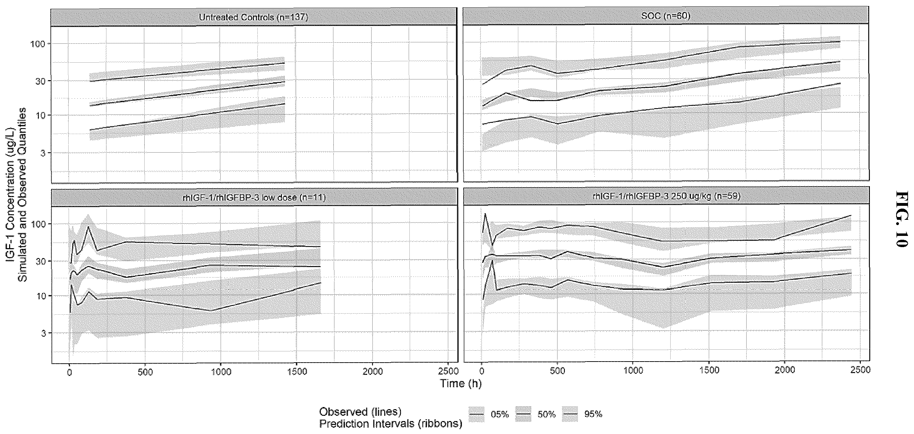

Based on concentration-time data of IGF-1 in premature infants, the pharmacokinetic characteristics of infused (or exogenous) IGF-1 was evaluated. Overall, a one-compartment disposition model with linear elimination combined to a baseline model taking into account the change in weight over time adequately characterized the concentration-time profiles of infused and endogenous IGF-1, respectively. In addition, the model included between-subject variability (BSV) on volume of distribution and clearance, as well as BSV on endogenous models (IGF-1 concentration at birth (C_{endo,0}), and in more mature infants on IGF-1 half-live for the initial decline (C_{end0, mat})). Since age and weight are important components for the endogenous production of IGF-1, as well as clearance of IGF-1 following IV infusion of rhIGF-1/rhIGFBP-3, a maturation model was developed to characterize the body weight increase as a function of PMA in premature infants. The model was validated as shown by graphs of IGF-1 concentration over time for subjects administered 250 micrograms/kg/day rIGF-1/rIGFBP-3 or a lower dose, relative to control subjects that received standard of care or untreated controls (FIG. 10).

**Table 12. Clinical Trial Simulations in 75 and 150 Premature Infants to Compare Probability of Success (i.e., no/mild BPD) in the Active group Compared to Standard of Care**

| **Arm** | **CavD7 (µg/L)** | **Prob (%) 95%CI** | **Median OR (95% CI)** | |
|---|---|---|---|---|
| Number of subjects (1:1:1) | | | **75** | **150** |
| 400 micrograms/kg/day | 50.3 | 73.2 [52.96,86.93] | 0.345 [0.831,1.52] | 0.351 [0.135, 0.901] |
| 250 micrograms/kg/day | 36.8 | 61.9 [47.85, 74.14] | 0.531 [0.138, 2.21] | 0.548 [0.212, 1.34] |
| SOC | 19.2 | 45.1 [34.47, 56.21] | | |

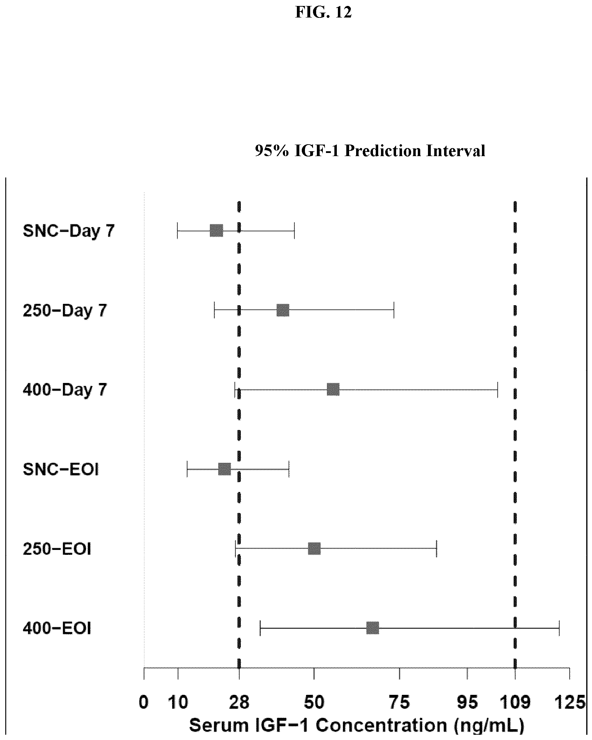

At the 400 micrograms/kg/24 hours regimen of rIGF-1/rIGFBP-3, a population PK model was developed using standard of care data and IGF-1 data. The model was used to simulate 95% prediction intervals of IGF-1 PK concentrations over duration of treatment to predict where the mean and 5th and 95th intervals would fall with respect to target therapeutic range (FIG. 12).

The results showed that mean IGF-1 levels were higher at a dose of 400 micrograms/kg/24 hours than 250 micrograms/kg/24 hours. By day 7, approximately 95% of the subjects administered 400 micrograms/kg/24 hours are expected to be in the therapeutic range. At EOI, 90% of subjects are expected to be below 109 ng/ml and 95% of subjects above 28 ng/ml. Majority of the subjects administered a dose of 400 micrograms/kg/24 hours are expected to achieve target IGF-1 levels (28 to 109 ng/ml) by day 7 and are expected to have a low risk for overshooting target IGF-1 levels by end of infusion. End of infusion is, for example, about 28, 29, 30, 31, or 32 weeks post-menstrual age (PMA). In some embodiments, end of infusion is about 28 weeks post menstrual age (PMA). In some embodiments, end of infusion is 29 weeks post menstrual age (PMA). In some embodiments, end of infusion is about 30 weeks post menstrual age (PMA). In some embodiments, end of infusion is about 31 weeks post menstrual age (PMA). In some embodiments, end of infusion is about 32 weeks post menstrual age (PMA).

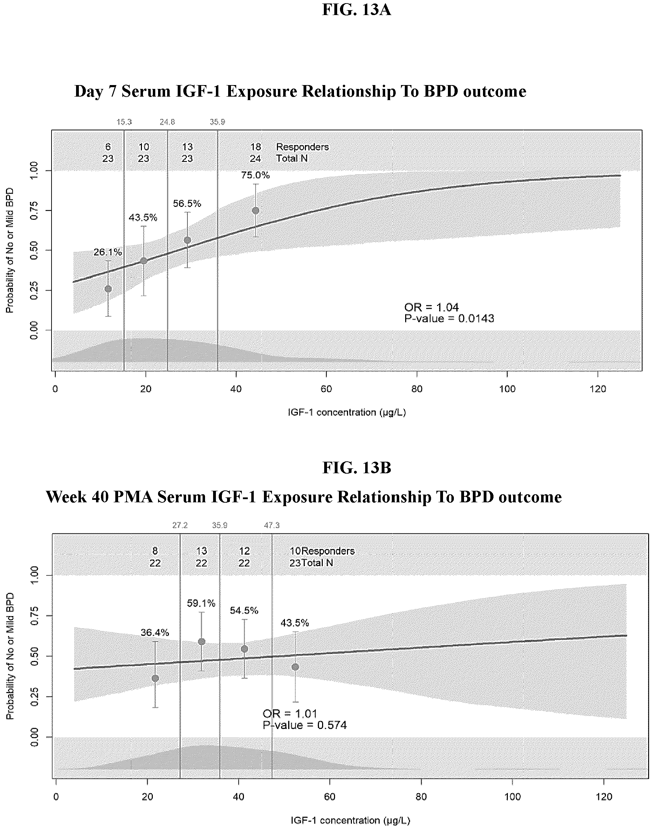

In order to evaluate whether early or higher IGF-1 serum exposure resulted in better BPD outcomes, IGF-1 serum exposure was measured at day 7 (FIG. 13A) and week 40 PMA (FIG. 13B).

Exploratory logistic regression analyses were conducted to explore the relationship between serum IGF-1 exposure at different time points and BPD outcome. Earlier time points showed significant relationship between higher IGF-1 exposure and favorable BPD outcome (mild or no BPD). To reduce bias, IGF-1 average concentrations over 7 days was selected for model development Weight at birth (higher WT-better outcome) and gender (females-better outcome) were identified as covariates influencing BPD outcome.

The simulation results further showed that rhIGF-1/rhIGFBP-3 administered at a 400 micrograms/kg/24 hours regimen was associated with a greater probability of no BPD or mild BPD relative to the 250 micrograms/kg/24 hours regimen. Based on simulation studies, the higher dose of 400 micrograms/kg/24 hours was found to be highly correlated with BPD prevention and amelioration of CLD.

While certain compounds, compositions and methods described herein have been described with specificity in accordance with certain embodiments, the following examples serve only to illustrate the compounds of the invention and are not intended to limit the same.

The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification. The publications, websites and other reference materials referenced herein to describe the background of the invention and to provide additional detail regarding its practice are hereby incorporated by reference.

### Numbered paragraphs:

1. A composition comprising a protein complex comprising recombinant insulin-like growth factor 1 (rIGF-1), recombinant insulin-like growth factor binding protein 3 (rIGFBP-3), and a polysorbate surfactant at a concentration of between about 0.0025% and 0.0075%, wherein the rIGF-1 and rIGFBP-3 are complexed in equimolar amounts.
2. The composition of paragraph 1, wherein the rIGF-1/IGFBP-3 is at a concentration of about 10 micrograms/mL - 1000 micrograms/mL.
3. The composition of paragraph 1 or 2, wherein the polysorbate surfactant is selected from polysorbate 20 (P20) or polysorbate 80 (P80).
4. The composition of paragraph 3, wherein the polysorbate surfactant is P20.
5. The composition of paragraph 1 or 2, wherein the polysorbate surfactant is present in the composition at a concentration of about 0.005%.
6. The composition of paragraph 1 or 2, wherein less than 20% of the IGF-1 exists as oxidized species after storage for about 6 months at 25 °C.
7. The composition of paragraph 1 or 2, wherein less than 10 % of the IGF-1 exists as oxidized species after storage for about 3 months at 40 °C.
8. The composition of any one of the preceding paragraphs, wherein the % of oxidized species is determined by Reversed Phase-Ultra Performance Liquid Chromatography (RP-UPLC).
9. The composition of any one of preceding paragraphs, wherein the IGFBP-3 comprises less than 5% of trisulfide variants.
10. The composition of any one of the preceding paragraphs further comprising a buffer comprising sodium acetate, acetic acid, and/or sodium chloride.
11. The composition of paragraph 10, wherein the sodium acetate or acetic acid is at a concentration of between about 10 and 100 mM.
12. The composition of paragraph 10, wherein the sodium acetate or acetic acid is at a concentration of about 50 mM.
13. The composition of any one of paragraphs 10-12, wherein the sodium chloride is at a concentration of about 20 mM and 200 mM.
14. The composition of paragraph 13, wherein the sodium chloride is at a concentration of about 105 mM.
15. The composition of any one of the preceding paragraphs, wherein the composition is at a pH between about 5.0 and 7.0.
16. The composition of paragraph 15, wherein the pH is about 5.3 - 5.8.
17. The composition of claim 1, wherein the rIGF-1/IGFBP-3 is at a concentration of about 45 - 55 micrograms/mL.
18. The composition of paragraph 17, wherein the rIGF-1/IGFBP-3 is at a concentration of about 50 micrograms/mL.
19. The composition of paragraph 17 or 18, wherein the composition is suitable for intravenous administration.
20 The composition of any one of the preceding paragraphs, wherein the composition has a storage life of between about 20 months to 24 months at a temperature of between 2-8 °C.
21. The composition of paragraph 20, wherein the composition has a storage life of at least 24 months at a temperature of between 2-8 °C.
22. The composition of any one of the preceding paragraphs, wherein the composition is stable at room temperature and/or ambient light for at least 8 hours, 12 hours, 24 hours, 36 hours, or 48 hours.
23. The composition of any one of the preceding paragraphs, wherein the composition comprising recombinant insulin-like growth factor 1 (rIGF-1), recombinant insulin-like growth factor binding protein 3 (rIGFBP-3), and polysorbate 20 is more stable at room temperature than a composition comprising recombinant insulin-like growth factor 1 (rIGF-1), recombinant insulin-like growth factor binding protein 3 (rIGFBP-3) without polysorbate 20.
24. The composition of any one of paragraphs 1-23, wherein the composition comprising recombinant insulin-like growth factor 1 (rIGF-1), recombinant insulin-like growth factor binding protein 3 (rIGFBP-3), and polysorbate 20 has less oxidation at room temperature than a composition comprising recombinant insulin-like growth factor 1 (rIGF-1), recombinant insulin-like growth factor binding protein 3 (rIGFBP-3) without polysorbate 20.
25. The composition of any one of paragraphs 1-24, wherein the % of the oxidized IGF-1 species does not increase more than 12% upon storage at a temperature of about 25 °C after six months.
26. The composition of any one of paragraphs 1-24, wherein the % of the oxidized IGF-1 species does not increase more than 10% upon storage at a temperature of about 25 °C after six months.
27. A pharmaceutical composition comprising the composition of any one of the preceding claims and a pharmaceutically acceptable carrier.
28. A method of treating or preventing intraventricular hemorrhage (IVH), bronchopulmonary dysplasia (BPD) or chronic lung disease of prematurity (CLD) comprising administering to a subject in need of treatment a composition of any one of the preceding claims.
29. A method of treating or preventing intraventricular hemorrhage (IVH), bronchopulmonary dysplasia (BPD) or chronic lung disease of prematurity (CLD) comprising administering to a subject in need thereof a composition comprising recombinant insulin-like growth factor 1 (rIGF-1), recombinant insulin-like growth factor binding protein 3 (rIGFBP-3), and a polysorbate 20 surfactant.
30. A method of treating or preventing intraventricular hemorrhage (IVH), bronchopulmonary dysplasia (BPD) or chronic lung disease of prematurity (CLD) comprising administering to a subject in need thereof a composition comprising recombinant insulin-like growth factor 1 (rIGF-1), recombinant insulin-like growth factor binding protein 3 (rIGFBP-3), and a polysorbate surfactant at a concentration of between about 0.0025% and 0.0075%, wherein the rIGF-1 and rIGFBP-3 are complexed in equimolar amounts, and wherein the rIGF-1/IGFBP-3 is at a concentration of about 10 micrograms/mL - 1000 micrograms/mL.
31. A method of treating or preventing intraventricular hemorrhage (IVH), bronchopulmonary dysplasia (BPD) or chronic lung disease of prematurity (CLD) comprising administering to a subject in need thereof a composition comprising recombinant insulin-like growth factor 1 (rIGF-1), recombinant insulin-like growth factor binding protein 3 (rIGFBP-3), wherein the subject in need is from 23 to 25 weeks + 6 days gestational age.
32. A method of treating or preventing intraventricular hemorrhage (IVH), bronchopulmonary dysplasia (BPD) or chronic lung disease of prematurity (CLD) comprising administering to a subject in need thereof a composition comprising recombinant insulin-like growth factor 1 (rIGF-1), recombinant insulin-like growth factor binding protein 3 (rIGFBP-3), at a therapeutic amount sufficient to achieve reduced incidence of intraventricular hemorrhage relative to a control.
33. The method of paragraph 32, wherein the reduced incidence is achieved between about 36 to 40 weeks gestational age as assessed by ultrasound or magnetic resonance imaging (MRI).
34. The method of paragraph 33, wherein the reduced incidence is achieved by 36 weeks.
35. The method of paragraph 33, wherein the reduced incidence is achieved by 40 weeks.
36. The method of paragraph 32, wherein the reduced incidence is assessed by ultrasound.
37. The method of paragraph 32, wherein the reduced incidence for intraventricular hemorrhage (IVH) is at least about 30% reduction in incidence of grade II, grade III, or grade IV IVH.
38. The method of treating or preventing intraventricular hemorrhage (IVH), bronchopulmonary dysplasia (BPD) or chronic lung disease of prematurity (CLD) comprising administering to a subject in need thereof the composition of claim 1 or 2.
39. The method of paragraph 29 or 30, wherein the polysorbate surfactant is selected from polysorbate 20 (P20) or polysorbate 80 (P80).
40. The method of paragraph 39, wherein the polysorbate surfactant is P20.
41. The method of paragraph 29 or 30, wherein the polysorbate is at a concentration of 0.005%.
42. The method of paragraph 29 or 30, wherein less than 20% of the IGF-1 exists as oxidized species after storage for about 3 months.
43. The method of paragraph 29 or 30, wherein the % of oxidized species is determined by RP-UPLC.
44. The method of paragraph 29 or 30, wherein the IGFBP-3 comprises less than 5% of trisulfide variants.
45. The method of paragraph 29-44, wherein the subject in need of treatment is an infant.
46. The method of paragraph 45, wherein the infant was prematurely born by at least 1 week, 2 weeks, 3 weeks, 4 weeks, 5 weeks, 6 weeks, 7 weeks, 8 weeks, 9 weeks, 10 weeks, 11 weeks, 12 weeks, 1 month, 2 months or 3 months.
47. The method of paragraph 45, wherein the infant was born at less than 32 weeks of gestational age (GA).
48. The method of paragraph 45, wherein the infant was born at less than 28 weeks of gestational age (GA).
49. The method of paragraph 45, wherein the infant was born at between < 26 weeks of gestational age (GA).
50. The method of paragraph 45, wherein the infant is between about 23 to 34 weeks of postmenstrual age (PMA).
51. The method of paragraph 50, wherein the infant is about 23 weeks of postmenstrual age (PMA).
52. The method of paragraph 50, wherein the infant is below 34 weeks of postmenstrual age (PMA).
53. The method of any one of paragraphs 29-52, wherein the composition is administered intravenously.
54. The method of any one of paragraphs 29-52, wherein the composition is administered at a dosage of about 100 to 1000 micrograms/kg/24 hours.
55. The method of paragraph 54, wherein the composition is administered at a dosage of about 800 micrograms/kg/24 hours.
56. The method of paragraph 54, wherein the composition is administered at a dosage of about 400 micrograms/kg/24 hours.
57. The method of paragraph 54, wherein the composition is administered at a dosage of about 250 micrograms/kg/24 hours.
58. The method of any one of paragraphs 51-54, wherein the composition is administered once, twice, three times, or four times in a 24 hour period.
59. The method of paragraph 58, wherein the composition is administered once in a 24 hour period.
60. The method of paragraph 58, wherein the composition is administered by continuous infusion for the 24 hour period.
61. The method of any one of paragraphs 29-60, wherein the composition is administered from the time of birth up to postmenstrual age (PMA) of about 23 to 34 weeks.
62. The method of any one of paragraphs 29-60, wherein the composition is administered from the time of birth up to PMA of about 23 to 32 weeks.
63. The method of any one of paragraphs 29-60, wherein the composition is administered from the time of birth up to PMA of about 29 weeks plus 6 days.
64. The method of any one of paragraphs 29-63, wherein the subject has reduced IGF-1 serum levels.
65. The method of any one of paragraphs 29-64, wherein administration of the composition results in elevated IGF-1 serum levels in comparison to a baseline value.
66. The method of paragraph 65, wherein the IGF-1 serum levels are elevated by at least about 25% - 50% in comparison to a baseline value.
67. The method of paragraph 65, wherein the IGF-1 serum levels remain elevated for at least 24 hours following administration.
68. The method of paragraph 65, wherein the IGF-1 serum levels remain elevated for about 48 hours.
69. The method of paragraph 65, wherein the IGF-1 serum levels remain elevated for at least about 7 days.
70. The method of paragraph 65, wherein the IGF-1 serum levels remain elevated through postmenstrual age (PMA) of at least about 23 weeks to 34 weeks.
71. The method of paragraph 65, wherein the IGF-1 serum levels remain elevated through postmenstrual age (PMA) of at least about 23 weeks to 12 months.
72. The method of any one of paragraphs 29-71, wherein the administration of the composition results in reduced incidence of intraventricular hemorrhage through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 8 months, 10 months, 12 months or 24 months.
73. The method of any one of paragraphs 29-72, wherein the administration of the composition additionally results in reduced incidence of bronchopulmonary dysplasia through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 6 months, 8 months, 10 months, 12 months or 24 months.
74. The method of any one of paragraphs 29-73, wherein the administration of the composition results in increased Functional Status as Assessed by PREMature Infant Index (PREMII) through postmenstrual age (PMA) 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, or 40 weeks.
75. The method of any one of paragraphs 29-74, wherein administration of the composition results in elevated IGF-1 serum target levels of between about 28-109 ng/ml.
76. The method of claim 72 or 73, wherein the reduced incidence is at least about 20% - 50% relative to an untreated control.
77. The method of paragraph 72 or 73, wherein the reduced incidence is at least about 20 - 50% relative to a control receiving standard of care.
78. The method of paragraph 74, wherein the increased functional status is at least about 20% - 50% relative to an untreated control.
79. The method of paragraph 74, wherein the increased functional status is at least about 20 - 50% relative to a control receiving standard of care.
80. A method for manufacturing a pharmaceutical composition, the method comprising providing a protein complex comprising insulin-like growth factor 1 (IGF-1) and insulin-like growth factor binding protein 3 (IGFBP-3), wherein the method comprises a use of a single-use bag during compounding the complex with a formulation solution, thereby producing a pharmaceutical composition, wherein an increase is oxidized species of the IGF-1 in the pharmaceutical composition is less than 20 % upon storage for 6 months at 25 °C.
81. The method of paragraph 80, wherein the formulation solution comprises a surfactant.
82. The method of paragraph 80, wherein the single-use bag is further used during a filtration step.
83. The method of paragraph 82, wherein the method comprises filling at least 30% of the single-use bag with the protein complex during the compounding step and/or the filtration step.
84. The method of paragraph 82, wherein the method comprises filling at least 40% of the single-use bag with the protein complex during the compounding step and/or the filtration step.
85. The method of paragraph 82, wherein the method comprises filling at least 75% of the single-use bag with the protein complex during the compounding step and/or the filtration step.
86. The method of paragraph 82, wherein the method comprises filling 75-90% of the single-use bag with the protein complex during the compounding step and/or the filtration step.
87. The method of paragraphs 80-86, wherein the single-use bag is about or greater than 500L.
88. The method of paragraphs 80-87, wherein an increase in oxidized species of the IGF-1 is less than 5 % upon storage for 6 months at 25 °C.
89. The method paragraphs 80-88, wherein less than 2 % of the IGF-1 exists as oxidized species in the protein complex upon storage for 6 months at 25 °C.
90. The method of paragraphs 80-89, wherein less than 10 % of the protein complex exists as low molecular weight species upon storage for 3 months at 40 °C.
91. The method of paragraphs 80-90, wherein less than 5 % of the protein complex exists as low molecular weight species upon storage for 6 months at 40 °C.
92. A composition comprising an isolated protein complex comprising insulin-like growth factor 1 (IGF-1) and insulin-like growth factor binding protein 3 (IGFBP-3) manufactured by the method of any one of claims 80-91.
93. The composition of paragraph 92, wherein the composition comprises 50 mM sodium acetate, 105 mM sodium chloride, and 0.005% (v/v) P20 at pH 5.5.
94. A composition comprising a protein complex comprising recombinant insulin-like growth factor 1 (rIGF-1) and recombinant insulin-like growth factor binding protein 3 (rIGFBP-3) in equimolar amounts, polysorbate 20 surfactant at a concentration of about 0.005% (v/v), and a buffer comprising sodium acetate, acetic acid, and/or sodium chloride, wherein the composition has a pH of about 5.3-5.8, wherein the rIGF-1/IGFBP-3 is at a concentration of about 50 micrograms/mL, and wherein less than 1.5% of the IGF-1 exists as oxidized species at release of the product.

## Claims

1. An aqueous liquid pharmaceutical composition for use in treatment of chronic lung disease in a neonate by intravenous administration as a dose 400 micrograms/kg/24 hours to 500 micrograms/kg/24 hours, said composition comprising:
a. a therapeutic protein complex comprising rIGF-1/IGFBP-3 in the range 45-55 micrograms/mL
b. a non-ionic surfactant in the range 0.0025% to 0.0075%
c. a pH in the range 5.0 to 7.0.

2. A pharmaceutical composition for use according to claim 1 wherein the chronic lung disease is bronchopulmonary dysplasia.

3. A pharmaceutical composition for use according to claim 1 or 2, wherein the surfactant minimises adsorption-related product loss.

4. A pharmaceutical composition for use according to claim 3, wherein the need to prime and flush intravenous infusion sets prior to drug product administration is eliminated.

5. A pharmaceutical composition for use according to claim 3 or 4, wherein there is a reduction in loss of drug product and improving potency.

6. A pharmaceutical composition for use according to any one of claims 1 to 5, wherein the composition delivers at least 90% of the dose of the therapeutic protein complex to the neonate in an active form.

7. A pharmaceutical composition for use according to any one of claims 1 to 6, wherein the composition further comprises a buffer of sodium acetate or acetic acid.

8. A pharmaceutical composition for use according to claim 7, where in the buffer is at a concentration in the range 10 to 100nM.

9. A pharmaceutical composition for use according to any one of claims 1 to 8, wherein the composition further comprises sodium chloride.

10. A pharmaceutical composition for use according to claim 9, wherein the sodium chloride is in the range 20 to 200nM.

11. A pharmaceutical composition for use according to any one of claims 1 to 10, wherein the composition has a minimal number and/or quantity of excipients.

12. A pharmaceutical composition for use according to any one of claims 1 to 11, wherein the therapeutic protein complex is equimolar.

13. A pharmaceutical composition for use according to any one of claims 1 to 12, wherein the non-ionic surfactant is polysorbate.

14. A pharmaceutical composition for use according to claim 13, wherein the polysorbate is polysorbate 20.

15. A pharmaceutic composition for use according to claim 13, wherein the polysorbate is polysorbate 80.

16. A pharmaceutic composition for use according to any one of claims 1 to 15.
